# EUROPEAN PATENT APPLICATION

(11) **EP 4 549 584 A2**
(43) Date of publication of application: **07.05.2025**
(21) Application number: 25161085.3
(22) Date of filing: 24.05.2019
(51) Int. Cl.: C12Q 1/6869

(54) **METHOD**

(30) Priority: 24.05.2018 GB 201808554
(62) Divisional of application: 19727476.4
(71) Applicant: Oxford Nanopore Technologies PLC, Oxford OX4 4DQ (GB)
(72) Inventor: Graham, James Edward, Oxford, OX4 4DQ (GB); Raimondeau, Etienne, Oxford, OX4 4DQ (GB); Bowen, Rebecca Victoria, Oxford, OX4 4DQ (GB)
(74) Representative: J A Kemp LLP

(57) **Abstract**

A method for selectively modifying a target polynucleotide in a sample of polynucleotides, the method comprising contacting a sample of polynucleotides with a guide polynucleotide that binds to a sequence in the target polynucleotide and a polynucleotide-guided effector protein such that the polynucleotide-guided effector protein cuts the target polynucleotide to produce a cut end comprising an overhang; and attaching an adapter to the cut end in the target polynucleotide.

## Description

### Field

The invention relates to methods of selectively adapting a target polynucleotide in a sample of polynucleotides. The invention also relates to methods of characterising the modified polynucleotides.

### Background

There is currently a need for rapid and cheap polynucleotide (e.g. DNA or RNA) sequencing and identification technologies across a wide range of applications. Existing technologies are slow and expensive mainly because they rely on amplification techniques to produce large volumes of polynucleotide and require a high quantity of specialist fluorescent chemicals for signal detection.

Transmembrane pores (nanopores) have great potential as direct, electrical biosensors for polymers and a variety of small molecules. In particular, recent focus has been given to nanopores as a potential DNA sequencing technology.

When a potential is applied across a nanopore, there is a change in the current flow when an analyte, such as a nucleotide, resides transiently in the barrel for a certain period of time. Nanopore detection of the nucleotide gives a current change of known signature and duration. In the strand sequencing method, a single polynucleotide strand is passed through the pore and the identity of the nucleotides are derived. Strand sequencing can involve the use of a molecular brake to control the movement of the polynucleotide through the pore.

There are many commercial situations, including polynucleotide sequencing and identification technologies, which require the preparation of a nucleic acid library. This is frequently achieved using a transposase. Depending on the transposase which is used to prepare the library it may be necessary to repair the transposition events *in vitro* before the library can be used, for example in sequencing.

### Summary

The inventors have devised a method of selectively adapting a target polynucleotide in a sample of polynucleotides. In the method, the ends of the polynucleotides are protected to prevent non-specific addition of adapters to the ends of the polynucleotides in the sample. The method utilises a guide polynucleotide and a polynucleotide-guided effector protein to cut within a target polypeptide and add one or more adapter to at least one of the cut ends. The target polynucleotide can then be characterised, such as by strand sequencing, without needing to physically separate the target polynucleotide from other polynucleotides in the sample. For example, in nanopore sequencing methods, the signals obtained from the target polynucleotides are effectively enhanced as the background signals resulting from polynucleotides adapted at their ends are very low.

The ends of the polynucleotides in the sample can be protected simply by chemically altering the ends of the polynucleotides. For example, the 5' ends of a polynucleotide are normally phosphorylated. When the ends of the polynucleotides are dephosphorylated and the target polynucleotide is cut using a polynucleotide guided effector protein, an adapter may be attached (e.g. ligated) to the cut ends but not to the dephoshorylated ends. This enables an adapter to be selectively covalently attached to the cut ends of the target polynucleotide. Dephosphorylation of the ends can be achieved simply and easily by adding a dephosphorylase to the sample of polynucleotides. The dephosphorylase does not need to be removed from the sample prior to further processing of the sample. The dephosphorylase can simply be heat inactivated prior to addition of the cutting enzyme.

Another example of a method of chemically altering the ends of the polynucleotides is to extend the 3' ends of the polynucleotides using a terminal transferase to add a 3' tail comprising at least one nucleotide. This prevents ligation to an adapter bearing a 3' overhang.. This enables an adapter being covalently attached to the cut ends of the target polynucleotide. Thus, no complicated steps are required to protect the ends of the polynucleotides in the sample and no adapters are added to polynucleotides in the sample that are not cut by the polynucleotide-guided effector protein. The selective addition of adapters to the target polynucleotides enables detection and/or characterisation of the target polypeptides without needing to physically separate the target polynucleotides from other polynucleotides in the sample, and the background signal in any detection/characterisation method is reduced compared to methods in which the ends are not protected. The selective addition of adapters to the target polynucleotides can also be used to physically separate the target polynucleotides from other polynucleotides in a sample. For example, the adapter may be used as a tag to separate the target polynucleotide, such as by using the adapter to attach biotin to the target polynucleotide, allowing the target polynucleotide to be attached to beads.

The method has the advantage of requiring minimal sample preparation. The steps of the method can be carried out without requiring clean up steps between the method steps and, in some embodiments, the method can be carried out in a single pot. The sample may be analysed directly to characterise the target polynucleotide without separation from the non-target polynucleotides. In the context of sequencing, the method enables long reads to be obtained. In the context of characterisation, the method enables long polynucleotides to be screened for modification, for example to detect methylated, or otherwise modified, bases, to identify structural changes in a polynucleotide, such as detecting a transposition event, detecting a polymorphism or monitoring expansion repeats. The cut sites in the target polynucleotide can also be designed to achieve coverage of a long polynucleotide as multiple fragments.

Accordingly, the following are provided:
- A method for selectively adapting a target polynucleotide in a sample of polynucleotides, the method comprising: protecting the ends of the polynucleotides in the sample; contacting the polynucleotides with a guide polynucleotide that binds to a sequence in the target polynucleotide and a polynucleotide-guided effector protein such that the polynucleotide-guided effector protein cuts the target polynucleotide to produce two opposing cut ends at a site determined by the sequence to which the guide polynucleotide binds; and attaching an adapter to one or both of the two opposing cut ends in the target polynucleotide, wherein the adapter attaches to one or both of the cut ends in the target polynucleotide but does not attach to the protected ends of the polynucleotides in the sample;
- A method of detecting and/or characterising a target polynucleotide comprising: contacting a sample obtained by the method above with a nanopore; applying a potential difference across the nanopore; and monitoring for the presence or absence of an effect resulting from the interaction of the target polynucleotide with the nanopore to determine the presence or absence of the target polynucleotide, thereby detecting the target polynucleotide in the sample and/or monitoring the interaction of the target polynucleotide with the nanopore to determine one or more characteristics of the target polynucleotide;
- A kit for selectively modifying a target polynucleotide in a sample of polynucleotides, the kit comprising a dephosphorylase, an adapter comprising a single N or polyN tail, wherein N is the nucleotide A, T, C or G, and optionally one or more of a polymerase, a ligase, a polynucleotide-guided effector protein and a guide polynucleotide; and
- A method for selectively adapting a target polynucleotide in a sample of polynucleotides, the method comprising: contacting the polynucleotides in the sample with two guide polynucleotides that bind to a sequences in the target polynucleotide and a polynucleotide-guided effector protein, wherein the sequences to which the two guide polynucleotides bind direct the polynucleotide-guided effector protein to two different sites that may or may not be closely located, such that the polynucleotide-guided effector protein cuts the target polynucleotide at at least one of the two sites to produce two opposing cut ends; and attaching an adapter to one or both of the two opposing cut ends in the target polynucleotide.

### Description of the Figures

It is to be understood that Figures are for the illustration purposes and are not intended to be limiting.

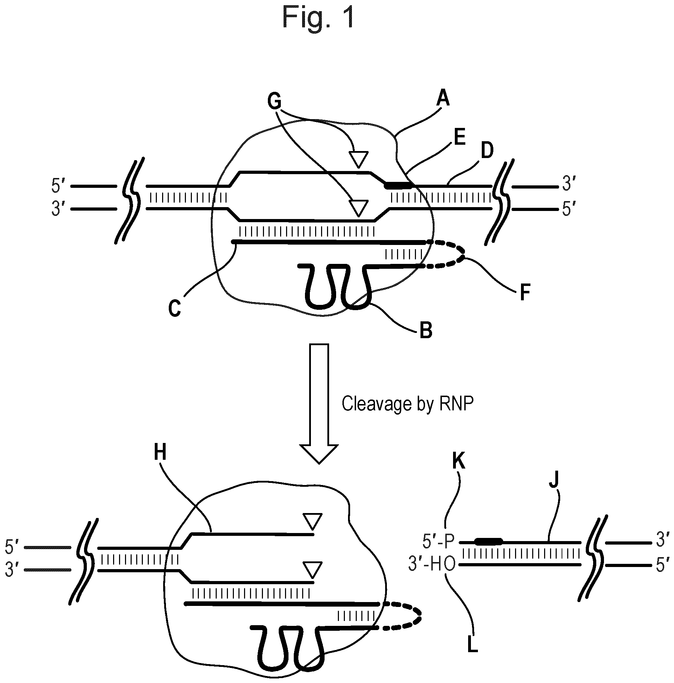

**Figure 1****:** shows schematically how a Cas9 enzyme **A,** with bound tracrRNA **B and** crRNA **C,** may be used to cleave a target dsDNA molecule **D** containing a protospacer-adjacent motif (PAM) **E.** The tracrRNA and crRNA may be incorporated as a single-guide RNA (sgRNA) molecule by interlinking the two with a hairpin **F.** Cas9 cleaves the molecule using two nuclease centres **G** to yield two dsDNA fragments, **H** and **J,** one of which **(H)** is protected by Cas9, and the other of which **(J)** bears a free 5' phosphate **K** and 3' hydroxyl group **L.**

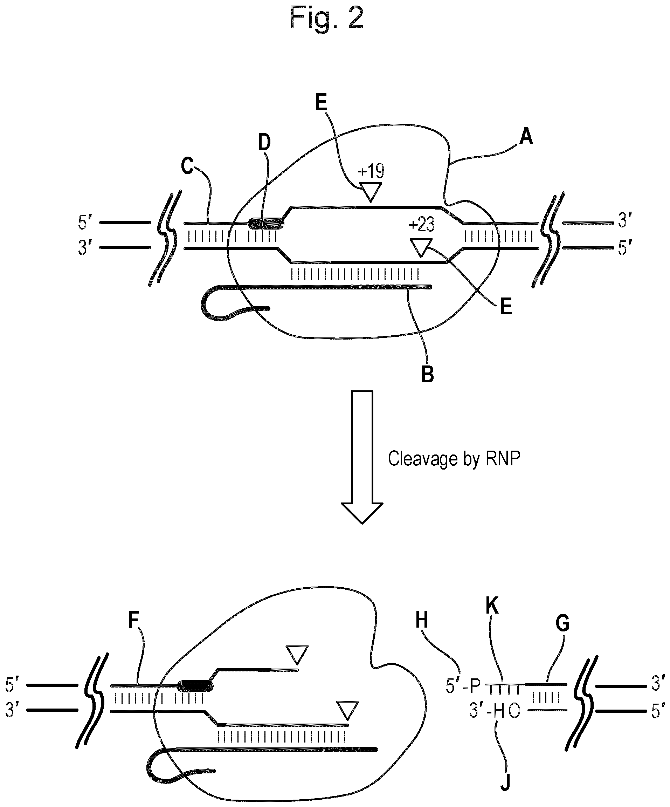

**Figure 2** shows schematically how a Cpf1 enzyme **A,** with bound crRNA **B,** may be used to cleave a target dsDNA molecule **C** containing a protospacer-adjacent motif (PAM) **D.** Cpf1 cleaves the molecule using a single nuclease centre at two sites **E** to yield two dsDNA fragments, **F** and **G,** one of which **(F)** is protected by Cpf1, and the other of which **(G)** bears a free 5' phosphate **H,** 3' hydroxyl group **J,** and 5' overhang **K.**

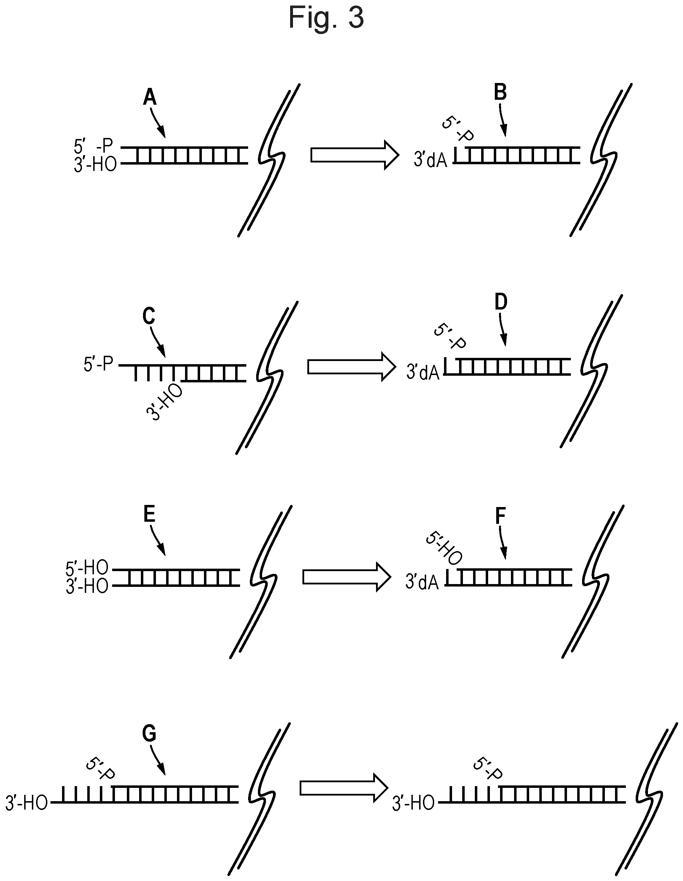

**Figure 3** shows schematically the treatment of various DNA products with DNA-processing enzymes: a blunt-ended dsDNA fragment **A** treated with a polymerase (e.g. Taq or Klenow exo- polymerase) and dATP to yield a 3'-dA-tailed fragment **B;** a 5' overhang fragment **C** treated with a polymerase (e.g. Taq or Klenow exo- polymerase) and a mixture of dATP, dCTP, dGTP and dTTP to yield a 3'-dA-taled fragment **D;** a 5'-dephosphorylated fragment **E** treated with a polymerase (e.g. Taq or Klenow exo-polymerase) and dATP to yield a 3'-dA-tailed, 5'-dephosphorylated fragment **F;** and a 3'-overhang fragment (such as produced by terminal transferase) **G** treated with a polymerase (e.g. Taq or Klenow exo- polymerase) and dNTPs that produces no overall change in the end-structure of the fragment.

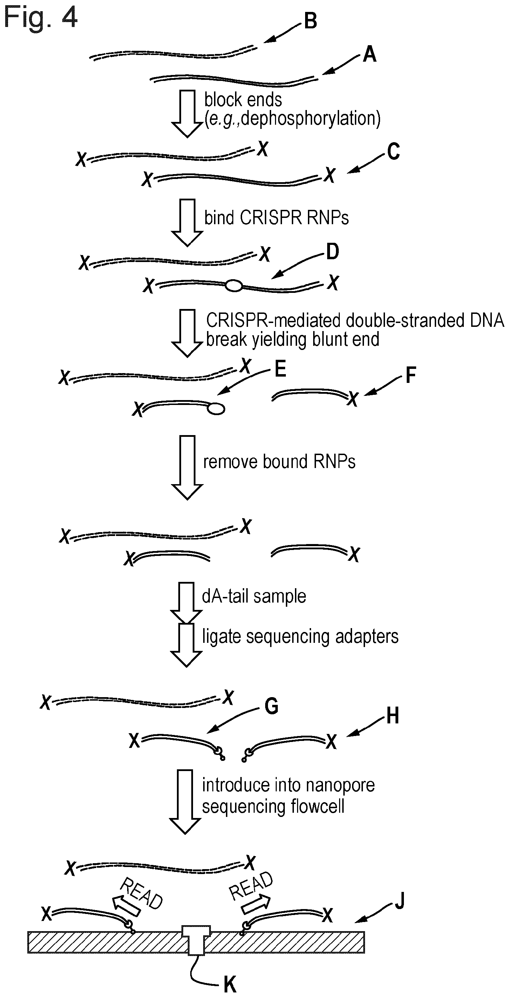

**Figure 4** shows one possible workflow by which a target DNA molecule may be sequenced by protecting the ends by dephosphorylation, revealing phosphates via polynucleotide-guided effector protein cleavage (e.g. CRISPR/Cas cleavage), removing the polynucleotide-guided effector protein (e.g. the Cas9 enzyme), dA-tailing the ends, ligating adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into two fragments **E** and **F.** Upon removal of bound complexes (e.g. RNPs) by deproteinisation, dA-tailing and ligation of sequencing adapters yields two adapter-ligated target fragments **G** and **H,** which when introduced into a nanopore sequencing flowcell comprising membrane J and pore K, may both be sequenced. Both target and non-target molecules are introduced into the flowcell, but only target molecules tether onto the membrane and are sequenced.

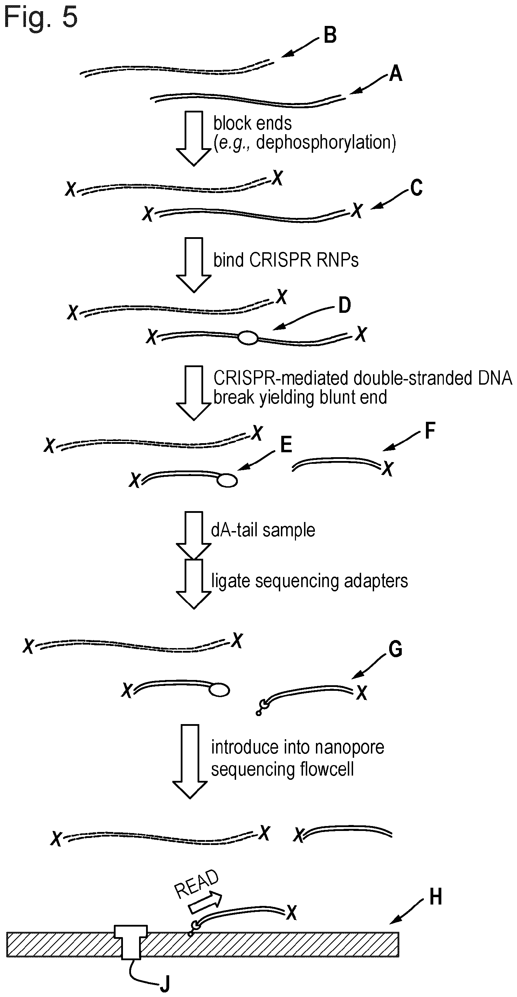

**Figure 5** shows one possible workflow by which a target DNA molecule may be sequenced by protecting the ends by dephosphorylation, revealing phosphates via polynucleotide-guided effector protein cleavage (e.g. CRISPR/Cas cleavage), dA-tailing the ends, ligating adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into two fragments **E** and **F.** dA-tailing and ligation of sequencing adapters yields one adapter-ligated target fragments **G,** which when introduced into a nanopore sequencing flowcell comprising membrane **H** and pore **J,** may be sequenced. Both target and non-target molecules are introduced into the flowcell, but only target molecules tether onto the membrane and are sequenced.

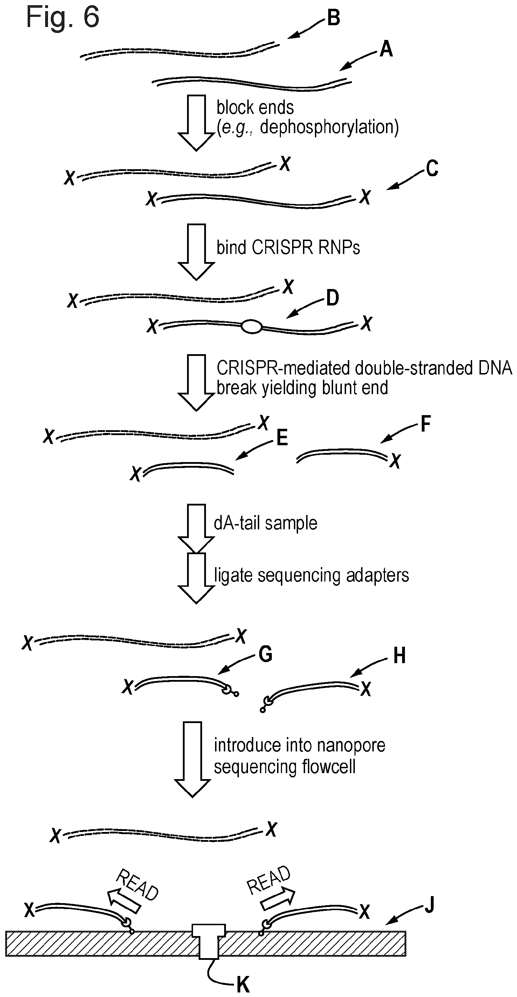

**Figure 6** shows one possible workflow by which a target DNA molecule may be sequenced by protecting the ends by dephosphorylation, revealing phosphates via polynucleotide-guided effector protein cleavage (e.g. CRISPR/Cas cleavage), dA-tailing the ends, ligating adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into two fragments **E** and **F.** Here, the complex (RNP) dissociates spontaneously. dA-tailing and ligation of sequencing adapters yields two adapter-ligated target fragments **G** and **H,** which when introduced into a nanopore sequencing flowcell comprising membrane **J** and pore **K,** may both be sequenced. Both target and non-target molecules are introduced into the flowcell, but only target molecules tether onto the membrane and are sequenced.

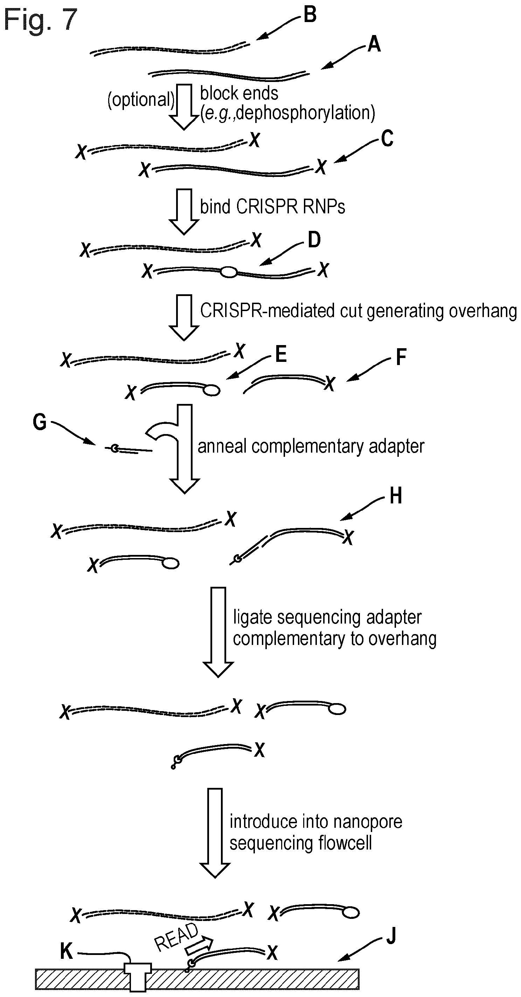

**Figure 7** shows one possible workflow by which a target DNA molecule may be sequenced by protecting the ends by dephosphorylation, revealing phosphates via polynucleotide-guided effector protein cleavage (e.g. CRISPR/Cas cleavage), ligating complementary adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into two fragments **E** and **F.** Here, the complex (RNP) dissociates spontaneously. Ligation of complementary sequencing adapters **(G)** yields one adapter-ligated target fragment H, which when introduced into a nanopore sequencing flowcell comprising membrane **J** and pore **K,** may both be sequenced. Both target and non-target molecules are introduced into the flowcell, but only target molecules tether onto the membrane and are sequenced.

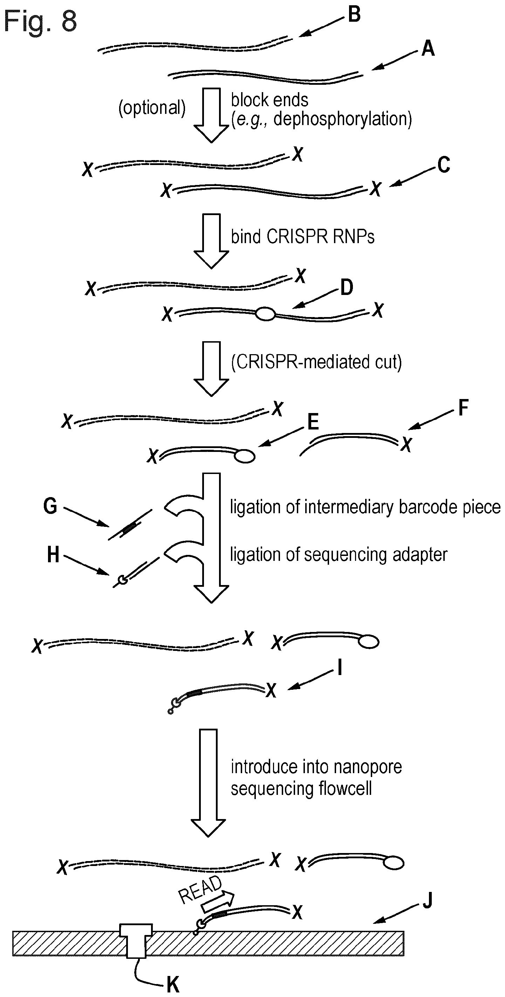

**Figure 8****:** shows one possible workflow by which a target DNA molecule may be sequenced by protecting the ends by dephosphorylation, revealing phosphates via polynucleotide-guided effector protein cleavage (e.g. CRISPR/Cas cleavage), ligating complementary intermediary barcode pieces and sequencing adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into two fragments **E** and **F.** Here, the RNP dissociates spontaneously. Ligation of complementary intermediary barcode **(G)** and sequencing adapters **(H)** yields one adapter-ligated target fragment **I,** which when introduced into a nanopore sequencing flowcell comprising membrane **J** and pore **K,** may both be sequenced. Both target and non-target molecules are introduced into the flowcell, but only target molecules tether onto the membrane and are sequenced.

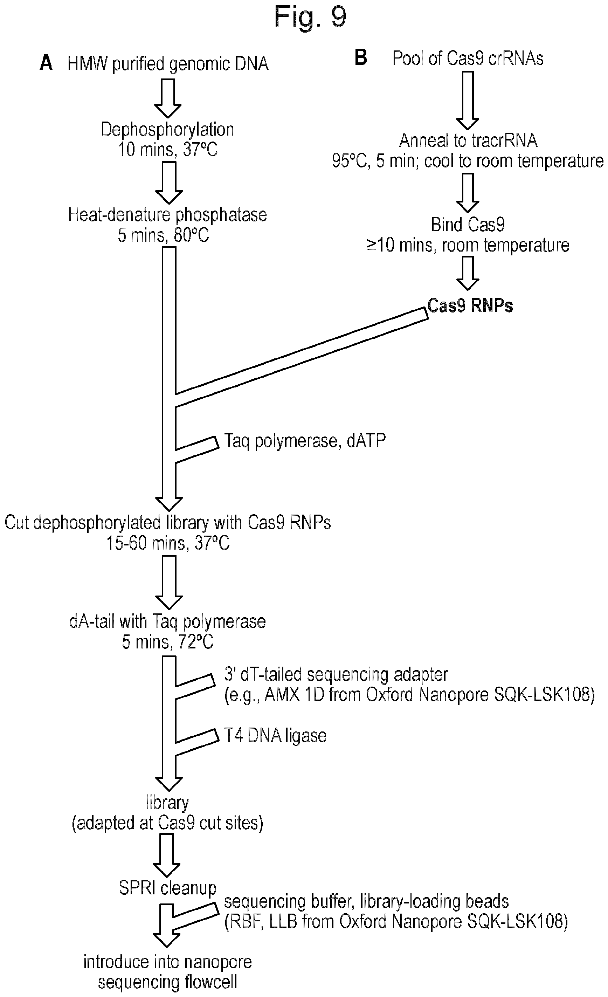

**Figure 9** shows an example of a workflow by which a target DNA molecule may be sequenced by protecting the ends by dephosphorylation, revealing phosphates via CRISPR/Cas9 cleavage, dA-tailing, ligating to sequencing adapters, and introducing into a sequencing device. In tube **A,** high molecular weight genomic DNA is dephosphorylated by dephosphorylase enzyme (such as calf intestinal phosphatase) for 10 minutes at 37°C and the enzyme is heat inactivated for 5 minutes at 80°C. Simultaneously in tube **B,** crRNAs are annealed to tracrRNA and RNPs are formed by incubating this mixture with Cas9 for 10 minutes at room temperature. Subsequently, the content of tube B is added to tube A, in addition to Taq polymerase and dATP. The mixture is incubated for 15-60 minutes at 37°C to allow cleavage and dA-tailing of the dephosphorylated target DNA. The fragments of interest are ligated to the sequencing adaptor using T4 DNA Ligase forming the sequencing library. Following SPRI purification of the library, the sample is introduced to the sequencing device.

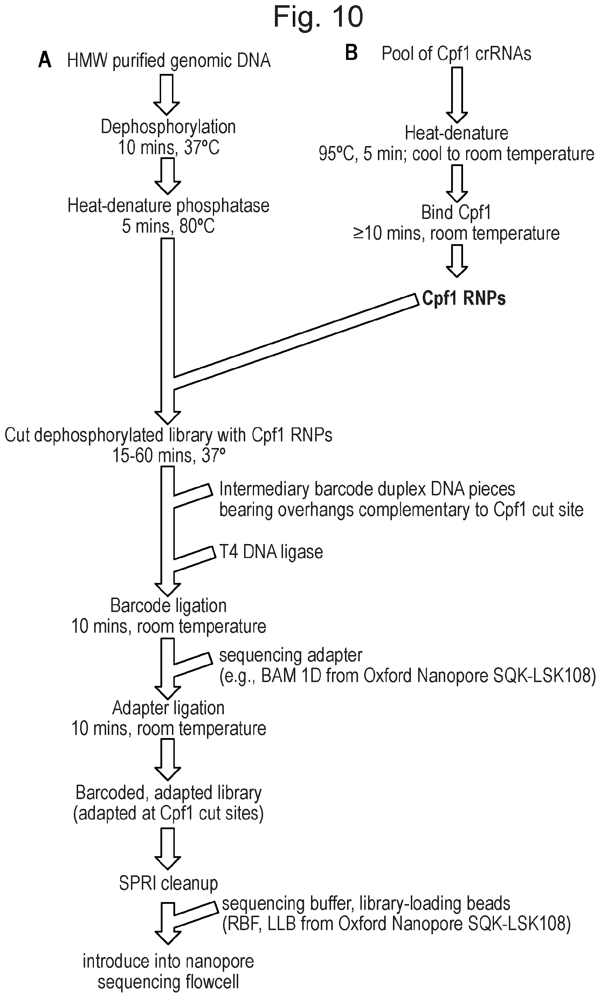

**Figure 10** shows an example of a workflow by which a target DNA molecule may be sequenced by protecting the ends by dephosphorylation, revealing phosphates via CRISPR/Cpf1 cleavage, dA-tailing, ligating to sequencing adapters, and introducing into a sequencing device. In tube **A,** high molecular weight genomic DNA is dephosphorylated by dephosphorylase enzyme (such as calf intestinal phosphatase) for 10 minutes at 37°C and the enzyme is heat inactivated for 5 minutes at 80°C. Simultaneously in tube **B,** crRNAs are heat denature and RNPs are formed by incubating this mixture with Cas9 for 10 minutes at room temperature. Subsequently, the content of tube B is added to tube A and incubated for 15-60 minutes at 37°C to allow cleavage of the dephosphorylated target DNA. The fragments of interest are ligated to the barcode and sequencing adaptor forming the sequencing library. Following SPRI purification of the library, the sample is introduced to the sequencing device.

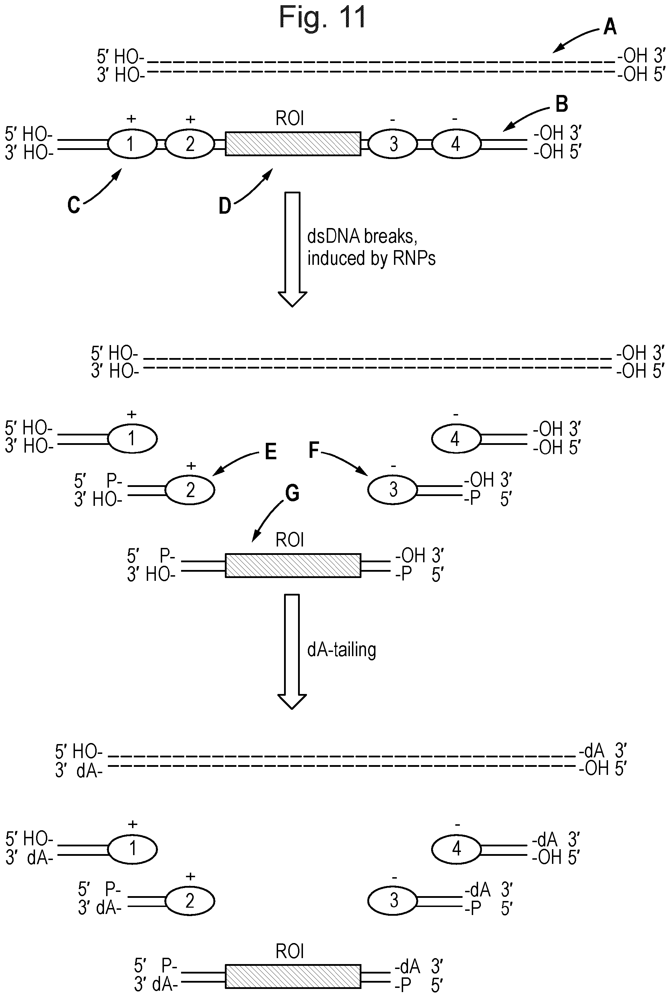

**Figure 11** shows schematically the cleavage pattern of the target DNA **(B)** but not of the non target DNA **(A)** induced by guide-polynucleotide/polynucleotide-guided effector protein cleavage (e.g. CRISPR/Cas RNPs) **(C)** with redundant probes complementary to flanking region of the region of interest **(D).** RNPs 1 and 2 are binding to the sense strand (+) upstream of the ROI and RNPs 3 and 4 are recognizing the antisense strand (-). Following cleavage by the RNPs, 5 fragments are generated. Only 3 out the fragments generated contain a 5' Phosphate **(E, F and G)** and can be read by the sequencing device. Fragment **G** is the only fragment containing both ligatable ends. dA-tailing is performed as shown in **Figure 3****.**

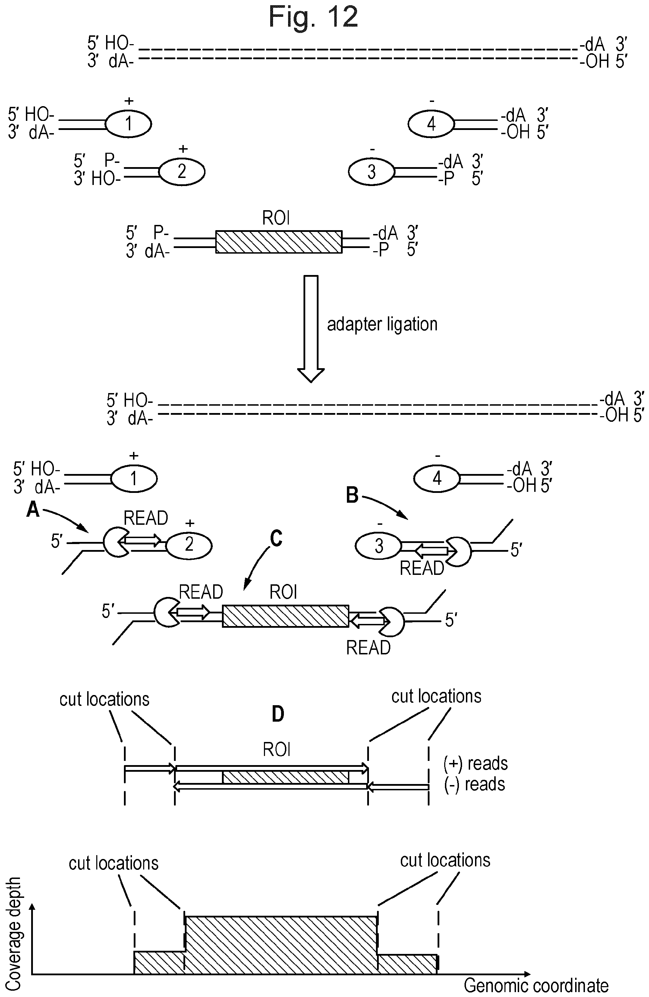

**Figure 12** shows the ligation of sequencing adapters to the target DNA fragments generated as shown in **Figure 11****.** Following dA-tailing, ligation of sequencing adapters yields three adapter-ligated target fragments **A, B** and **C.** Fragment **A** can be sequenced in the sense direction, while Fragment **B** can be read from the antisense direction. Both ends of fragment **C** were cleaved by RNPs allowing the ligation of two sequencing adaptors at both ends and thus the sequencing in both sense and antisense directions. The length and directions of the sequencing reads are summarised in the schematic **D.** The plotting of the number of reads or coverage depth along the genomic coordinates show a classical increase in coverage between RNPs 2 and 3 due to the bidirectionality of the sequencing of fragment C.

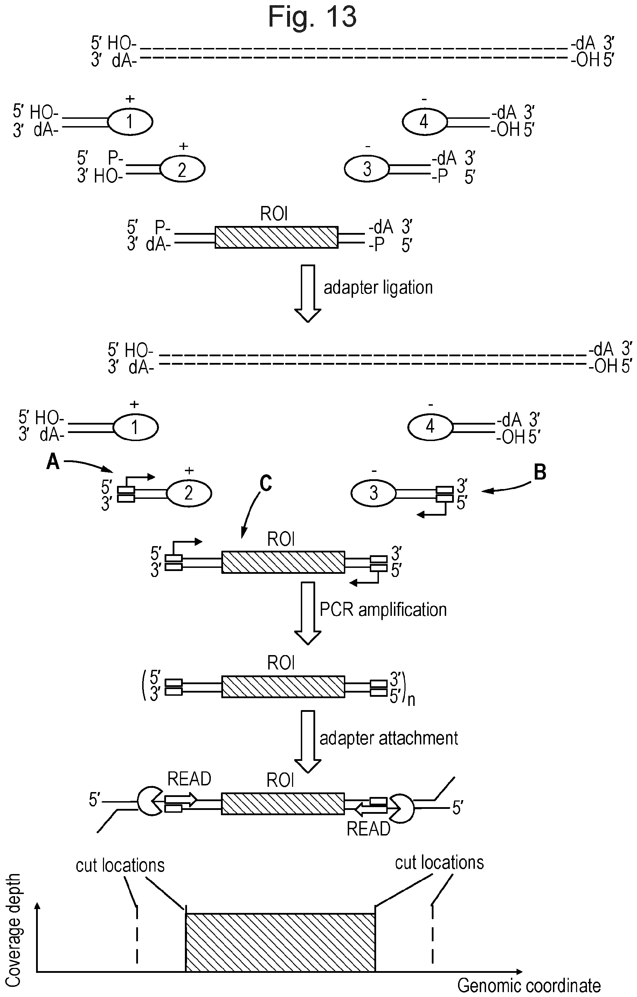

**Figure 13** shows the PCR amplification of target DNA fragments generated as shown in **Figure 11** for sequencing purposes. Following dA-tailing, the annealing of PCR adapters yields three adapter-ligated target fragments **A, B** and **C.** Both ends of fragment **C** were cleaved by RNPs allowing the ligation of two PCR adaptors at each end thus allowing PCR amplification. Following PCR, the amplified region of interest is ligated to sequencing adaptor allowing sequencing in both sense and antisense direction. In this case, the plotting of the coverage depth along the genomic coordinates show only coverage between cutting sites for RNPs 2 and 3.

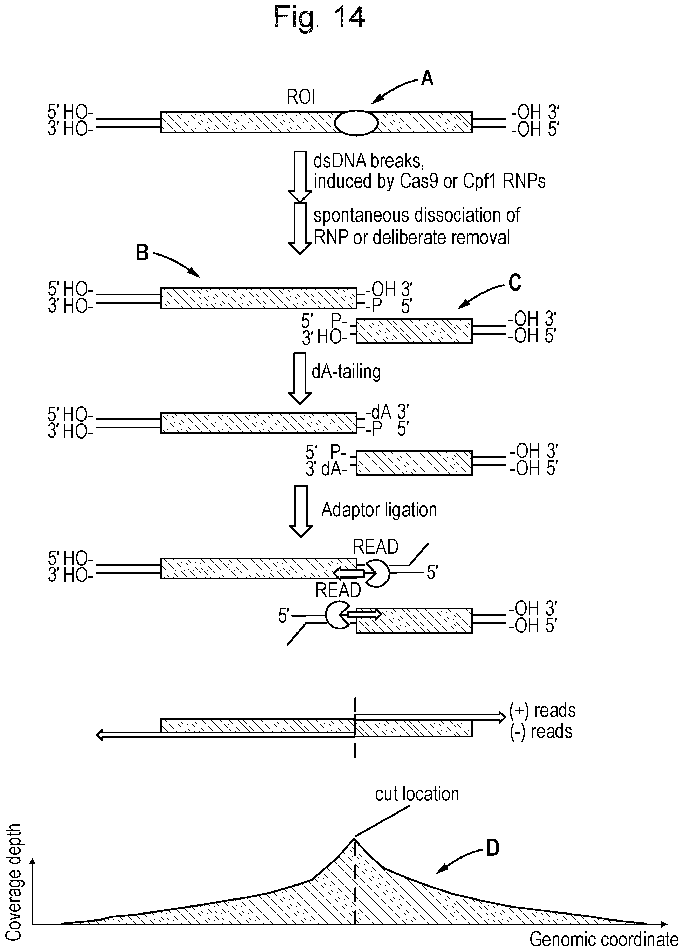

**Figure 14** explores the sequencing pattern of a single dsDNA break in the region of interest (ROI) induced by guide-polynucleotide/polynucleotide-guided effector protein cleavage (e.g. CRISPR/Cas RNPs) **(A).** In the event that the RNP released both sides of the cut, the two fragments **(B** and **C)** are accessible for dA-tailing and sequencing adaptor ligation. Fragment B is read in the antisense direction (-) and fragment C in the sense direction (+) resulting in a decreasing coverage depth **(D)** from the cut location in both direction.

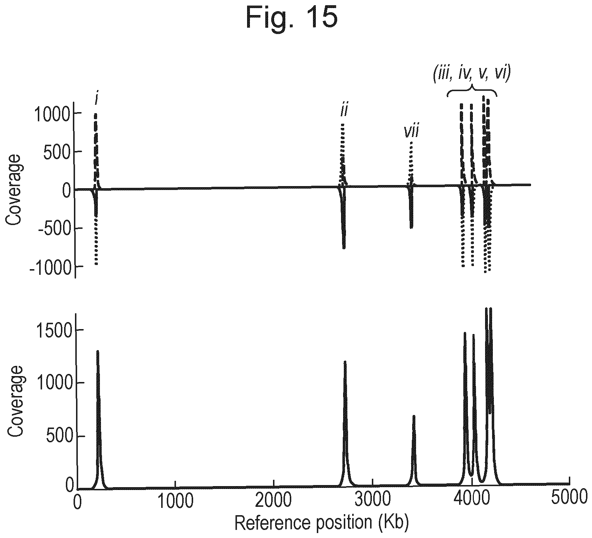

**Figure 15** shows an example coverage plot showing the enrichment of all1 6S (*rrs*) genes from a total *E. coli* genomic sample, using a degenerated crRNA probe directed against the *rrs* genes of *E. coli* K-12, strain MG1655. The panel shows a plot of coverage versus position for forwards (positive numbers) and reverse (negative numbers) direction reads. Seven target peaks, *i* to *vii,* are indentified, which are over-represented against background

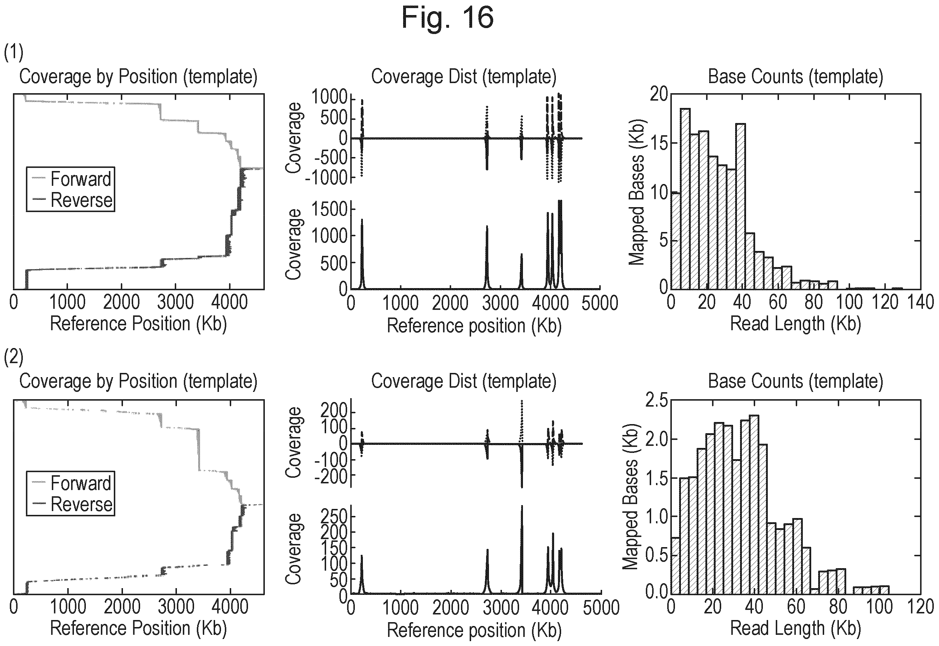

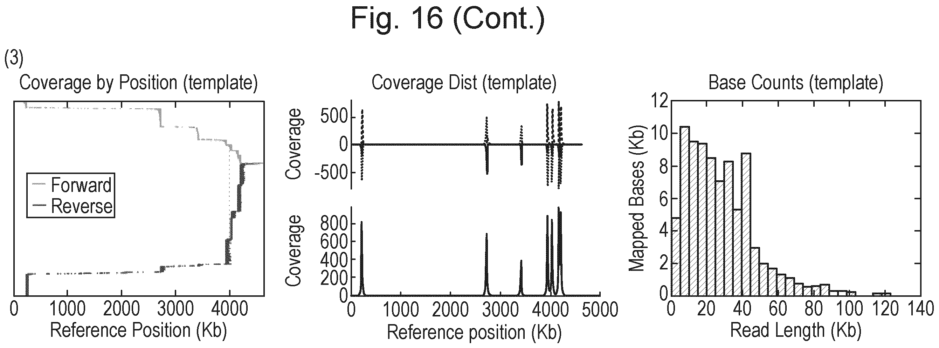

**Figure 16** highlights the differences between the three approaches (1), (2) and (3) used in **Example 1.** The left and middle panels in each of (1), (2) and (3) show the coverage obtained using the three approaches and the right panels in each of (1), (2) and (3) show the pileups resulting from alignment of the sequencing reads to the E. coli reference.

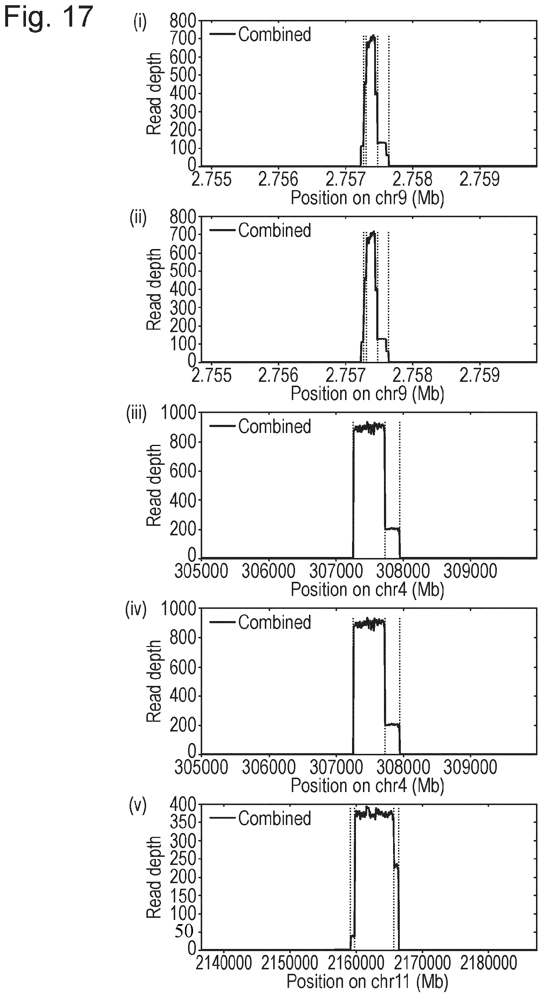

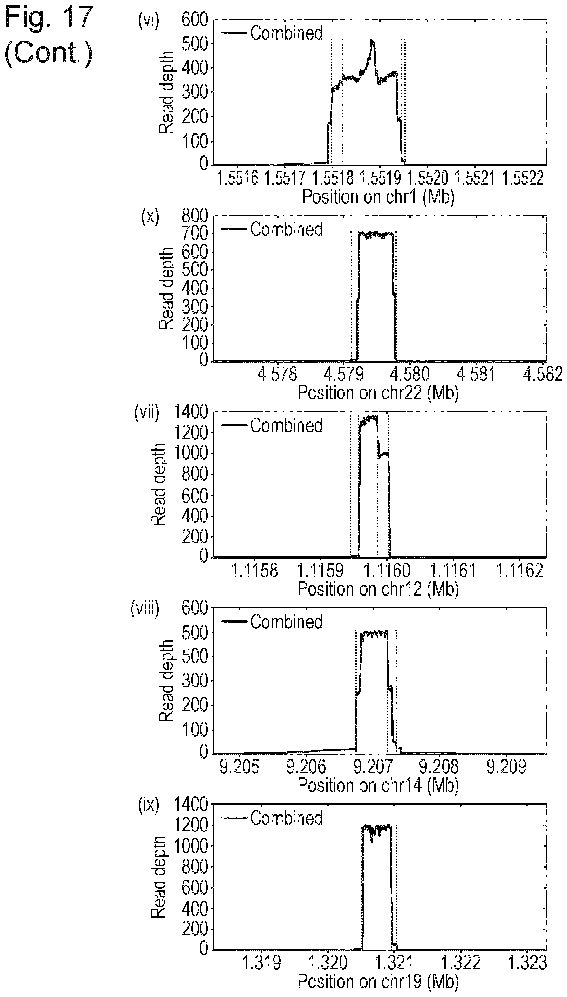

**Figure 17****:** shows Cas9 enrichment of library A described in **Example 2**. The panel shows the pileups resulting from alignment of sequencing reads to the human NA12878 reference following dA-tailing by Klenow exo- subsequently to Cas9 cleavage.

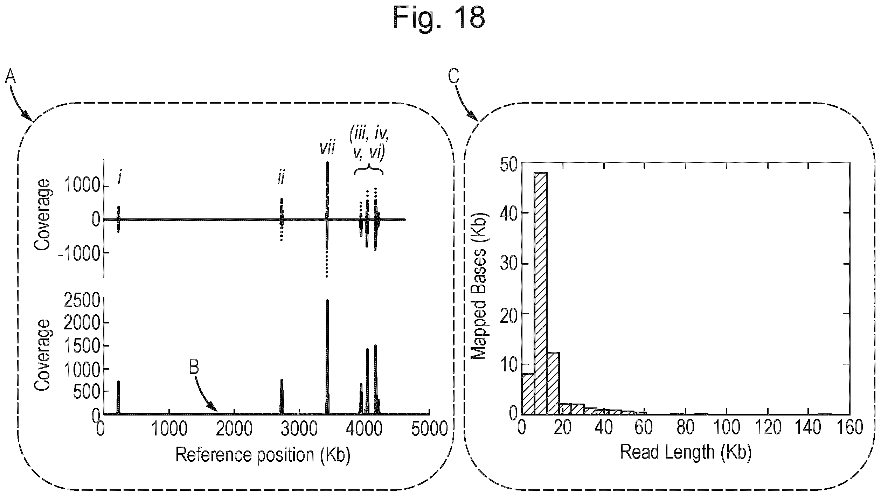

**Figure 18** shows an example coverage plot showing the enrichment of all 16S (*rrs*) genes from a total *E. coli* genomic sample, using crRNA probes directed against the *rrs* genes of *E. coli* K-12, strain MG1655. **A, left** shows a plot of coverage versus position for forwards (positive numbers) and reverse (negative numbers) direction reads. Seven target peaks, *i* to *vii,* are identified, which are over-represented against background **B. A,** bottom shows the aggregation of forwards and reverse direction reads. **C** shows a histogram of the read length of all reads that successfully mapped to the reference, normalised to the number of bases mapped in each bin.

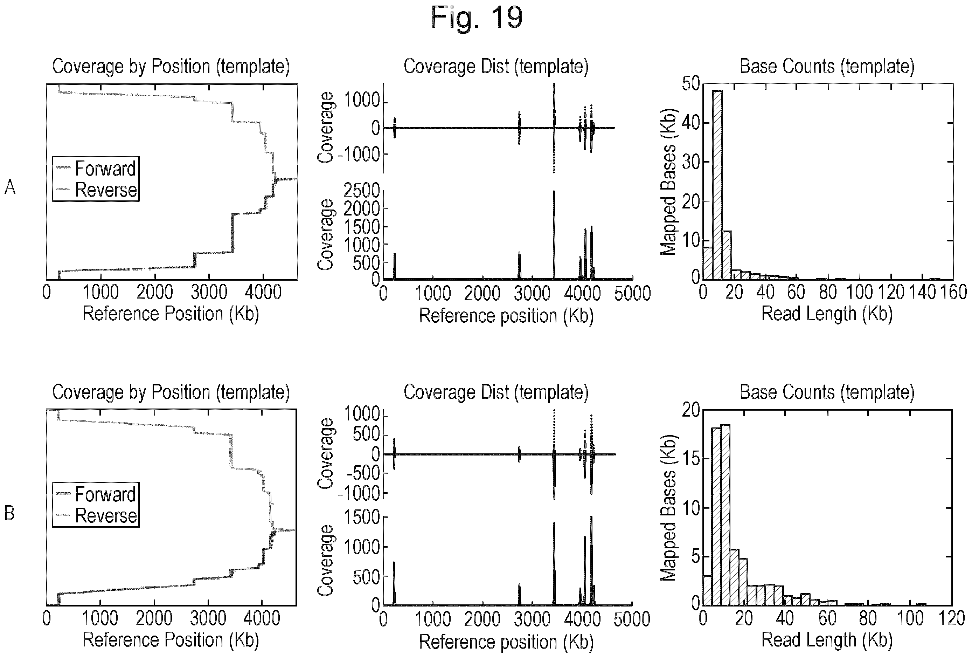

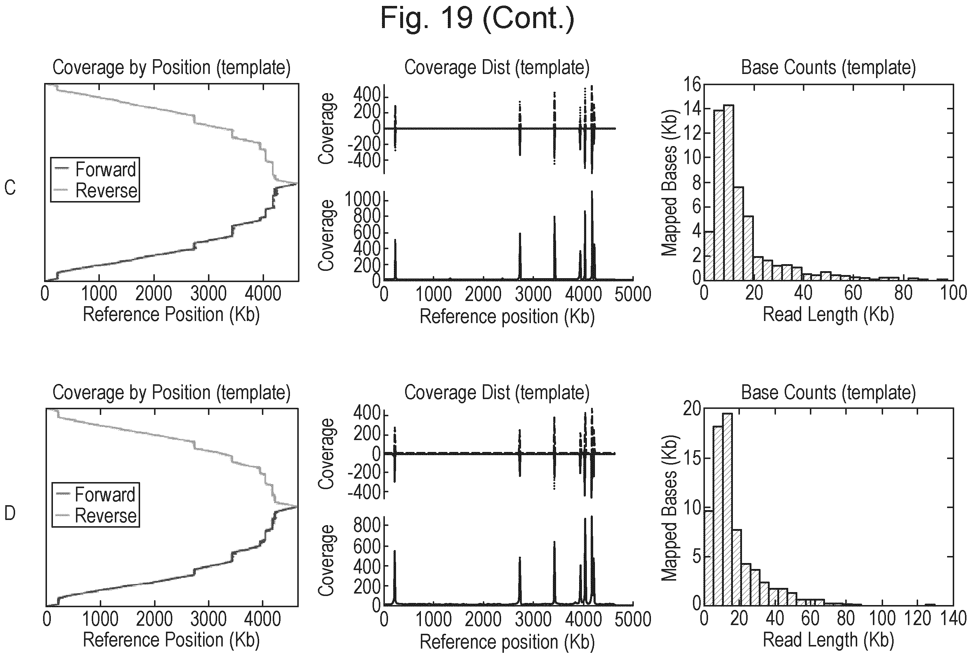

**Figure 19** compares the different approaches use for Cpf1 enrichment. **A** shows an experiment in which specific barcodes to the 5'nt overhang cutting site sequences were used to sequence *E. coli* rrs 16S genes. **B** shows an equivalent experiment in which generic barcodes able to bind to multiple 5'nt overhang sequences. **C** and **D** compare equivalent experiments where the enzyme (Klenow (exo-) or Taq, respectively, are used to fill and dA-tail the 5'nt overhang.

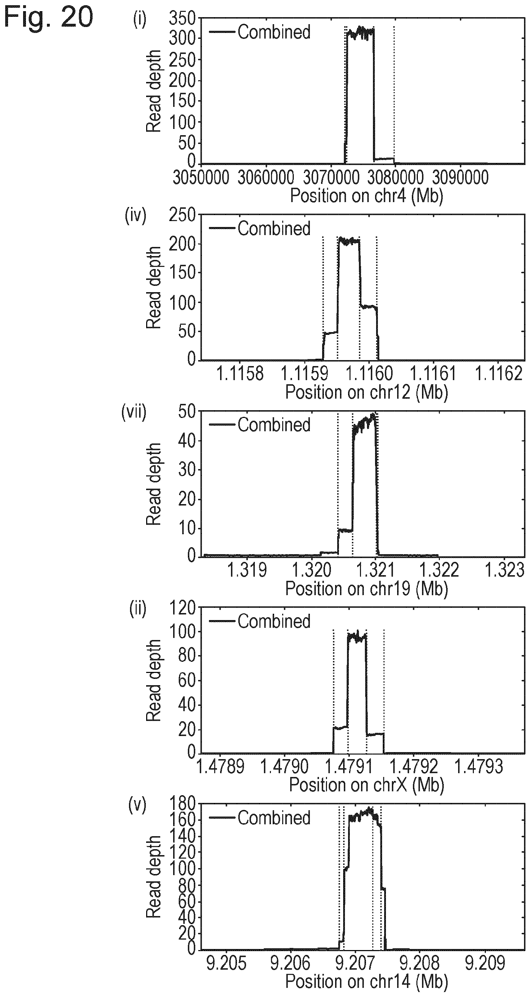

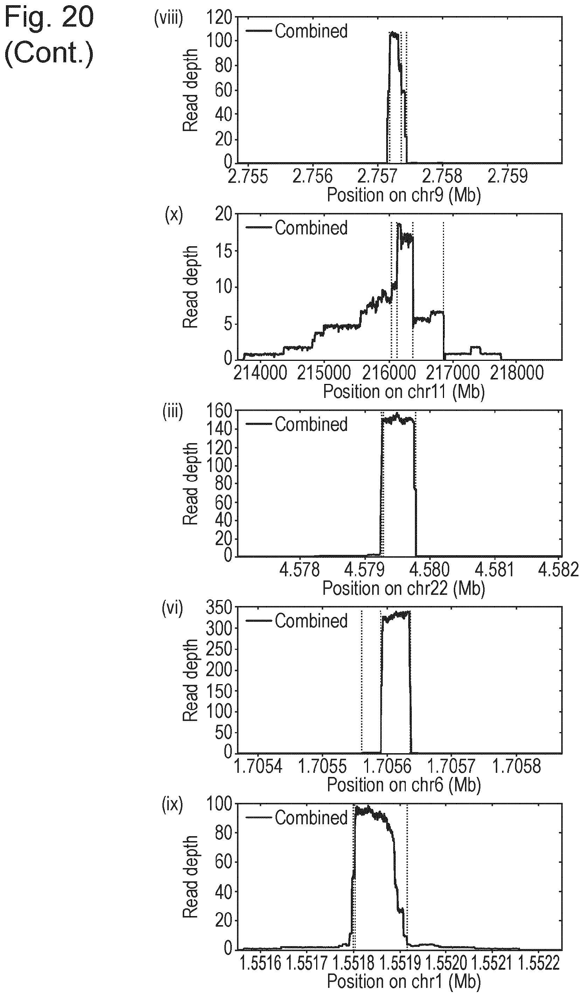

**Figure 20** shows the pileups resulting from alignment of sequencing reads to the human NA12878 reference obtained using the specific barcode approach for Cpf1 enrichment with a human genomic DNA sample.

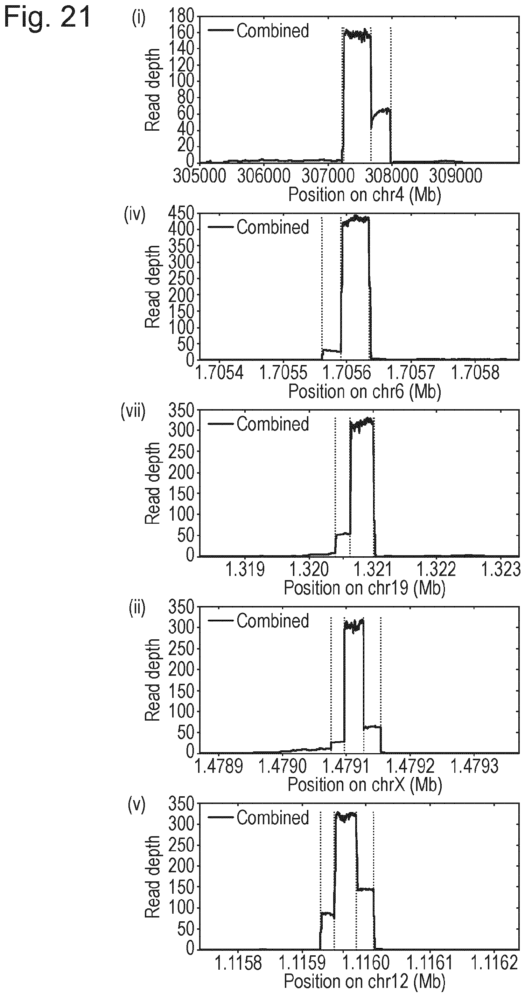

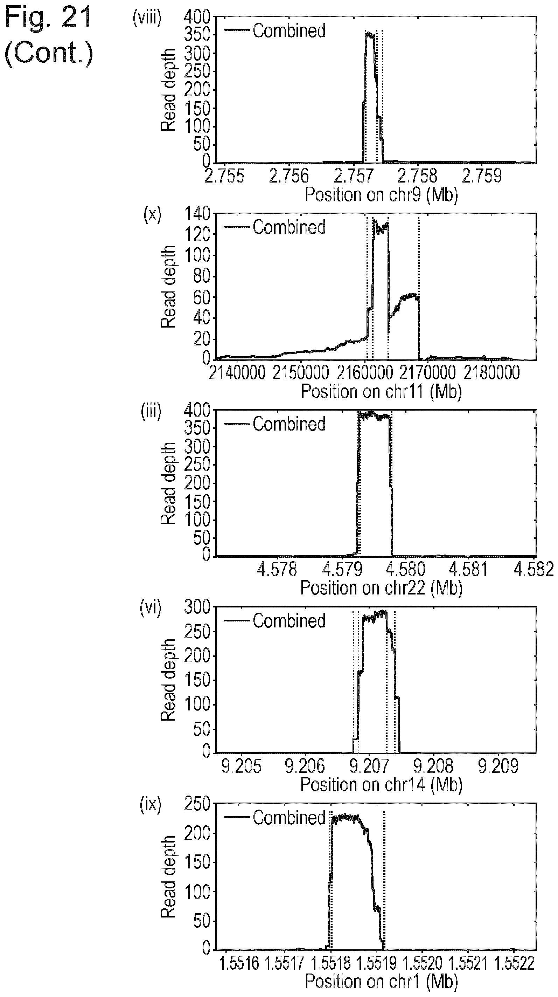

**Figure 21** shows the pileups resulting from alignment of sequencing reads to the human NA12878 reference obtained using the dA-tailing with Klenow (exo-) approach for Cpf1 enrichment with a human genomic DNA sample.

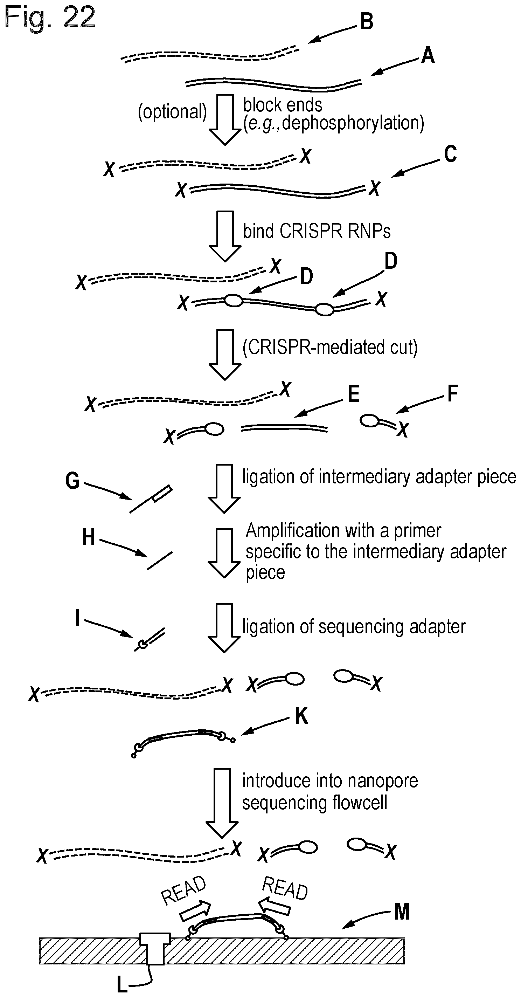

**Figure 22** shows one possible workflow by which a target DNA molecule may be sequenced by protecting the ends by dephosphorylation, revealing phosphates via polynucleotide-guided effector protein cleavage (e.g. CRISPR/Cas cleavage) at two sites, optionally dA-tailing the ends, ligating adapters, and introducing into a sequencing device. A mixture of target **(A)** and non-target **(B)** high-molecular weight DNA is treated by a dephosphorylase enzyme (such as calf intestinal phosphatase) to yield library molecules with blocked ends **C.** Upon binding guide polynucleotide/polynucleotide-guided effector protein complexes (e.g. CRISPR RNPs) **D,** a double-strand break is introduced that cleaves the target molecule into three fragments **E** and **F.** Here, the complex (RNP) remains bound to the two outer fragments **F.** An intermediate adapter piece **G** comprising a single stranded outer region is ligated to the inner fragment E. Fragment E is amplified using a primer **H** specific to the single stranded outer region of the intermediate adapter piece **G.** Ligation of sequencing adapters yields an adapter-ligated target fragments **K,** which when introduced into a nanopore sequencing flowcell comprising membrane **M** and pore **L,** may be sequenced. Both target and non-target molecules are introduced into the flowcell, but only target molecules tether onto the membrane and are sequenced.

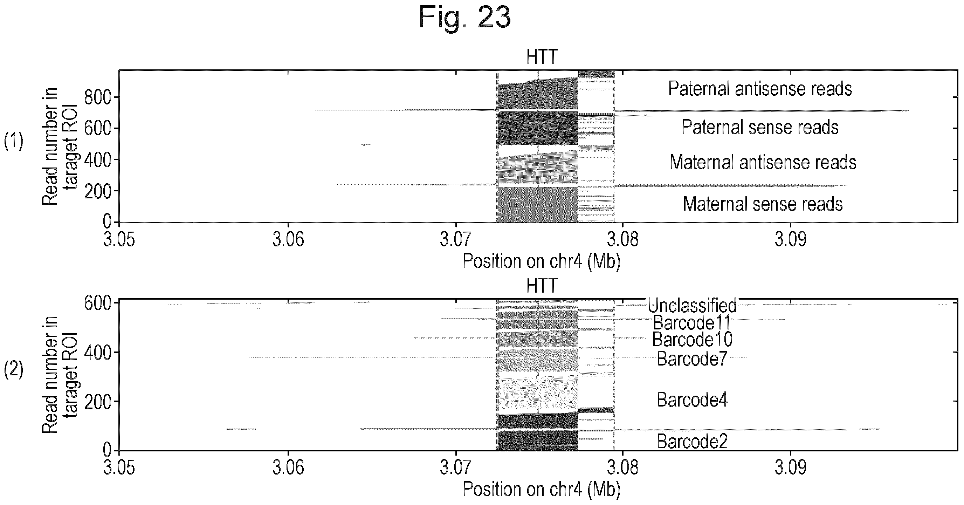

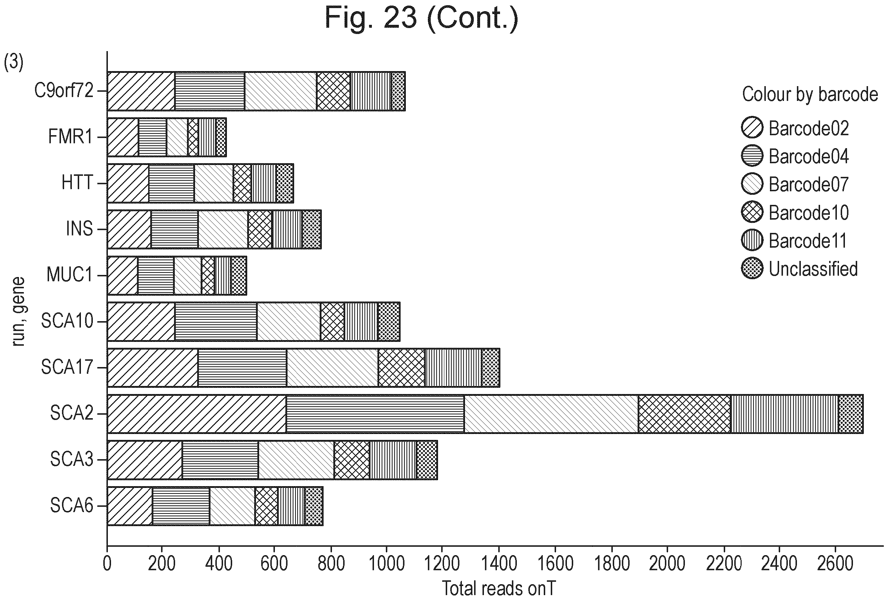

**Figure 23** shows the pileups resulting from alignment of sequencing reads to the human NA12878 reference (HTT gene) for **Library A** (1) **and B** (2) as well as the number of reads per barcodes per gene in **library B** (3) as described in Example 5.

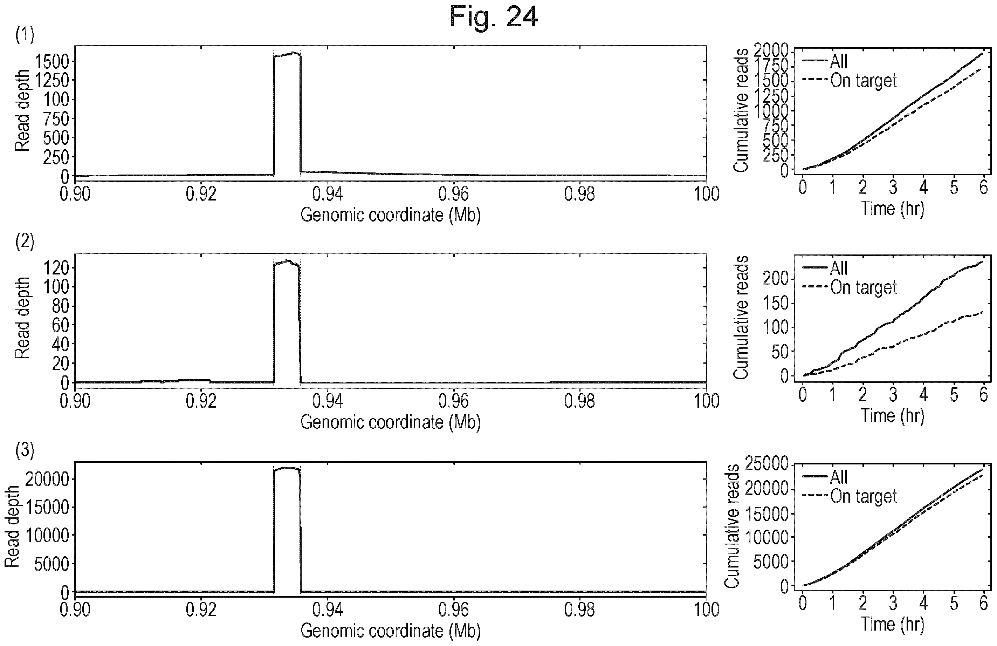

**Figure 24** shows the pileups resulting from alignment of sequencing reads to the *E. coli* SCS110 reference following the no amplification (1), amplification with phosphorylated (2) or dephosphorylated (3) PCR adapter approaches of Example 6.

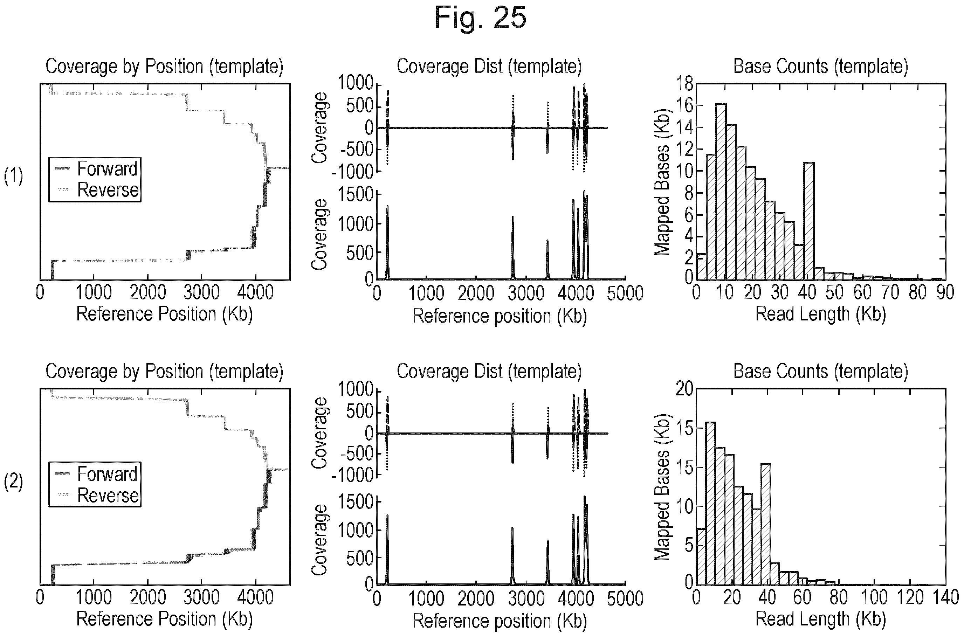

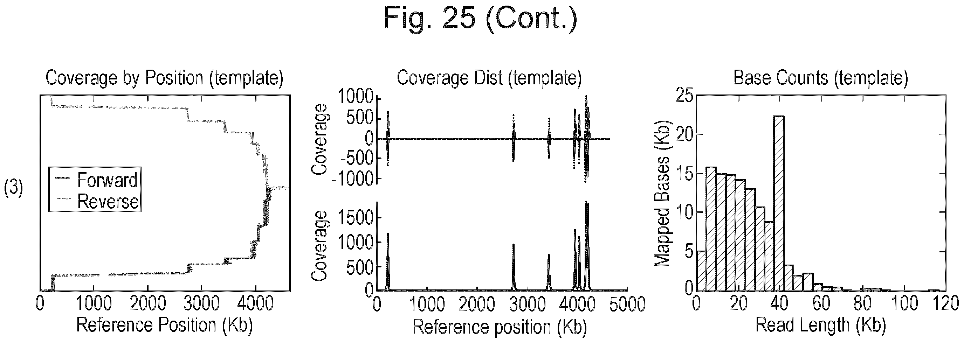

**Figure 25** shows the pileups resulting from alignment of sequencing reads to the *E. coli* reference as described in Example 7. (1) shows the pileups from a reaction in which the sequencing adapter was ligated to the target-cleaved, dA-tailed sample. (2) shows the pileups from a reaction in which the target-cleaved was digested by RNAseH then dA-tailed by Taq Polymerase prior to ligation of the sequencing adapter. (3) shows the pileups from a reaction in which the target-cleaved DNA, was incubated with RNAseH following Cas9 denaturation and then dA-tailed prior to ligation of the sequencing adapter.

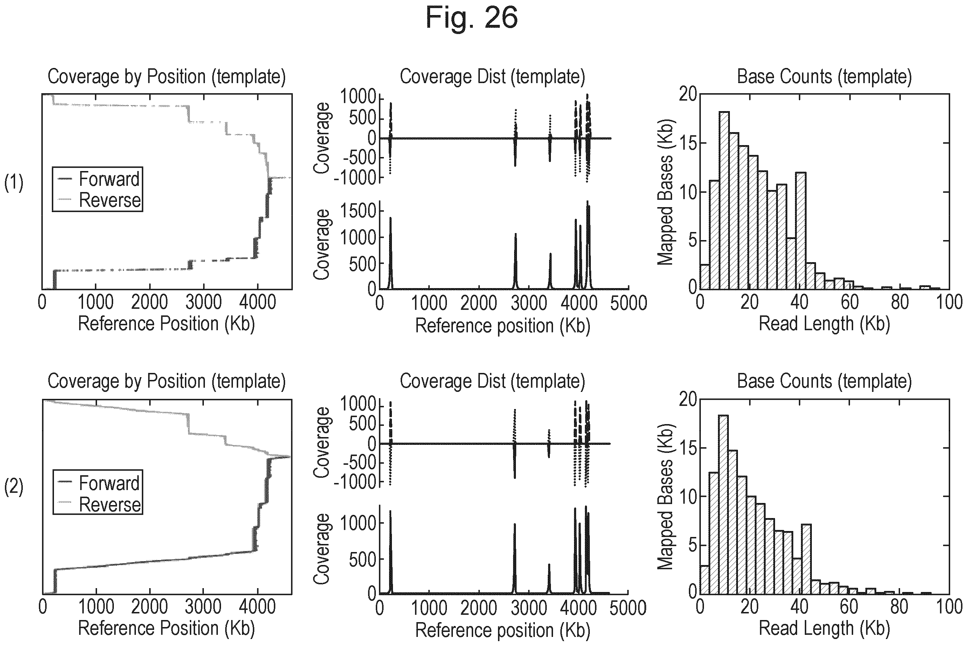

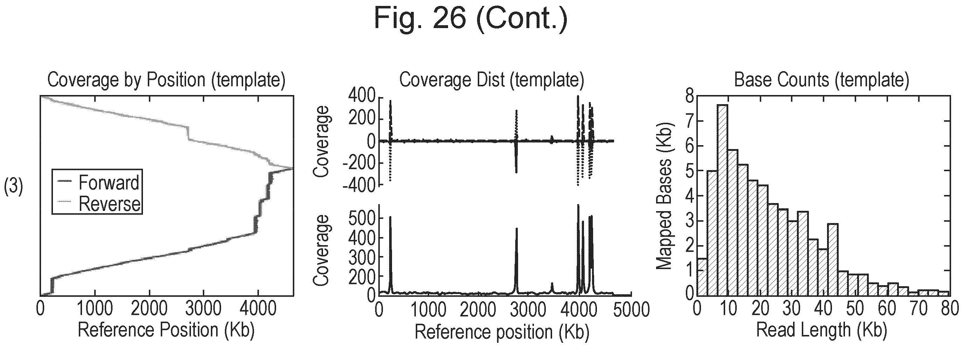

**Figure 26** shows the pileups resulting from alignment of sequencing reads to the *E. coli* reference as described in Example 8. (1) shows the pileups from a reaction in which the sequencing adapter was ligated to the target-cleaved, dA-tailed sample. (2) shows the pileups from a reaction in which the target-cleaved DNA, was incubated with T4 DNA polymerase and then dA-tailed prior to ligation of the sequencing adapter. (3) shows the pileups from a reaction in which the target-cleaved, was incubated with RNAseH following Cas9 denaturation and dA-tailed prior to ligation of the sequencing adapter.

### Detailed Description

It is to be understood that different applications of the disclosed methods and products may be tailored to the specific needs in the art. It is also to be understood that the terminology used herein is for the purpose of describing particular embodiments of the methods and products only, and is not intended to be limiting. Also features defined as pertaining to an embodiment may be combined with features pertaining to another embodiment.

In addition as used in this specification and the appended claims, the singular forms "a", "an", and "the" include plural referents unless the content clearly dictates otherwise. Thus, for example, reference to "a polynucleotide" includes two or more polynucleotides, reference to "an anchor" refers to two or more anchors, reference to "a helicase" includes two or more helicases, and reference to "a transmembrane pore" includes two or more pores and the like.

All publications, patents and patent applications cited herein, whether supra or infra, are hereby incorporated by reference in their entirety.

The present inventors have devised a method for selectively modifying a target polynucleotide in a sample of polynucleotides. The method results in the selective modification of a target polynucleotide in a sample of polynucleotides. This means that the adapter is added only to the target polynucleotide, or target polynucleotides. The target polynucleotide(s) can then be analysed or characterised without needing to be separated from other (non-target) polynucleotides in the sample.

The method devised by the inventors results in the selective adaptation of a target polynucleotide, or target polynucleotides, in a sample of polynucleotides, the method comprising: protecting the ends of the polynucleotides in the sample; contacting the polynucleotides with a guide polynucleotide that binds to a sequence in the target polynucleotide and a polynucleotide-guided effector protein such that the polynucleotide-guided effector protein cuts the target polynucleotide to produce two opposing cut ends at a site determined by the sequence to which the guide polynucleotide binds; and attaching an adapter to one or both of the two opposing cut ends in the target polynucleotide, wherein the adapter attaches to one or both of the cut ends in the target polynucleotide but does not attach to the protected ends of the polynucleotides in the sample.

The method may be used to produce a library of adapted polynucleotides, wherein multiple guide polynucleotides are used to direct one or more polynucleotide-guided effector protein to cut one or more target polynucleotide, and/or to cut within multiple sites within the same target polynucleotide.

### Protecting the ends

The method comprises a step of protecting the ends of the polynucleotides in the sample. The ends of the polynucleotides in the sample are protected to prevent adapters from attaching to the ends of the polynucleotides. Ideally the ends of every polynucleotide in the sample are protected. However, in practice only a proportion of the polynucleotides in the sample may have both ends protected. For example, about 50% or more, about 60% or more, about 70% or more, about 80% or more, about 90% or more or about 95% or more of the polynucleotides in the sample may have protected ends.

The ends of the polynucleotides in the sample can be protected by chemically altering the ends of the polynucleotides. The ends are preferably protected enzymatically. This means that the ends are protected by adding an enzyme to the sample, optionally with a substrate such as one or more free dNTPs. The enzyme may, for example, be a dephosphorylase or a terminal transferase.

For example, the 5' ends of a polynucleotide are normally phosphorylated. When the ends of the polynucleotides are dephosphorylated and the target polynucleotide is cut using a polynucleotide guided effector protein, an adapter may be attached (e.g. ligated) to the cut ends but not to the dephoshorylated ends. This enables an adapter comprising, for example, a single T overhang or a polyT overhang to be selectively hybridised and covalently attached to the cut ends of the target polynucleotide. Dephosphorylation of the ends can be achieved simply and easily by adding a dephosphorylase to the sample of polynucleotides. The dephosphorylase does not need to be removed from the sample prior to further processing of the sample. The dephosphorylase can simply be heat inactivated prior to addition of the cutting enzyme.

Thus, in the method the ends of the polynucleotides in the sample may be protected by dephosphorylating the 5' ends of the polynucleotides. The method may comprise adding a dephosphorylase to the sample of polynucleotides. The dephosphorylase may be added to the sample and incubated for a suitable amount of time. The skilled person will readily be able to determine a suitable time period. For example, the period for which the sample is incubated with the dephosphorylase may be from about 5 to about 30 minutes, such as from about 10 to about 15 minutes, preferably about 10 minutes. The incubation temperature is typically determined by the optimal temperature of the dephosphorylase used, but may for example be in the range of about 20°C to about 40°C, such as about 30°C, or preferably about 37°C.

Another example of a method of chemically altering the ends of the polynucleotides is to extend the 3' ends of the polynucleotides using a terminal transferase to add a 3' tail comprising at least one nucleotide. This prevents ligation to an adapter bearing a 3' overhang. This enables an adapter being covalently attached to the cut ends of the target polynucleotide. A dephosphorylase and a terminal transferase may both be used to protect the ends of the polynucleotides.

The method of protecting the ends of the polynucleotide preferably does not involve joining the 5' and 3' ends of the opposite strands of double stranded polynucleotides in the sample, for example, the method does not comprise attaching a hairpin loop between the adjoining 5' and 3' ends of the opposite strands of the double stranded polynucleotides. However, the ends may be protected by circularisation of the polynucleotide, e.g. by joining the 5' end of the each strand of a double stranded polynucleotide to the 3' end of the same strand.

The ends of the polynucleotides in the sample can be protected using blocking chemistry. For example, biotin may be attached to the ends of the polynucleotides on one or both of the strands and then bound to streptavidin. Alternatively, one or both ends of each polynucleotide may be attached to a solid surface, such as the surface of a bead, using a suitable attachment means, such as biotin-streptavidin, or other affinity molecules.

### Sample

The sample may be any suitable sample comprising polynucleotides.

The sample may be a biological sample. The invention may be carried out *in vitro* on a sample obtained from or extracted from any organism or microorganism. The organism or microorganism is typically archaean, prokaryotic or eukaryotic and typically belongs to one the five kingdoms: plantae, animalia, fungi, monera and protista. The invention may be carried out *in vitro* on a sample obtained from or extracted from any virus.

The sample is preferably a fluid sample. The sample typically comprises a body fluid. The body fluid may be obtained from a human or animal. The human or animal may have, be suspected of having or be at risk of a disease. The sample may be urine, lymph, saliva, mucus, seminal fluid or amniotic fluid, but is preferably whole blood, plasma or serum. Typically, the sample is human in origin, but alternatively it may be from another mammal such as from commercially farmed animals such as horses, cattle, sheep or pigs or may alternatively be pets such as cats or dogs.

Alternatively a sample of plant origin is typically obtained from a commercial crop, such as a cereal, legume, fruit or vegetable, for example wheat, barley, oats, canola, maize, soya, rice, bananas, apples, tomatoes, potatoes, grapes, tobacco, beans, lentils, sugar cane, cocoa, cotton, tea or coffee.

The sample may be a non-biological sample. The non-biological sample is preferably a fluid sample. Examples of non-biological samples include surgical fluids, water such as drinking water, sea water or river water, and reagents for laboratory tests.

The sample may be processed prior to carrying out the method, for example by centrifugation or by passage through a membrane that filters out unwanted molecules or cells, such as red blood cells. The method may be performed on the sample immediately upon being taken. The sample may also be typically stored prior to the method, preferably below -70°C.

The sample may comprise genomic DNA. Preferably the genomic DNA is not fragmented. The genomic DNA may be from any organism. The genomic DNA may be human genomic DNA.

### Target polynucleotide

The polynucleotide can be a nucleic acid, such as deoxyribonucleic acid (DNA) or ribonucleic acid (RNA). The polynucleotide can comprise one strand of RNA hybridised to one strand of DNA. The polynucleotide may comprise one or more synthetic nucleotide. Synthetic nucleotides known in the art include peptide nucleic acid (PNA), glycerol nucleic acid (GNA), threose nucleic acid (TNA), locked nucleic acid (LNA) or other synthetic polymers with nucleotide side chains.

The polynucleotide is preferably DNA, RNA or a DNA/RNA hybrid, most preferably DNA. The target polynucleotide preferably comprises a double stranded region to which the guide-polynucleotide and polynucleotide-guided effector protein bind. The target polynucleotide may be double stranded. The target polypeptide may be single stranded and a small single stranded polynucleotide may be hybridised to the target site of the guide polynucleotide and polynucleotide-guided effector protein. The target polypeptide may comprise single stranded regions and regions with other structures, such as hairpin loops, triplexes and/or quadruplexes. The DNA/RNA hybrid may comprise DNA and RNA on the same strand. Preferably, the DNA/RNA hybrid comprises one DNA strand hybridized to a RNA strand. In a preferred embodiment, the polynucleotide is genomic DNA. The genomic DNA is typically double stranded.

The target polynucleotide can be any length. For example, the polynucleotides can at least 500 nucleotides or nucleotide pairs in length. The target polynucleotide can be 1000 or more nucleotides or nucleotide pairs, 5000 or more nucleotides or nucleotide pairs in length or 100000 or more nucleotides or nucleotide pairs in length.

The target polynucleotide may be a polynucleotide associated with a disease and/or a microorganism.

The method may involve multiple target polynucleotides. The target polynucleotides may be a group of polynucleotides. For instance, the group may be associated with a particular phenotype. The group may be associated with a particular type of cell. For instance, the group may be indicative of a bacterial cell. The group may be indicative of a virus, a fungus, a bacterium, a mycobacterium or a parasite.

The target polynucleotides may be a group of two or more polynucleotides that are biomarkers associated with a particular disease or condition. The biomarkers can be used to diagnose or prognose the disease or condition. Suitable panels of biomarkers are known in the art, for example as described in Edwards et al (2008) Mol. Cell. Proteomics 7: 1824-1837; Jacquet et al (2009) Mol. Cell. Proteomics 8: 2687-2699; Anderson et al (2010) Clin. Chem. 56: 177-185. The disease or condition may, for example, be cancer, heart disease, including coronary heart disease and cardiovascular disease, or an infectious disease, such as tuberculosis or sepsis. The disease or condition may be a disease associated with expansion repeats, such as Huntington's Disease, Fragile X, Spinal and Bulbar Muscular Atropy or Myotonic Dystrophy.

The target polynucleotide may be a microRNA (or miRNA) or a small interfereing RNA (siRNA). The group of two or more target polynucleotides may be a group of two or more miRNAs. Suitable miRNAs for use in the invention are well known in the art. For instance, suitable miRNAs are stored on publically available databases.

The sequence of the target polynucleotide may be known or unknown. At least a portion of the target polynucleotide is preferably known so that a guide polynucleotide may target an effector protein to the target polynucleotide.

### Polynucleotide-guided effector protein

The polynucleotide-guided effector protein may be any protein that binds to a guide-polynucleotide and which cuts the polynucleotide to which the guide polynucleotide binds. The guide polynucleotide may be a guide RNA, a guide DNA, or a guide containing both DNA and RNA. The guide polynucleotide is preferably a guide RNA. Therefore the polynucleotide-guided effector protein is preferably a RNA-guided effector protein.

The RNA-guided effector protein may be any protein that binds to the guide-RNA. The RNA-guided effector protein typically binds to a region of guide RNA that is not the region of guide RNA which binds to the target polynucleotide. For example, where the guide RNA comprises crRNA and tracrRNA, the RNA-guided effector protein typically binds to the tracrRNA and the crRNA typically binds to the target polynucleotide. The RNA-guided effector protein preferably also binds to a target polynucleotide. The RNA-guided effector protein typically binds to a double stranded region of the target polynucleotide. The site of the target polynucleotide which is cut by the RNA-guided effector protein binds is typically located close to the sequence to which the guide RNA hybridizes.

The RNA-guided effector protein may cut upstream or downstream of the sequence to which the guide RNA binds. For example, the RNA-guided effector protein may bind to a protospacer adjacent motif (PAM) in DNA located next to the sequence to which the guide RNA binds. A PAM is typically a 2 to 6 base pair sequence, such as 5'-NGG-3' (wherein N is any base), 5'-NGA-3', 5'-YG-3' (wherein Y is a pyrimidine), 5'TTN-3' or 5'-YTN-3'. Different RNA-guided effector proteins bind to different PAMs. RNA-guided effector proteins may bind to a target polynucleotide which does not comprise a PAM, in particular, where the target is RNA or a DNA/RNA hybrid.

The RNA-guided effector protein is typically a nuclease, such as a RNA-guided endonuclease. The RNA-guided effector protein is typically a Cas protein. The RNA-guided effector protein may be Cas, Csn2, Cpf1, Csf1, Cmr5, Csm2, Csy1, Cse1 or C2c2. The Cas protein may Cas3, Cas 4, Cas8a, Cas8b, Cas8c, Cas9, Cas10, Cas10d, Cas12a (Cpf1) or Cas13. Preferably, the Cas protein is Cas9 or Cas12a. Cas, Csn2, Cpf1, Csf1, Cmr5, Csm2, Csy1 or Cse1 is preferably used where the target polynucleotide comprises a double stranded DNA region. C2c2 is preferably used where the target polynucleotide comprises a double stranded RNA region. A DNA-guided effector protein, such as a protein from the RecA family may be used to target DNA. Examples of proteins from the RecA family that may be used are RecA, RadA and Rad51.

The nuclease activity of the RNA-guided endonuclease may be partially disabled. One or more of the catalytic nuclease sites of the RNA-guided endonuclease may be inactivated, provided that the enzyme retains the ability to cut at least one strand of the target polynucleotide. For example, where the RNA-guided endonuclease comprises two catalytic nuclease sites, one of the catalytic sites may be inactivated. Typically one of the catalytic sites will cut one strand of the polynucleotide to which it specifically binds and the other catalytic site will cut the opposite strand of the polynucleotide. Therefore, the RNA-guided endonuclease may cut both strands or one strand of a double stranded region of a target polynucleotide.

A polynucleotide-guided endonuclease that is capable of cutting only one strand of a double stranded target polynucleotide may be referred to as a nickase. A nickase typically produces a single stranded break in the target polynucleotide. Two nickases may be used to produce a cut end with an overhang where a first nickase cuts one strand of the target polynucleotide and a second nickase cuts the other strand of the target polynucleotide. For example, the nickases may be partially inactivated versions of the same endonuclease, wherein in one nickase a first catalytic site has been inactivated and in the other nickase a second catalytic site has been inactivated. In an exemplary embodiment of this, the first nickase may be a Cas9 endonuclease in which the RuvC domain is inactivated and the second nickase may be a Cas9 endonuclease in which the HNH domain is inactivated. The first and second nickases may be guided by different guide polynucleotide so that the nickases cut at different places in the double stranded target polynucleotide such that a cut end with an overhang of the desired length is produced.

Catalytic sites of a RNA-guided endonuclease may be inactivated by mutation. The mutation may be a substitution, insertion or deletion mutation. For example, one or more, such as 2, 3, 4, 5, or 6 amino acids may be substituted or inserted into or deleted from the catalytic site. The mutation is preferably a substitution or insertion, more preferably a substitution of a single amino acid at the catalytic site. The skilled person will be readily able to identify the catalytic sites of a RNA-guided endonuclease and mutations that inactivate them. For example, where the RNA-guided endonuclease is Cas9, one catalytic site may be inactivated by a mutation at D10 and the other by a mutation at H640.

Where the effector protein is a nickase, the method may further comprise adding an enzyme with 5' to 3' or 3' to 5' exonuclease activity to the sample to remove nucleotides adjacent to one side of the nick in the nicked strand of the target polynucleotide to expose a stretch of single stranded polynucleotide to which an adapter, such as an adapter comprising a single stranded portion (typically 3') comprising a universal sequence, can hybridise. A polymerase may be used to close any gap between the end of the adapter (typically 3') and the end of the double stranded region of the target polynucleotide (typically 5') prior to covalent attachment, such as ligation of the adapter to the target polynucleotide.

### Guide polynucleotide

The guide polynucleotide comprises a sequence that is capable of hybridising to a target polynucleotide and is also capable of binding to a polynucleotide-guided effector protein. The guide polynucleotide may have any structure that enables it to bind to the target polynucleotide and to a polynucleotide-guided effector protein.

The guide polynucleotide typically hybridizes to a sequence of about 20 nucleotides in the target polynucleotide. The sequence to which the guide RNA binds may be from about 10 to about 40, such as about 15 to about 30, preferably from about 18 to about 25 nucleotides, such as 21, 22, 23 or 24 nucleotides. The guide polynucleotide is typically complementary to a portion of one strand of a double stranded region of the target polynucleotide.

The guide RNA may be complementary to a region in the target polynucleotide that is 5' or 3' to a PAM. This is preferred where the target polynucleotide comprises DNA, particularly where the RNA effector protein is Cas9 or Cpf1. The guide RNA may be complementary to a region in the target polynucleotide that is flanked by a guanine. This is preferred where the target polynucleotide comprises RNA, particularly where the RNA effector protein is C2c2.

The guide RNA may have any structure that enables it to bind to the target polynucleotide and to a RNA-guided effector protein. The guide RNA may comprise a crRNA that binds to a sequence in the target polynucleotide and a tracrRNA. The tracrRNA typically binds to the RNA-guided effector protein. Typical structures of guide RNAs are known in the art. For example, the crRNA is typically a single stranded RNA and the tracrRNA typically has a double stranded region of which one strand is attached to the 3' end of the crRNA and a part that forms a hairpin loop at the 3' end of the strand that is not attached to the crRNA. The crRNA and tracrRNA may be transcribed in vitro as a single piece sgRNA.

The guide RNA may comprise other components, such as additional RNA bases or DNA bases or other nucleobases. The RNA and DNA bases in the guide RNA may be natural bases or modified bases. A guide DNA may be used in place of a guide RNA, and a DNA-guided effector protein used instead of a RNA-guided effector protein. The use of a guide DNA and a DNA-guided effector protein may be preferred where the target polynucleotide is RNA.

Customised guide polynucleotides are commercially available, for example from Integrated DNA Technologies (IDT).

The method may comprise contacting the sample of polynucleotides with multiple guide polynucleotides. For example, from 1 to 100, such as 2 to 50, for example 4, 6, 8, 10, 20 or 30 guide polynucleotides may be used. The multiple guide polynucleotides may bind to sequences at different sites in the same target polynucleotide, for example at the ends of (flanking) a region of interest in the target polynucleotide, or such that coverage of all of or a long length of the target polynucleotide can be obtained by generating fragments of the target polynucleotide to which adapters can be attached. The fragments may be distinct or overlapping fragments. The multiple guide polynucleotides may bind to sequences in different target polynucleotides.

In one embodiment, the method may utilise two guide polynucleotides designed so that one guide polynucleotide directs a nickase to cut one strand of a double stranded target polynucleotide and the other guide polynucleotide guides a nickase to cut the other strand of the double stranded polynucleotide. In this way opposing cut ends each with an overhang may be produced. The method may utilise two or more pairs of such guide polynucleotides to produce cut ends with overhangs at two or more in a target polynucleotide.

In one embodiment, the cut site may include one or more of the terminal 20 nucleotides of a region of interest in the target polynucleotide and/or may be within from 0 to 50 nucleotides of the end of the region of interest in the target polynucleotide, such as from 1 to 40, 5 to 30 or 10 to 20 nucleotides.

In one embodiment the polynucleotide-guided effector protein cuts at one site in the target polynucleotide.

In another embodiment, the polynucleotide-guided effector protein cuts at two or more sites in the target polynucleotide. In this embodiment, the two sites are preferably at the ends of the target polynucleotide or at the ends of a region of interest in the target polynucleotide. Hence, the method may comprise contacting a sample of polynucleotides with two or more guide polynucleotides, wherein a first guide polynucleotide binds to a sequence near one end of the target polynucleotide and a second guide polynucleotide binds to a sequence near the other end of the target polynucleotide, or wherein a first guide polynucleotide binds to a sequence near one end of the region of interest and a second guide polynucleotide binds to a sequence near the other end of the region of interest. Alternatively, the method may comprise contacting a sample of polynucleotides with two or more pairs of guide polynucleotides, wherein a first pair directs a pair of nickases to cut at one end of the target polynucleotide, or region of interest, and a second directs a pair of nickases to cut at the other end of the target polynucleotide, or region of interest.

In one embodiment, three or more sites, for example 4, 5, 6, 7, 8, 9, 10 or more sites, within a target polynucleotide are cut. The method may, for example, involve using three guide polynucleotides, or three pairs of guide polynucleotides, wherein one binds to a sequence within the target polynucleotide, or region of interest, and the other two bind to sequences at the ends of the target polynucleotide, or region of interest.

The guide polynucleotides may be designed such that the action of the polynucleotide-guided effector proteins cuts out the region of interest from a longer polynucleotide or such that it cuts out the entire target polynucleotide. For example, the method may utilise two guide polynucleotides, or two pairs of guide polynucleotides, wherein one guide polynucleotide, or one pair of guide polynucleotides, binds to a site at one end of the target polynucleotide and the other guide polynucleotide or pair of guide polynucleotides binds to a site at the other end of the target polynucleotide.

The guide polynucleotide may be bound to the polynucleotide-guided effector protein, i.e. the guide polynucleotide and polynucleotide-guided effector protein may form a complex which may be referred to as a ribonucleoprotein (RNP). Conditions for forming RNPs are well know in the art. For example, an equimolar pool of crRNA may be annealed to tracrRNA at about 95°C for about 5 minutes to form the guide polynucleotide which is then cooled to room temperature before adding the polynucleotide-guided effector protein and incubating for at least about 10 minutes to allow the polynucleotide-guided effector protein to bind to the guide polynucleotide. The complex comprising the guide polynucleotide and the polynucleotide-guided effector protein may be added to the sample. Where the method uses two or more different guide polynucleotides each may be complexed with a polynucleotide-guided effector protein. The method may therefore comprise adding two or more, for example 3, 4, 5, 7, 8, 9, 10 or more, such complexes to the sample.

Where the method uses two or more guide polynucleotides that bind to sequences in two or more different target polynucleotides, the guide polynucleotides may be used to attach adapters within or flanking at least one region of interest in each of the target polynucleotides.

### Cut end

In the method, the polynucleotide-guided effector protein cuts the target polynucleotide to produce two opposing cut ends. The polynucleotide-guided effector protein and guide polynucleotide are typically incubated with the dephosphorylated sample of polynucleotides at a temperature of about 20°C to about 40°C, such as about 30°C, preferably about 37°C for a period of about 15 minutes to about an hour or more, such as about 30 minutes. The reaction conditions including for example the amount of sample, the effector protein concentration, the incubation temperature and the incubation time period can be adjusted as appropriate.

The polynucleotide-guided effector protein typically cuts the target polynucleotide in a double stranded region to produce two opposing cut ends. The opposing cut ends may be in just one strand of the double stranded polynucleotide, for example, where the polynucleotide-guided effector protein is a nickase. The opposing cut ends may be in both strands of the double stranded polynucleotide. The opposing cut ends may be blunt ended, i.e. the polynucleotide-guided effector protein may cut both strands of the double stranded polynucleotide at the same point. Thus, in one embodiment, the polynucleotide-guided effector protein cuts both strands of a double stranded polynucleotide to produce a blunt end. In another embodiment, the polynucleotide-guided effector protein cuts both strands of a double stranded polynucleotide to produce a single stranded overhang. The opposing cut ends may each have a single stranded overhang, wherein the single stranded overhang on each end is a 5' overhang, or the single stranded overhang on each end is a 3' overhang. The single stranded overhangs are preferably 3' overhangs.

In one embodiment, the cut ends each comprise a single stranded overhang. The single stranded overhang may be produced by a single polynucleotide-guided effector protein, such as for example Cas12a (Cpf1). In another embodiment, the cut end comprising a single stranded overhang is produced by the action of two polynucleotide-guided effector proteins, wherein each protein cuts a different strand of the target polynucleotide. In the method, an adapter is attached to one or both of the cut ends produced by the effector protein(s). The overhang may be of any suitable length. Typically, the overhang comprises from 4 to 30, such as 5 to 25, 6 to 20, 7 to 15, 8 to 12 or 9 to 10 nucleotides.

The sequence of the overhang may be known or unknown. The guide polynucleotide may be directed to a particular, known sequence in the target polynucleotide. The site at which the polynucleotide-guided effector protein cuts on target will be known so that the sequence of the overhang is predetermined. An adapter may therefore be designed such that it has a single stranded region, such as a single stranded overhang on the opposite strand to the overhang on the cut end to which it is wished to bind the adapter, wherein the sequence of the single stranded region in the adapter is complementary to the sequence in the overhang of the cut end. The overhang of the cut end of the target polynucleotide is capable of hybridizing to the single stranded region, such as the overhang, of the adapter.

In one embodiment, the sequence of the overhang in the adapter is exactly complementary to the sequence in the cut end. It is possible that there may be one or more base pair mismatches between the two overhang sequences. For example, there may be from 1 to 4 base pair mismatches, such as two or three base pair mismatches. Typically however, there will be at least 4, such as from 5 to 20, 6 to 15 or 8 to 10 matched bases between the two overhang sequences.

In one embodiment the adapter may be missing a 5' phosphate. This can help prevent the adapters self ligating.

In one embodiment, the sequence of the single stranded overhang in the adapter is a universal sequence. The universal sequence in the adapter may be from about 3 to about 15 nucleotides in length, such as from about 4, 5, 6 or 7 to about 12, 10 or 8 nucleotides in length. The universal sequence comprises universal nucleotides that can hybridise to any polynucleotide sequence in the overhang produced by cutting the double stranded polynucleotide.

A universal nucleotide is one which will hybridise to some degree to all of the nucleotides in the template polynucleotide. A universal nucleotide is preferably one which will hybridise to some degree to nucleotides comprising the nucleosides adenosine (A), thymine (T), uracil (U), guanine (G) and cytosine (C). A universal nucleotide may hybridise more strongly to some nucleotides than to others. For instance, a universal nucleotide (I) comprising the nucleoside, 2'-deoxyinosine, will show a preferential order of pairing of I-C > I-A > I-G approximately = I-T. It is only necessary that the universal nucleotides used in the adapter hybridise to all of the nucleotides in the double stranded polynucleotide. For example, when the double stranded polynucleotide is DNA, the universal nucleotides in the adapter need only bind to A, C, G and T.

A universal nucleotide may comprise one of the following nucleobases: hypoxanthine, 4-nitroindole, 5-nitroindole, 6-nitroindole, 3-nitropyrrole, nitroimidazole, 4-nitropyrazole, 4-nitrobenzimidazole, 5-nitroindazole, 4-aminobenzimidazole or phenyl (C6-aromatic ring. The universal nucleotide more preferably comprises one of the following nucleosides: 2'-deoxyinosine, inosine, 7-deaza-2'-deoxyinosine, 7-deaza-inosine, 2-aza-deoxyinosine, 2-aza-inosine, 4-nitroindole 2'-deoxyribonucleoside, 4-nitroindole ribonucleoside, 5-nitroindole 2'-deoxyribonucleoside, 5-nitroindole ribonucleoside, 6-nitroindole 2'-deoxyribonucleoside, 6-nitroindole ribonucleoside, 3-nitropyrrole 2'-deoxyribonucleoside, 3-nitropyrrole ribonucleoside, an acyclic sugar analogue of hypoxanthine, nitroimidazole 2'-deoxyribonucleoside, nitroimidazole ribonucleoside, 4-nitropyrazole 2'-deoxyribonucleoside, 4-nitropyrazole ribonucleoside, 4-nitrobenzimidazole 2'-deoxyribonucleoside, 4-nitrobenzimidazole ribonucleoside, 5-nitroindazole 2'-deoxyribonucleoside, 5-nitroindazole ribonucleoside, 4-aminobenzimidazole 2'-deoxyribonucleoside, 4-aminobenzimidazole ribonucleoside, phenyl C-ribonucleoside or phenyl C-2'-deoxyribosyl nucleoside.

Where it is wished to attach an adapter to a cut end with a 5' overhang, the complementary or universal single stranded region is at the 5' end of a single stranded adapter, or is a single stranded 5' overhang on a double stranded adapter. For example, where the adapter has a universal overhang or a single stranded overhang complementary to the overhang of the cut end, if the overhang of the cut end is a 5' overhang on the top strand, the overhang of the adapter is a 5' overhang on the bottom strand, or vice versa. Alternatively, where it is wished to attach an adapter to a cut end with a 3' overhang, the universal or complementary single stranded region is at the 3' end of a single stranded adapter, or is a 3' overhang on a double stranded adapter. For example, where the overhang of the cut end is a 3' overhang on the bottom strand, the overhang of the adapter is a 3' overhang on the top strand, or vice versa.

The length of the overhang on the adapter is typically the same as the length of the overhang on the cut end. It is possible that one of the overhangs may be shorter than the other overhang. Typically, the overhangs are capable of hybridizing over a region of from 4 to 30, such as 5 to 25, 6 to 20, 7 to 15, 8 to 12 or 9 to 10 nucleotides. Where, after hybridization, there is a stretch of single stranded nucleotides, the gap may be filled, for example using a polymerase. Preferably, the lengths of the two complementary overhangs are identical, or the length of the overhang in the target sequence and the universal overhang are identical.

In an embodiment where the action of the polynucleotide-guided effector protein(s) results in a single stranded overhang, the method may comprise contacting the sample with a polymerase and dNTPs to fill in the overhang to produce a blunt end.

Where the adapter comprises a dT tail, the method may further comprise contacting the sample with a polymerase and dATP to add a dA tail to at least one of the cut ends in the target polynucleotide. The dA tail may be added to a blunt end or to an single strand overhang. As an alternative, where the adapter comprises a dA tail, the method may further comprise contacting the sample with a polymerase and dTTP to add a dT tail to at least one of the cut ends in the target polynucleotide. Similarly dG and dC could be used in place of dA and dT.

### Free cut ends for adapter attachment

After cutting the polynucleotide the polynucleotide-guided effector protein may remain bound to one side of the cut site, or may be released from the target polynucleotide. Where the polynucleotide-guided effector protein remains bound to one side of the cut site, binding of an adapter to the cut end on the side of the cut site to which the effector protein remains attached may be prevented. In this case there is a bias to addition of the adapter to the cut end on the side of the cut site to which the effector protein is not attached. Thus, in one embodiment of the method, the polynucleotide-guided effector protein remains attached to one of the two opposing cut ends and the adapter is attached to the other one of the two opposing cut ends.

The guide polynucleotide may be designed to direct the polynucleotide-guided effector protein to cut the polynucleotide and remain on the opposite side of the cut site to the region of interest. Guide polynucleotides may be designed to direct the polynucleotide-guided effector protein to cut the polynucleotide and remain on the opposite side of the cut site upstream of the region of interest and to cut the polynucleotide and remain on the opposite side of the cut site downstream of the region of interest. Typically the polynucleotide-guided effector protein remains attached to the PAM-distal side of the cut site, leaving the PAM-proximal side of the cut site accessible to a dA-tailing enzyme and/ore adapter attachment.

Polynucleotide-guided effector proteins do not cut at each targeted site 100% of the time. The inventors have devised a method to increase the likelihood of a target polynucleotide being cut and adapted. The method may be used, for example, to ensure that an adapter is added at both sides of a region of interest. In this method, the guide polynucleotides are designed to direct polynucleotide-guided effector proteins to two or more, such as 3, 4, 5, 6 or more, sites in the same region of the target polynucleotide, typically wherein the polynucleotide-guided effector proteins are in the same orientation, e.g. so that after cutting the target polynucleotide the effector protein remains bound to the opposite side of the cut site to the region of interest. This means that adapters can be attached as desired in the event that the effector protein cuts the target polynucleotide at either one or both of the cut sites. The two cut sites in the same region may be located within about 10kb, 5kb, 1kb, 500 nucleotides or 100 nucleotides of each other, such as within about 90, 80, 70, 60, 50, 40, 30, 20 or 10 nucleotides of each other. Where there are cut sites at both sides of a defined region of interest, there may be two or more, such as 3, 4, 5, 6 or more, cut sites at either side of the region of interest. The cut sites in the same region of the target polynucleotide may be sites to which the same polynucleotide guided effector protein is directed, or sites to which different polynucleotide guided effector proteins, such as for example Cas9 and Cas12a (Cpf1), are directed.

Thus, provided is a method for selectively adapting a target polynucleotide in a sample of polynucleotides, the method comprising: contacting the polynucleotides in the sample with two guide polynucleotides that bind to a sequences in the target polynucleotide and a polynucleotide-guided effector protein, wherein the sequences to which the two guide polynucleotides bind direct the polynucleotide-guided effector protein to two closely located sites, such that the polynucleotide-guided effector protein cuts the target polynucleotide at at least one of the two sites to produce two opposing cut ends; and attaching an adapter to one or both of the two opposing cut ends in the target polynucleotide.

The region of interest is a region of the target polynucleotide to be characterised, such as sequenced. The region of interest may be defined by targeted cut sites at its ends. The region of interest may be "open ended" in the sense that one end is defined by the position of a target cut site and the region of interest extends away from the target cut site in one or both directions. Characterisation of the region of interest in one particular direction away from the cut site can be biased by designing the guide polynucleotide such that the effector protein remains attached to the opposite side of the cut site to the side it is wished preferentially to characterise, e.g. the region of interest.

The target polynucleotide may comprise a polymorphism, such as for example a SNP. In one embodiment, the guide polynucleotide/polynucleotide guided effector protein may be designed to target the site of a polymorphism, such as a SNP, and may only bind to and cut the target polynucelotide in the presence (or absence) of the polymorphism. The guide polynucleotide/polynucleotide guided effector protein may alternatively be designed to cut the target polynucleotide such that the region containing the polymorphism can be characterised, e.g. so that the region of interest is the region that may or may not include the polymorphism.

Where the polynucleotide guided effector protein cuts to leave blunt ends in the target polynucleotide, the ends may be modified to facilitate adapter ligation. For example, where the adapter has a dT tail, such as a single or polyT tail, the cut ends may be dA-tailed, for example to add a single dT or a polyT tail. Methods for adding a dA tail to a blunt end are known in the art. Any suitable method may be used. In one embodiment a dA tail is added using a polymerase. The polymerase may, for example, be a heat resistant or thermostable polymerase. The heat resistant polymerase or thermostable polymerase typically remains stable at temperatures over about 50°C, about 60°C, about 70°C about 75°C or about 80°C. Typically, the heat resistant polymerase or thermostable polymerase has polymerase activity at temperatures over about 50°C, about 60°C, about 70°C, about 75°C or about 80°. For example, the heat resistant polymerase or thermostable polymerase may be Taq polymerase. Where Taq polymerase is used, the dA tail may be added at a temperature of about 72°C, for example.

Prior to dA tailing the cut sites, the effector protein may be inactivated. Typically inactivation may be achieved by heating the sample, for example to at least about 50°C, about 60°C, about 70°C, about 75°C or about 80°C. The sample may be heated to inactivate the effector protein for about 2 minutes to about 20 minutes, such as about 5 minutes to about 15 minutes or about 10 minutes. Where a heat resistant polymerase or thermostable polymerase is used for dA tailing, it may be added prior to heat inactivation of the effector protein. For example, the heat stable polymerase may be added to the sample at the same time as the polynucleotide-guided effector protein. In this embodiment, the dA tail can be added to the cut sites during the effector protein inactivation step. Where a polymerase that is not active at the temperature used to inactivate the effector protein is used for dA tailing, e.g. a mesophilic polymerase, after heat inactivation the sample is typically cooled to the temperature at which the polymerase used for dA tailing is optimally active, such as for example about 37°C or room temperature, prior to adding the polymerase to the sample. Alternatively, the mesophilic polymerase may be added to the sample at the same time as the polynucleotide-guided effector protein such that it is active concomitantly with the polynucleotide-guided effector protein. However, in this embodiment the number of ends which are accessible for dA tailing may be less than when dA tailing is carried out after heat inactivation of the effector protein. An example of a suitable mesophilic polymerase is a Klenow fragment, such as 3'-5' exo- Klenow, an exonuclease mutant of *E. coli* DNA Polymerase I.

In one embodiment of the method, the polynucleotide-guided effector protein is removed from the target polynucleotide. In another embodiment of the method, the polynucleotide-guided effector protein does not remain attached to the target polynucleotide.

Heat inactivation of the effector protein may aid dissociation of the effector protein from the target polynucleotide and hence increase the number of cut ends accessible for dA tailing and/or adapter attachment, and in particular, facilitate attachment of adapters to both of the two opposing ends formed at a cut site. The effector protein is typically denatured in this step.

The sample may, in one embodiment, be deproteinised to remove any effector proteins that remain bound to the target polynucleotide after cutting. For example, a proteinase may be added to the sample after the sample has been incubated with the effector protein for a sufficient period, either before or after heat inactivation of the effector protein. Typically the deproteinising step is carried out before adding a polymerase to carry out a dA tailing step. The aim of the deproteinisation step is to release bound effector proteins so that adapters can be attached to both of the opposing cut ends formed by the action of the effector protein.

In some instances, the effector protein may be released from the target polynucleotide after cutting, for example where the effector protein is Cas12a (Cpf1) or a homologue of *S*. *pyogenes* Cas9. In this case, deproteinisation is not required in order to attach adapters to both of the two opposing ends at the cut site. Heat inactivation of the effector protein may also not be necessary.

The method may comprise contacting the polynucleotides in the sample with one or more guide polynucleotides that bind to one or more target polynucleotide. The one or more guide polynucleotides may bind to a target polynucleotide within a region of interest, or outside a region of interest. Thus, the method may comprise adding two or more, for example 3, 4, 5, 7, 8, 9, 10, 20, 50, 100, 200, 300, 400, 500, 1000, 5000, 10,000 or 100,000 or more, guide polynucleotides to the sample of polynucleotides. The guide polynucleotides may be targeted to one, two or more, such as, for example, 3, 4, 5, 7, 8, 9, 10, 50, 100, 500, 1000, 10,000 or 100,000 or more, target polynucleotides.

When a sample of polynucleotides is contacted with two or more guide polynucleotides that bind to different sequences in a target polynucleotide, the polynucleotide-guided effector protein may cut the target polynucleotide at two or more sites to produce two opposing cut ends at each site. In one embodiment, at least one of the two or more sites is located on a first side of the region of interest in the target polynucleotide, at least one of the two or more sites is located on a second side of the region of interest in the target polynucleotide, and none of the two or more sites is located within the region of interest.

The guide polynucleotides may be orientated such that, after cutting the target polynucleotide at the sites located on each side of the region of interest, the polynucleotide-guided effector protein remains attached to the cut end of the polynucleotide that does not contain the region of interest. In this way an adapter can be added to both ends of the polynucleotide comprising the region of interest without relying on the polynucleotide-guided effector protein falling off the target polynucleotide, or including a step to actively remove the polynucleotide-guided effector protein.

In one embodiment, the two or more sites targeted by guide polynucleotides comprise at least two sites on either side of a region of interest in the target polynucleotide. In one embodiment, the same polynucleotide-guided effector protein is used to cut at all of the two or more sites. In another embodiment, different polynucleotide-guided effector proteins are used to cut at the two or more sites. For example, where there are at least two sites targeted by guide polynucleotides on either side of a region of interest, one of the sites on a first side of the region of interest may be targeted by a first guide polynucleotide and a first polynucleotide-guided effector protein and another of the sites may be targeted by a second guide polynucleotide and a second polynucleotide-guided effector protein.

The read bias resulting from the effector protein remaining bound to one side of the cut site may be increased or decreased to improve the directionality of the reads or to increase the number of bidirectional reads as desired. In some embodiments, the bias may be reduced by heat inactivating (denaturing) the effector protein and/or by deproteinising the sample.

In some embodiments, the bias may be reduced by treating the cleaved polynucleotide, typically DNA, with RNAaseH. RNAaseH cleaves the RNA in a RNA/DNA substrate. The RNAaseH treatment may be carried out before or after deproteinisation or heat inactivation of the effector protein, preferably afterwards, or may be carried out in the absence of a proteinisation or heating inactivation step. The RNAase is typically added to the sample prior to dA tailing and adapter ligation.

In some embodiments, the bias may be increased by treating the cleaved polynucleotide an enzyme having 3'-5' exonuclease activity. One example of such an enzyme is a polymerase comprising an exonuclease domain that possesses 3'-5' exonuclease activity. The polymerase is typically added in the absence of dNTPs so that it does not have polymerase activity. Another example of such an enzyme is a 3'-5' exonuclease. Preferably, the enzyme having 3'-5' exonuclease activity does not have 5'-3' exonuclease activity. Examples of suitable enzymes having 3'-5' exonuclease activity include, but are not limited to Exonuclease I, Exonuclease III, Exonuclease T, T4 DNA polymerase, E. coli DNA polymerase I, phi29 DNA polymerase and T7 DNA polymerase. The polymerase may be added before or after deproteinisation or heat inactivation of the effector protein, preferably afterwards, or deproteinisation or heat inactivation steps may be absent from the method. The polymerase is typically added to the sample prior to dA tailing and adapter ligation.

### Attaching an adapter

The adapter may be hybridised to one or more cut ends, or one or more modified cut end, such as, for example, a cut end that has been dA tailed.

If the adapter hybridises to the target polynucleotide such that there is a gap between the terminal end (e.g. the 3' end) of the adapter and the terminal end (e.g. the 5' end) of the target polynucleotide strand hybridised to the target polynucleotide strand to which the adapter has also hybridised, the gap can be filled. This enables the terminal end (e.g. the 3' end) of the adapter and the terminal end (e.g. the 5' end) of the target polynucleotide to be covalently attached to each other.

Methods are known in the art for repairing single stranded gaps in the double stranded constructs. For instance, the gaps can be repaired using a polymerase and a ligase, such as DNA polymerase and a DNA ligase. Alternatively, the gaps can be repaired using random oligonucleotides of sufficient length to bridge the gaps and a ligase.

For example, a polymerase that acts in the 5' to 3' direction may be used to extend the end of the adapter after hybridisation of the adapter to the single stranded region to close the gap between the 3' end of the adapter and the 5' end of the flanking double stranded DNA. Suitable polymerases that act in the 5' to 3' direction include Taq polymerase, *E. coli* DNA polymerase I**,** Klenow fragment, Bst DNA polymerase, M-MuLV reverse transcriptase, phi29 polymerase, T4 DNA polymerase, T7 DNA polymerase, Vent and Deep Vent DNA polymerase.

The method may further comprise covalently attaching the adapter to the double stranded polynucleotide. Typically the 3' terminal nucleotide of the adapter is covalently attached to the 5' terminal nucleotide adjacent to the single stranded region. The covalent attachment may be achieved by any suitable means, for example by ligation or click chemistry.

Thus, the method may further comprise covalently attaching, for example ligating the adapter to the double stranded polynucleotide. For example, a ligase, such as for example T4 DNA ligase, may be added to the sample to ligate the adapter to the double stranded polynucleotide. The adapter may be ligated to the double stranded polynucleotide in the absence of ATP or using gamma-S-ATP (ATPγS) instead of ATP. Examples of ligases that can be used include T4 DNA ligase, *E. coli* DNA ligase, Taq DNA ligase, Tma DNA ligase and 9°N DNA ligase. The adapter may be attached using a topoisomerisase. The topoisomerase may, for example be a member of any of the Moiety Classification (EC) groups 5.99.1.2 and 5.99.1.3.

### Adapter

The adapter may typically comprise a 3' portion, or region, and a 5' portion, or region. The 3' portion of the adapter comprises a 3' stretch of single stranded polynucleotide that hybridises to the exposed stretch of single stranded polynucleotide in the double stranded polynucleotide.

The 3' stretch of single stranded polynucleotide in the adapter may be from about 1, 2 or 3 to about 15 nucleotides in length, such as from about 4, 5, 6 or 7 to about 12, 10 or 8 nucleotides in length.

In one embodiment, the 3' stretch of single stranded polynucleotide in the adapter comprises universal nucleotides that can hybridise to any polynucleotide sequence in the exposed stretch of single stranded polynucleotide in the double stranded polynucleotide.

In one embodiment, the 3' stretch of single stranded polynucleotide in the adapter comprises a sequence that is at least about 80%, such as at least about 90% or 95%, complementary to a polynucleotide sequence which is exposed in a single stranded overhang in a targeted cut site. For example, the 3' stretch of single stranded polynucleotide in the adapter may comprise a sequence that is exactly complementary to a polynucleotide sequence in the exposed stretch of single stranded polynucleotide in the double stranded polynucleotide.

In one embodiment, the 3' stretch of single stranded polynucleotide in the adapter hybridises to the exposed stretch of single stranded polynucleotide in the double stranded polynucleotide such that nucleotide at the 3' terminus of the 3' portion of the adapter hybridises to the nucleotide at the 5' end of the single stranded overhang.

The 3' stretch of single stranded polynucleotide in the adapter may be the same length as the single stranded overhang in a target polynucleotide, or the 3' stretch of single stranded polynucleotide in the adapter may be shorter than the length of the overhang in a target polynucleotide.

The 5' portion of the adapter does not hybridise to the target polynucleotide. The 5' portion may be double stranded or single stranded. Typically the 5' portion is single stranded or comprises a single stranded region. The single stranded region in the 5' portion of the adapter may, for example, be used to attach the adapter to a further polypeptide, such as a sequencing, or other, adapter, or a primer.

The 5' portion may have a length of, for example, from about 3 to about 45 nucleotides, such as about 6, 8, 10 or 15 to about 30, 25 or 20 nucleotides. The single stranded region of the 5' portion, which may be all of the 5 portion, is typically at least about 3, 6, 8, 10 or 15 nucleotides in length.

The adapter typically has a length of from about 10 to about 50 or about 60 nucleotides, such as from about 15 to about 40 or about 20 to about 30 nucleotides.

In one embodiment, the adapter is or comprises a single stranded polynucleotide. The single stranded polynucleotide may have a 3' portion that is designed to hybridise, e.g. is complementary, to the sequence that will be exposed in a targeted cut site in a target polynucleotide, e.g. in a 5' overhang, when the target polynucleotide is cut by a polynucleotide-guided effector protein at the cut site. The adapter may be present in a library of single stranded polynucleotide. The library may comprise single stranded polynucleotide designed to hybridise to multiple different cut sites in one or more target polynucleotide. In this embodiment, the single stranded polynucleotides may be referred to as barcodes. Each single stranded polynucleotide in the library may have a common sequence to which a complementary strand may be hybridised to produce an adapter comprising a 5' or central double stranded portion. Where the single stranded polynucleotides in the library have sequences that are exactly complementary to the sequence that will be exposed in a targeted cut site in a target polynucleotide, e.g. in a 5' overhang, when the target polynucleotide is cut by a polynucleotide-guided effector protein at the cut site, the single stranded polynucleotides may be considered to be specific barcodes. Where the single stranded polynucleotides in the library have sequences that are only partially complementary to the sequence that will be exposed in a targeted cut site in a target polynucleotide, e.g. in a 5' overhang, when the target polynucleotide is cut by a polynucleotide-guided effector protein at the cut site, the single stranded polynucleotides may be considered to be generic barcodes.

In one embodiment, the adapter comprises a double stranded polynucleotide, wherein the two strands are hybridised in a central region and one strand of the double stranded polynucleotide comprises a 3' portion comprising a first single stranded overhang. The first single stranded overhang may comprise a first sequence that is complementary to the sequence of an overhang produced when the polynucleotide-guided effector protein cuts a target polynucleotide, or the first single stranded overhang may comprise, for example, a dT tail that can hybridise to a dA tail.

The adapter may comprise a second single stranded overhang having a sequence at the opposite side of the central region to the first single stranded overhang, wherein the second sequence is different to the first sequence. The second single stranded overhang may be in the same strand as the first single stranded overhang, or may be in the opposite strand to the first single stranded overhang. The second single stranded overhang may have a length of from 1, 2, 3 or 4 to 30, such as 5 to 25, 6 to 20, 7 to 15, 8 to 12 or 9 to 10 nucleotides. The second single stranded overhang may be a 5' overhang or a 3' overhang. In one embodiment, the method further comprises attaching a further adapter to an adapter attached to a cut end in the target polynucleotide by hybridising the further adapter to the second single stranded overhang sequence.

The adapter is typically a polynucleotide and may comprise DNA, RNA, modified DNA (such as a basic DNA), RNA, PNA, LNA, BNA and/or PEG. The adapter preferably comprises single stranded and/or double stranded DNA and/or RNA.

The adapter may further comprise a chemical group (e.g. click chemistry) for attachment of the 5' portion of the adapter to a further adapter and/or a chemical group (e.g. click chemistry) for attachment of the 3' portion of the adapter to the double stranded polynucleotide.

The adapter may further comprise a reactive group in the 3' portion and/or in the 5' portion. The reactive group in the 3' portion may be used to covalently attach the adapter to the double stranded polynucleotide and/or the reactive group in the 5' portion may be used to covalently attach the adapter to a further adapter.

The reactive group may be used to ligate the fragments to the overhangs using click chemistry. Click chemistry is a term first introduced by Kolb *et al.* in 2001 to describe an expanding set of powerful, selective, and modular building blocks that work reliably in both small- and large-scale applications (Kolb HC, Finn, MG, Sharpless KB, Click chemistry: diverse chemical function from a few good reactions, Angew. Chem. Int. Ed. 40

(2001) 2004-2021). They have defined the set of stringent criteria for click chemistry as follows: "The reaction must be modular, wide in scope, give very high yields, generate only inoffensive by-products that can be removed by non-chromatographic methods, and be stereospecific (but not necessarily enantioselective). The required process characteristics include simple reaction conditions (ideally, the process should be insensitive to oxygen and water), readily available starting materials and reagents, the use of no solvent or a solvent that is benign (such as water) or easily removed, and simple product isolation. Purification if required must be by non-chromatographic methods, such as crystallization or distillation, and the product must be stable under physiological conditions".

Suitable examples of click chemistry include, but are not limited to, the following:
(a) copper-free variant of the 1,3 dipolar cycloaddition reaction, where an azide reacts with an alkyne under strain, for example in a cyclooctane ring;
(b) the reaction of an oxygen nucleophile on one linker with an epoxide or aziridine reactive moiety on the other; and
(c) the Staudinger ligation, where the alkyne moiety can be replaced by an aryl phosphine, resulting in a specific reaction with the azide to give an amide bond.

Any reactive group may be used in the invention. The reactive group may be one that is suitable for click chemistry. The reactive group may be any of those disclosed in WO 2010/086602, particularly in Table 4 of that application.

In one embodiment, the adapter attached to the cut site may be a sequencing adapter. The adapter may be ligated to a cut end of the target polynucleotide. The adapter may be ligated to the target polynucleotide in the absence of ATP or using gamma-S-ATP (ATPγS) instead of ATP. It is preferred that the adapter is ligated to the polynucleotide in the absence of ATP where the adapter is a sequencing adapter to which a nucleic acid handling enzyme is bound.

Where the method involves cutting at two or more sites, which may be in the same target polynucleotide or in different target polynucleotides, to produce single stranded overhangs, the overhangs produced at the cut ends may have different nucleotide sequences. In this embodiment, the method may comprise contacting the sample with multiple adapters, wherein different adapters comprise different single stranded polynucleotide sequences, which are typically overhang sequences. The different sequences in the different adapters are designed to hybridize to different overhang sequences produced by the action of the polynucleotide-guided effector protein on different target polynucleotides or at different sites in the same target polynucleotide.

In a method that utilises multiple adapters, wherein each adapter comprises a different first sequence, all of the adapters may comprise the same second sequence. In this embodiment, the second sequence may be used to further process all of the target polynucleotides to which an adapter has been attached in the same manner. For example, a further adapter comprising a single stranded polynucleotide capable of hybridizing to the second sequence in the 5' overhang on the first adapter may be attached to all of the target polynucleotides in the sample. The further adapter typically comprises a single stranded overhang having a sequence that is complementary to the second sequence in the first. The second sequence in the first adapter is capable of hybridizing to the complementary sequence in the overhang of the further adapter.

Where the first adapter is a single stranded polynucleotide adapter, the further adapter may hybridise to all or part of the single stranded adapter that forms an overhang when the first adapter binds to the cut end.

Preferably, the second sequence in the first adapter is exactly complementary to the overhang sequence in the further adapter. It is possible that there may be one or more base pair mismatches between the two overhang sequences. For example, there may be from 1 to 4 base pair mismatches, such as two or three base pair mismatches. Typically however, there will be at least 4, such as from 5 to 20, 6 to 15 or 8 to 10 matched bases between the two overhang sequences.

Where it is wished to attach a further adapter to a 5' overhang, the complementary single stranded region is preferably a 5' overhang on a double stranded further adapter. For example, if the overhang of the adapter exposed when it is bound to the cut end is a 5' overhang on the top strand, the overhang of the further adapter is a 5' overhang on the bottom strand, or vice versa. Alternatively, where it is wished to attach a further adapter to a 3' overhang, the complementary single stranded region is typically a 3' overhang on a double stranded adapter. For example, where the overhang of the adapter exposed when it is bound to cut end is a 3' overhang on the bottom strand, the overhang of the adapter is a 3' overhang on the top strand, or vice versa.

The length of the overhang on the further adapter is typically the same as the length of the overhang in the first adapter that is exposed when the first adapter is attached to the cut end. It is possible that one of the overhangs may be shorter than the other overhang. Typically, the overhangs are capable of hybridizing over a region of from 4 to 30, such as 5 to 25, 6 to 20, 7 to 15, 8 to 12 or 9 to 10 nucleotides. Where, after hybridization, there is a stretch of single stranded nucleotides, the gap may be filled, for example using a polymerase. Preferably, the lengths of the two complementary overhangs are identical.

The further adapter that is attached to the universal overhang may, for example, be a sequencing adapter. The sequencing adapter may be an adapter designed for sequencing methods that utilize a transmembrane pore.

The target polynucleotide may be sequenced from within a single cut site within the target polynucleotide. The whole target polynucleotide may be sequenced. Alternatively, only a region of interest within the target polynucleotide may be sequenced.

The adapter or the further adapter may be an adapter for characterising the target polynucleotide using a transmembrane pore. The adapter for characterising the target polynucleotide using a transmembrane pore preferably comprises a leader sequence, a polynucleotide binding protein and/or a membrane or pore anchor.

The first adapter and/or further adapter may comprise a single stranded polynucleotide to which a nucleic acid handling enzyme is bound.

An adapter or the further adapter may comprise a tag for binding to a bead.

The adapter is preferably synthetic or artificial. The adapter preferably comprises a polymer. The polymer is preferably a polynucleotide. The polynucleotide adapter may comprise DNA, RNA, modified DNA (such as a basic DNA), RNA, PNA, LNA, BNA and/or PEG. The adapter more preferably comprises DNA or RNA.

The first adapter or the further adapter may be a sequencing adapter. The sequencing adapter may be a Y adapter. A Y adapter is typically a polynucleotide adapter. A Y adapter is typically double stranded and comprises (a) a region where the two strands are hybridised together and (b) an end region where the two strands are not complementary. The non-complementary parts of the strands form overhangs. The presence of a non-complementary region in the Y adapter gives the adapter its Y shape since the two strands typically do not hybridise to each other unlike the double stranded portion. The double-stranded portion preferably has a length of from 5 to about 50, such as 6 to about 30, 7 to about 20, 8 to 15, or 9 to about 12 nucleotides base pairs. The overhang regions preferably have lengths of from 5 to about 50, such as 6 to about 30, 7 to about 20, 8 to 15, or 9 to about 12 nucleotides.

One of the non-complementary strands Y adapter typically comprises a leader sequence, which when contacted with a transmembrane pore is capable of threading into the pore. The leader sequence typically comprises a polymer. The polymer is preferably negatively charged. The polymer is preferably a polynucleotide, such as DNA or RNA, a modified polynucleotide (such as abasic DNA), PNA, LNA, polyethylene glycol (PEG) or a polypeptide. The leader preferably comprises a polynucleotide and more preferably comprises a single stranded polynucleotide. The single stranded leader sequence most preferably comprises a single strand of DNA, such as a poly dT section. The leader sequence preferably comprises the one or more spacers.

The leader sequence can be any length, but is typically 10 to 150 nucleotides in length, such as from 20 to 120, 30 to 100, 40 to 80 or 50 to 70 nucleotides in length.

A nucleic acid handling enzyme may be bound to an overhang, which is preferably a overhang comprising a leader sequence, and/or to the double stranded region. The enzyme is preferably stalled, typically by or at a spacer. Any configuration of enzymes and spacers disclosed in WO 2014/135838 may be used. Preferred spacers include from 2 to 20, such as 4, 6, 8 or 12 iSpC3 groups, iSp18 groups or iSp9 groups, more preferably 4, 12 or 20 iSpC3 groups, 6 iSpC9 groups or 2 or 6 iSpC18 groups. One of the non-complementary strands Y adapter typically comprises a leader sequence, which when contacted with a transmembrane pore is capable of threading into the pore.

In one embodiment, the Y adapter comprises a membrane anchor or a pore anchor. The anchor may be attached to a polynucleotide that is complementary to and hence that is hybridised to the overhang to which an enzyme is not bound. The polynucleotide to which the anchor is attached is preferably from 5 to about 50, such as 6 to about 30, 7 to about 20, 8 to 15, or 9 to about 12 nucleotides in length.

The Y adapter typically comprises a further single stranded overhang at the opposite end of the hybridised region to the overhangs that give the adapter its Y shape. Where the first adapter is a Y adapter, the Y adapter comprises a single stranded region which is complementary to the overhang at the cut end of the target polynucleotide, and which is at the opposite end of the Y adapter to the end region where the two strands are not complementary. Where the further adapter is a Y adapter, the Y adapter comprises a single stranded overhang which is complementary to the overhang at the end of a first adapter attached to at the cut end of the target polynucleotide, and which is at the opposite end of the Y adapter to the end region where the two strands are not complementary.

In one embodiment, where an adapter is attached to a cut site at each end of a target polynucleotide, one of the adapters may be a hairpin loop adapter, or the further adapter added to a adapter at one of the two ends may be a hairpin loop adapter. A hairpin loop adapter is an adapter comprising a single polynucleotide strand, wherein the ends of the polynucleotide strand are capable of hybridising to each other, or are hybridized to each other, and wherein the middle section of the polynucleotide forms a loop. Suitable hairpin loop adapters can be designed using methods known in the art. The loop may be any length. The loop is preferably from about 2 to 400, from 5 to 300, from 10 to 200, from 20 to 100 nucleotides or from 30 to 50 in length. The double stranded section of the adapter formed by two hybridized sections of the polynucleotide strand is called a stem. The stem of the hairpin loop is preferably from 4 to 200, such as 5 to 150, 10 to 100, 20 to 90, 30 to 80, 40 to 70 or 50 to 60 nucleotide pairs in length. Where a nucleic acid handling enzyme is bond to or binds to a hairpin adapter, it typically binds to the loop of the hairpin, rather than to the stem.

In one embodiment, a Y adapter may be added to one end of a target polynucleotide and a hairpin loop adapter to the other end.

In one embodiment, the sequencing adapter, such as the Y adapter and/or hairpin adapter, further comprises a membrane anchor or pore anchor. Suitable anchors are known in the art, as described, for example, in WO 2012/164270 and WO 2015/150786. Preferably the anchor is a membrane anchor. Preferably the membrane anchor comprises cholesterol or a fatty acyl chain. For example, any fatty acyl chain having a length of from 6 to 30 carbon atom, such as hexadecanoic acid, may be used.

In one embodiment, the adapter or the further adapter comprises a barcode sequence. Polynucleotide barcodes are well-known in the art (Kozarewa, et al (2011) Methods Mol. Biol. 733: 279-298).

In an embodiment, the adapter or further adapter may comprise a sequence complementary to an amplification primer, such as a PCR primer or a primer for isothermal amplification. The method may further comprise amplifying a region of interest in a target polynucleotide using a pair of PCR sequences that hybridise to sequences within the adapters that flank the region of interest in the adapted polynucleotide. The method may further comprise amplifying a region of interest in a target polynucleotide using an one or more primers that hybridise to a sequence within an adapter attached to a target polynucleotide.

In one embodiment, the cleaved target polynucleotide may be amplified prior to adapter attachment. In this embodiment, an amplification adapter, such as a PCR adapter, is added to the dA tailed ends of the cleaved polynucleotide. An amplification reaction, such as PCR, is then carried out prior to addition of a sequencing adapter.

The amplification adapter, such as a PCR adapter, may be phosphorylated or dephosphorylated. Dephosphorylation of the amplification adapter is preferred in some embodiments. Amplification increases the number of target reads, for example by up to at least about 5%, at least about 10% or more.

In one embodiment, the effector protein(s) is/are targeted to cut sites on either side of a target polynucleotide such that amplification adapters (e.g. PCR adapters) are ligated to both ends of the target polynucleotide, which is then amplified using primers (e.g. PCR primers) that bind to an overhang on the amplification adapters (e.g. PCR adapters) ligated to the target DNA. The overhang is typically a 5' overhang that is complementary to the primer.

Thus, in one embodiment, the amplification primer (e.g. PCR primer) typically comprises a double stranded portion and a single stranded portion. The single stranded portion is typically a 5' overhang. The single stranded portion may, for example, have a length of from about 10 to about 100, such as from about 30 to about 80, or about 40 to about 60, such as about 50 nucleotides. All or part of the single stranded region is complementary to a primer for amplification, such as a PCR primer. The double stranded portion may have a blunt end. The blunt end may be ligated to a blunt ended cut site. Alternatively, the double stranded region may be central in the amplification adapter, and the amplification adapter may comprise a second single stranded region, wherein the second single stranded region is a 3' overhang. The 3' overhang is a 3' stretch of single stranded polynucleotide that may have the same features as the 3' stretch of single stranded polynucleotide of the adapter described above.

In an embodiment, the first adapter or further adapter may enable the targeted polynucleotides to be captured, for example by using a biotinylated first adapter or a biotinylated further adapter, or a first adapter or further adapter to which is attached another affinity molecule or a polynucleotide sequence that can bind to a capture strand. A signal may be attached to the first adapter or further adapter to enable the easy detection and/or identification of a target polynucleotide. The signal may, for example, be a molecular beacon or a fluorophore. In one embodiment the first adapter may comprise a quencher and the further adapter may comprise a fluorophore, or vice versa.

In an embodiment, the adapter may comprise a barcode sequence. Barcode sequences are known in the art. A barcode is a specific sequence of polynucleotide that produces a distinctive signal, for example by affecting the current flowing through the pore in a specific and known manner. The method may be a multiplex method for analysing multiple samples, wherein multiple adapters, each with a different barcode are utilised. For example, in one embodiment, multiple, such as for example from two to about 100 or more, such as about 5, about 10, about 20, or about 50, samples are analysed, wherein each sample is treated by a method as disclosed herein and wherein an adapter comprising a unique barcode is used for each sample tested. The products of the methods using the samples may be pooled after barcode-adapter ligation.

The barcodes may be comprised in intermediate adapters, for example amplification adapters, and/or in sequencing adapters. In an embodiment where the barcodes are in sequencing adapters, the products of the methods carried out on different samples may be pooled prior to, or after, attachment of the sequencing adapter.

### Adding sequencing adapter

In one embodiment, the method further comprises attaching a sequencing adapter to the 5' portion of the adapter that us attached to the cut site. Hence the adapter may act as a first adapter or an intermediate adapter.

The sequencing adapter may comprise a single stranded portion that hybridises to a stretch of single stranded polynucleotide in the 5' portion of the first adapter. The sequencing adapter may comprises a single stranded leader sequence, a polynucleotide binding protein and/or a membrane or pore anchor. The sequencing adapter may have any of the features of an adapter described above.

After hybridisation, the sequencing adapter may be covalently attached to the adapter using a ligase or by click chemistry. The ligase may, for example, be T4 DNA ligase, *E. coli* DNA ligase, Taq DNA ligase, Tma DNA ligase and 9°N DNA ligase. The adapter may be attached using a topoisomerisase. The topoisomerase may, for example be a member of any of the Moiety Classification (EC) groups 5.99.1.2 and 5.99.1.3. The sequencing adapter may be ligated to the target polynucleotide in the absence of ATP or using gamma-S-ATP (ATPγS) instead of ATP. It is preferred that the adapter is ligated to the polynucleotide in the absence of ATP where the a nucleic acid handling enzyme is bound to the sequencing adapter.

The sequencing adapter may be attached to the adapter after the adapter has been attached to the target polynucleotide. Hence the method may comprise a step of attaching a first adapter to a cut site in a target polynucleotide and a sequential step of attaching a sequencing adapter to the first adapter. Thus, the first (intermediate) adapter may be added to the sample prior to adding the sequencing adapter to the sample.

The sequencing adapter may be attached to the first adapter before the first adapter is attached to the target polynucleotide. Also, the method may comprise attaching a first adapter to the target polynucleotide and attaching a sequencing adapter to the first adapter in a single step. Thus, the sequencing adapter and the first (intermediate) adapter may be added to the sample at the same time.

The sequencing adapter may, in one embodiment, be added to the target polynucleotide after amplification of a target polynucleotide to which amplification adapters have been attached.

### Nucleic acid handling enzyme

The nucleic acid handling enzyme on the adapter may be any protein that is capable of binding to a polynucleotide and processing the polynucleotide. In processing the polynucleotide, the nucleic acid handling enzyme moves along the polynucleotide. The direction of movement of the enzyme is consistent. Consistent movement means that the enzyme moves from the 5' end to the 3' end of the polynucleotide or vice versa. The enzyme may modify the polynucleotide as it processes it. It is not essential that modification of the polynucleotide occurs. Therefore, the nucleic acid handling enzyme may be a modified enzyme that retains its ability to move along a polynucleotide.

The nucleic acid handling enzyme may be, for example, a translocase, a helicase, a polymerase or an exonuclease.

The nucleic acid handling enzyme may move along a single stranded polynucleotide, such as single stranded DNA or single stranded RNA, or may move along a double stranded polynucleotide such as double stranded DNA or a DNA/RNA hybrid. For example, helicases or translocases that act on either single stranded or double stranded DNA may be used. Examples of suitable helicases include Dda, Hel308, NS3 and TraI. These helicases typically work on single stranded DNA. Examples of helicases that can move along both strands of a double stranded DNA include FtfK and hexameric enzyme complexes such as RecBCD.

The helicase may be any of the helicases, modified helicases or helicase constructs disclosed in WO 2013/057495, WO 2013/098562, WO2013098561, WO 2014/013260, WO 2014/013259, WO 2014/013262 and WO/2015/055981. The Dda helicase preferably comprises any of the modifications disclosed in WO/2015/055981 and WO 2016/055777.

The nucleic acid handling enzyme may be a polymerase. A polymerase will typically synthesize a complementary polynucleotide strand as it moves along a polynucleotide. Otherwise, a polymerase may be used in a similar manner to a translocase. The polymerase may be a modified polymerase which retains its ability to move along a polynucleotide, but which does not synthesize a complementary strand. The polymerase may, for example, be PyroPhage^{®} 3173 DNA Polymerase (which is commercially available from Lucigen^{®} Corporation), SD Polymerase (commercially available from Bioron^{®}) or variants thereof. The enzyme is preferably Phi29 DNA polymerase or a variant thereof. The topoisomerase is preferably a member of any of the Moiety Classification (EC) groups 5.99.1.2 and 5.99.1.3.

The nucleic acid handling enzyme may be an exonuclease. An exonuclease typically digest the polynucleotide as it moves along it. The exonuclease typically cleaves one strand of a double stranded polynucleotide to form individual nucleotides or shorter chains of nucleotides, such as di- or tri-nucleotides. Where an exonuclease is used, the polynucleotides which are ultimately selected are the undigested strands of double stranded polynucleotide, or polynucleotides in which one of the strands is partially digested and the other strand is intact.

The nucleic acid handling enzyme is preferably one that is able to process long polynucleotide strands. Typically, the nucleic acid handling enzyme is capable of moving along a polynucleotide strand of from 500 nucleotide base pairs up to 250 million nucleotide base pairs, such as from 1,000, 2,000, 5,000, 10,000, 50,000 or 100,000 nucleotide base pairs up to 200 million, 100 million, 10 million or 1 million nucleotide base pairs.

The enzyme may be modified or unmodified. The enzyme may be modified to form a closed-complex. A closed-complex is an enzyme in which the polynucleotide binding site is modified such that the enzyme is closed around the polynucleotide in such a way that the enzyme does not fall off the polynucleotide other than when it reaches the end of the polynucleotide. Examples of suitable closed-complex enzymes and methods for modifying enzymes to produce closed complexes are disclosed in, for example, WO 2014/013260 and WO 2015/055981.

### Characterisation Method

A method of characterising a polynucleotide is provided. The method described above may further comprise characterising the target polynucleotide.

The method of detecting and/or characterising a target polynucleotide typically comprises:
(a) contacting modified polynucleotide sample obtained by a method described herein with a membrane comprising a transmembrane pore;
(b) applying a potential difference across the membrane; and
(c) monitoring for the presence or absence of an effect resulting from the interaction of the complex with the transmembrane pore to determine the presence or absence of the complex, thereby detecting the target polynucleotide in the sample and/or monitoring the interaction of the complex with the transmembrane pore to determine one or more characteristics of the target polynucleotide.

The method may involve measuring two, three, four or five or more characteristics of each polynucleotide. The one or more characteristics are preferably selected from (i) the length of the polynucleotide, (ii) the identity of the polynucleotide, (iii) the sequence of the polynucleotide, (iv) the secondary structure of the polynucleotide and (v) whether or not the polynucleotide is modified. Any combination of (i) to (v) may be measured in accordance with the invention, such as {i}, {ii}, {iii}, {iv}, {v}, {i,ii}, {i,iii}, {i,iv}, {i,v}, {ii,iii}, {ii,iv}, {ii,v}, {iii,iv}, {iii,v}, {iv,v}, {i,ii,iii}, {i,ii,iv}, {i,ii,v}, {i,i,iv}, {i,iii,v}, {i,iv,v}, {ii,iii,iv}, {ii,iii,v}, {i,iv,v}, {iii,iv,v}, {i,ii,iii,iv}, {i,ii,iii,v}, {i,ii,iv,v}, {i,iii,iv,v}, {ii,iii,iv,v} or {i,ii,iii,iv,v}.

The target polynucleotide is preferably characterised by sequencing.

For (i), the length of the polynucleotide may be measured for example by determining the number of interactions between the polynucleotide and the pore or the duration of interaction between the polynucleotide and the pore.

For (ii), the identity of the polynucleotide may be measured in a number of ways. The identity of the polynucleotide may be measured in conjunction with measurement of the sequence of the polynucleotide or without measurement of the sequence of the polynucleotide. The former is straightforward; the polynucleotide is sequenced and thereby identified. The latter may be done in several ways. For instance, the presence of a particular motif in the polynucleotide may be measured (without measuring the remaining sequence of the polynucleotide). Alternatively, the measurement of a particular electrical and/or optical signal in the method may identify the polynucleotide as coming from a particular source.

For (iii), the sequence of the polynucleotide can be determined as described previously. Suitable sequencing methods, particularly those using electrical measurements, are described in Stoddart D et al., Proc Natl Acad Sci, 12;106(19):7702-7, Lieberman KR et al, J Am Chem Soc. 2010;132(50):17961-72, and International Application WO 2000/28312.

For (iv), the secondary structure may be measured in a variety of ways. For instance, if the method involves an electrical measurement, the secondary structure may be measured using a change in dwell time or a change in current flowing through the pore. This allows regions of single-stranded and double-stranded polynucleotide to be distinguished.

For (v), the presence or absence of any modification may be measured. The method preferably comprises determining whether or not the polynucleotide is modified by methylation, by oxidation, by damage, with one or more proteins or with one or more labels, tags or spacers. Specific modifications will result in specific interactions with the pore which can be measured using the methods described below. For instance, methylcyotsine may be distinguished from cytosine on the basis of the current flowing through the pore during its interaction with each nucleotide.

The methods may be carried out using any apparatus that is suitable for investigating a membrane/pore system in which a pore is present in a membrane. The method may be carried out using any apparatus that is suitable for transmembrane pore sensing. For example, the apparatus comprises a chamber comprising an aqueous solution and a barrier that separates the chamber into two sections. The barrier typically has an aperture in which the membrane containing the pore is formed. Alternatively the barrier forms the membrane in which the pore is present. Transmembrane pores are known in the art. Suitable membranes and devices are also known, as are methods for analysing the current signal to determine sequence and other characteristics of the polynucleotides. The methods may be carried out using the apparatus described in WO 2008/102120. A variety of different types of measurements may be made. This includes without limitation: electrical measurements and optical measurements. A suitable optical method involving the measurement of fluorescence is disclosed by J. Am. Chem. Soc. 2009, 131 1652-1653. Possible electrical measurements include: current measurements, impedance measurements, tunnelling measurements (Ivanov AP et al., Nano Lett. 2011 Jan 12; 11(1):279-85), and FET measurements (International Application WO 2005/124888). Optical measurements may be combined with electrical measurements (Soni GV et al., Rev Sci Instrum. 2010 Jan; 81(1):014301). The measurement may be a transmembrane current measurement such as measurement of ionic current flowing through the pore.

The characterisation method typically comprises measuring the current passing through the transmembrane pore as the polynucleotide moves with respect to the transmembrane pore.

Beads may be used to facilitate delivery of the target polynucleotides to the pore, for example as disclosed in WO 2016/059375.

### Kits

Also provided is a kit for selectively modifying a target polynucleotide in a sample of polynucleotides. In one embodiment, the kit for selectively modifying a target polynucleotide in a sample of polynucleotides comprises a dephosphorylase, an adapter, and optionally one or more of a polymerase, a ligase, a polynucleotide-guided effector protein and a guide polynucleotide. The kit may further comprises one or more guide polynucleotides and/or one or more polynucleotide-guided effector proteins. The adapter in the kit may comprise a dN tail, such as a single N or a polyN tail, wherein N is the nucleotide A, T, C or G.

In one embodiment, the kit may comprise one or more first adapters together with one or more guide polynucleotides and/or one or more first adapters as described herein. The kit may further comprise one or more polynucleotide-guided effector proteins and/or one or more further adapters as defined herein.

In one embodiment, the kit may comprise: a guide polynucleotide that binds to a sequence in the target polynucleotide; a polynucleotide-guided effector protein capable of cutting the target polynucleotide to produce a cut ends comprising an overhang; and a first adapter comprising a central double-stranded region, a first single stranded region at one end having a first sequence that is complementary to the sequence of an overhang produced when the polynucleotide-guided effector protein cuts the target polynucleotide

The first adapter may be any of the adapters defined herein. The first adapter may optionally further comprise a second single stranded overhang at the other end of the adapter to the first single stranded overhang, wherein the second single stranded overhang has a second sequence that is different to the first sequence and the kit may comprise a further adapter comprising a single stranded region having a sequence that is complementary to the second sequence in the first adapter.

Also provided is a kit comprising: a first adapter comprising a central double-stranded region, a first single stranded region at one end having a first sequence that is complementary to the sequence of an overhang produced when the polynucleotide-guided effector protein cuts the target polynucleotide and a second single stranded region at the other end having a second sequence, wherein the second sequence is different to the first sequence; and a further adapter comprising a single stranded region having a sequence that is complementary to the second sequence in the first adapter.

The first adapter may be any of the adapters defined herein. The further adapter may be any of the further adapters defined herein.

In either of the above kit embodiments described above, the kit may comprise one or more, such as from 2 to 50, 3 to 40, 5 to 30 or 10 to 20, first adapters as described herein and one or more further adapter, such as from 2 to 50, 3 to 40, 5 to 30 or 10 to 20 further adapters as defined herein.

Preferably, the kit comprises a panel of first adapters, wherein each adapter has a different sequence in the first overhang region and the same sequence in the second overhang region. Where the first adapters in the panel have the same sequence in the second overhang region, the kit preferably comprises one type of further adapter.

### System

In one aspect, a system for selectively adapting a target polynucleotide in a sample of polynucleotides is provided, the system comprising:
(a) a means for protecting the ends of polynucleotides;
(b) a guide polynucleotide that binds to a sequence in a target polynucleotide;
(c) a polynucleotide-guided effector protein; and
(d) an adapter compatible with cut polynucleotide ends created by the polynucleotide-guided effector protein.

In one embodiment, the means for protecting the ends of polynucleotides is a dephosphorylase. The dephosphorylase protects the ends of the polynucleotides in the sample by dephosphorylating the 5' ends of the polynucleotides.

Also provided is a system for detecting the presence of a target polynucleotide in a sample, the system further comprising a nanopore, for example, a nanopore present in a membrane. In some embodiments the system comprises a flow cell compatible with a sequencing device or apparatus.

In the system, the polynucleotide-guided effector protein is, in some embodiments, an RNA-guided effector protein, such as Cas3, Cas4, Cas8a, Cas8b, Cas8c, Cas9, Cas10, Cas10d, Cas12a, Cas13, Csn2, Csf1, Cmr5, Csm2, Csy1, Cse1, C2c2, Cas14, CasX or CasY. In some embodiments, the polynucleotide-guided effector protein cuts one strand of a double stranded polynucleotide. In other embodiments, the polynucleotide-guided effector protein cuts both strands of a double stranded polynucleotide to produce a blunt end. In yet other embodiments, the polynucleotide-guided effector protein cuts both strands of a double stranded polynucleotide to produce a single stranded overhang.

In the system, in some embodiments, the adapter comprises a single N or polyN tail, wherein N is the nucleotide A, T, C or G. In one embodiment, the adapter comprises a single T or polyT tail. In one embodiment, the adapter is an intermediate adapter and the system further comprises a sequencing adapter comprising a portion complementary to the intermediate adapter. The sequencing adapter may, for example, a single stranded leader sequence, a polynucleotide binding protein and/or a membrane or pore anchor.

In one embodiment, the system comprises two or more guide polynucleotides that bind to different sequences in the target polynucleotide such that the polynucleotide-guided effector protein cuts the target polynucleotide at two or more sites to produce two opposing cut ends at each site.

In one embodiment, the system further comprises a pair of PCR primers complementary to sequences within the adapter.

In some embodiments, the system further comprises a polymerase and/or a ligase.

The following non-limiting Examples illustrate the invention.

### Example 1

This Example demonstrates how a single degenerate synthetic crRNA probe can be used to enrich for a duplicated region of a bacterial genome for nanopore sequencing. The enrichment occurs not by physical separation of target versus non-target DNA, but by protection and deprotection of DNA ends against adapter ligation by dephosphorylation and CRISPR/Cas9-mediated cleavage of the target region, respectively. Here is described a simple, one-pot approach, in which the enzymatic steps (dephosphorylation, Cas9-mediated cleavage, dA-tailing, and adapter ligation) are performed sequentially.

### Materials and Methods

High-molecular weight genomic DNA **("gDNA")** was purified by extraction from *Escherichia coli* (strain SCS110) using a Qiagen tip-500, according to the manufacturer's instructions. 5 µg gDNA was dephosphorylated via treatment with calf intestinal dephosphorylase. 2.5 µL Quick CIP (from 'NEB Quick CIP kit', New England Biolabs, Inc., Cat # M0508) was added to the 5 µg of gDNA in a total of 50 µL NEB CutSmart Buffer (New England Biolabs, Inc., Catalogue # B7204) for 10 min at 37°C, followed by heat inactivation of the dephosphorylase at 80°C for 2 min. This step yielded "end-**protected gDNA".**

Wild-type *S. pyogenes* Cas9 ribonucleoprotein complexes (RNPs) were prepared as follows. Oligonucleotides AR363 (synthetic tracrRNA bearing 5' DNA extension, here not used) and AR400 (synthetic crRNA) were first annealed by incubating 1 µL of AR363 (at 100 µM), 1 µL AR400 (at 100 µM) and 8 µL nuclease-free duplex buffer (Integrated DNA Technologies, Inc., Cat # 11-01-03-01) at 95°C for 5 min, followed by cooling to room temperature to form 10 µM **tracrRNA-crRNA complex.** RNPs were then formed by incubating 9 µL of tracrRNA-crRNA complex (600 nM final concentration) with 200 nM *S. pyogenes* Cas9 (New England Biolabs, Inc., Cat # M0386M) in a total of 150 µL NEB CutSmart buffer at room temperature for 20 minutes. This step yielded 150 µL of "Cas9

### RNPs".

Three distinct reactions were performed in three single tubes as follows:
**(1)** A target cleavage reaction in which dA-tailing was performed using Taq polymerase, wherein Cas9 RNPs and Taq polymerase were added simultaneously to the reaction mix, but the dA-tailing reaction is initiated by raising the temperature from 37°C (a temperature at which Cas9 target cleavage is close to optimally active) to 72°C (a temperature which heat-inactivates Cas9, but at which Taq polymerase is optimally active for dA-tailing).
   **500 ng of end-protected gDNA** was cleaved and dA-tailed by incubation of 5 µL (500 ng) of the dephosphorylated library **(end-protected gDNA,** above), 25 µL Cas9 RNPs (above), 200 µM dATP (1.6 µL of 10 mM stock), 5,000 units (1 µL) Taq polymerase (New England Biolabs, Inc., Cat # M0273), 4.5 µL NEB CutSmart Buffer, 40.5 µL nuclease-free water for a total of 77.6 µL. This mixture was incubated at 37°C for 30 min to cleave target sites using Cas9, then 72°C for 5 min to both denature Cas9 and dA-tail all accessible 3' ends, using a PCR thermocycler, to yield 500 ng **"target-cleaved DNA, dA-tailed by Taq polymerase".** This step was performed in the same tube as the dephosphorylation step above and carried forwards for the next ligation step.
**(2)** A target cleavage reaction in which dA-tailing was performed concomitantly with Cas9-mediated target cleavage using an exonuclease mutant of *E. coli* DNA Polymerase I, Klenow fragment.
   **500 ng of end-protected gDNA** was cleaved by incubation of 5 µL (500 ng) of the dephosphorylated library (**end-protected gDNA**, above), 25 µL Cas9 RNPs (above), 200 µM dATP (1.6 µL of 10 mM stock), 4.5 µL NEB CutSmart Buffer, 4.5 µL (22,500 units) of Klenow fragment (5'-3' exo⁻; NEB, Cat # M0212) and 40.5 µL nuclease-free water for a total of 79.5 µL. This mixture was incubated at 37°C for 30 min to cleave target sites using Cas9 and dA-tail all accessible 3' ends. Cas9 and Klenow fragment were subsequently heat-denatured at 75°C for 20 min. This step yielded 500 ng **"target-cleaved DNA, dA-tailed concomitantly by Klenow fragment".**
**(3)** A target cleavage reaction in which cleavage and dA-tailing were performed sequentially using Cas9 RNPs and an exonuclease mutant of *E. coli* DNA Polymerase I, Klenow fragment.
   **500 ng of end-protected gDNA** was cleaved by incubation of 5 µL (500 ng) of the dephosphorylated library (**end-protected gDNA**, above), 25 µL Cas9 RNPs (above), 200 µM dATP (1.6 µL of 10 mM stock), 40.5 µL nuclease-free water and 4.5 µL NEB CutSmart Buffer for 30 min at 37°C. Cas9 was then heat-inactivated by incubation for 20 min at 75°C and cooling to room temperature. To the same tube, 4.5 µL (22,500 units) of Klenow fragment (5'-3' exo⁻; NEB, Cat # M0212) were added, for a total of 79.5 µL. This mixture was incubated at 37°C for 30 min to dA-tail accessible DNA ends. Klenow fragment was subsequently heat-denatured at 75°C for 20 min. This step yielded 500 ng **"target-cleaved DNA, dA-tailed sequentially by Klenow fragment".**

Following the target cleavage and dA-tailing steps, sequencing adapter was ligated to each sample. Adapter ligation was performed in the same tube by incubating target-cleaved, dA-tailed gDNA with 40 µL 4x ligation buffer (**ONLS13117**), 2.35 µL **AMX 1D** (from Oxford Nanopore LSK-108, concentrated to 1.7 µM using a Vivaspin-500 concentrator; Sartorius), 10 µL T4 DNA ligase (2 million units/mL, from NEB Quick Ligase kit; NEB, Cat # M2200) and 26.7 µL nuclease-free water for a total volume of ~160 µL. This mixture was incubated for 10 min at room-temperature to yield **adapter-ligated gDNA.** The mixture was then subjected to SPRI purification to remove unligated adapter and other contaminants. 0.4 volumes (~64 µL) SPRI beads (AMPure XP beads, Beckman Coulter, Inc.) were added to adapter-ligated DNA, mixed gently by inversion, and incubated for 10 min at room temperature to bind the adapter-ligated DNA to the beads. The beads were pelleted using a magnetic separator, the supernatant removed, and washed twice with 250 µL **ABB** (from Oxford Nanopore LSK-108), with complete resuspension of the beads at each wash and repelleting of the beads following the wash. Following the second wash, the beads were pelleted once more, the excess wash buffer removed, and the DNA eluted from the beads by resuspension of the bead pellet in 16 µL Tris elution buffer (10 mM Tris-Cl, 20 mM NaCl, pH 7.5 at room temperature) for 10 min at room temperature. The beads were pelleted once more and the eluate (supernatant), containing purified gDNA, adapted at the target sites, retained. 23.3 µL **RBF** and 11.7 µL **LLB** (both from Oxford Nanopore Technologies' LSK-108) were added to 15 µL of the eluate to yield

### "MinION sequencing mix".

To sequence target DNA, an Oxford Nanopore Technologies FLO-MIN106 flowcell was prepared by introducing 800 µL **flowcell preparation mix** (prepared using: 480 µL RBF from **Oxford Nanopore LSK-108,** 520 µL nuclease-free water, 0.5 µL of 100 µM of a cholesterol adapter-tether **SK43**) via the inlet port. The SpotON port was subsequently opened and a further 200 µL **flowcell preparation mix** perfused via the inlet port. 50 µL of MinION sequencing mix were added to the flowcell via the SpotON port, and the ports closed. 6 h of sequencing data were collected using Oxford Nanopore Technologies' MinKNOW (version 1.10.6), and subsequently basecalled (using **Albacore**) and aligned to the *E. coli* SCS110 reference genome offline.

### Results

Figure 15 and Table 1 below examine the bias between forwards and reverse orientation reads from the Taq polymerase condition (condition (1)). The *rrs* gene, targeted by the degenerate crRNA probe, is found in both orientations in the *E. coli* SCS110 reference. Six out of the seven *rrs* genes exhibited a clear bias in read direction, which correlated with the orientation of the gene in the reference genome. Very similar bias was observed with the other two conditions (conditions (2) and (3), Figure 15).

Figure 16 shows the pileups resulting from alignment of sequencing reads to the *E. coli* reference. The crRNA used in the experiment described above targets a protospacer sequence common to all seven copies of the *rrs* gene in strain *E. coli* SCS110. Enrichment of the target region as observed, as expected, at each of the seven *rrs* genes (the locations of which are shown in Table 1 below), showing that Cas9 cut predominantly in the correct location, an that the cut sites were released (to varying extents) and dA-tailed, and that the adapter was efficiently ligated to the cut sites.

Figure 16 also highlights the differences between the approaches used. The highest on-target throughput (8698) was obtained when the cleaved sample was dA-tailed at 72°C using Taq polymerase (condition (1)). Conversely, the lowest number of on-target reads (1095) was obtained when the cleaved sample was dA-tailed concomitantly with Cas9 cleavage at 37°C (condition (2)). An intermediate number of reads (5191) was obtained when the sample was dA-tailed following heat-inactivation of Cas9 (condition (3)). The percentage of on target reads was 84.1% when the cleaved sample was dA-tailed at 72°C using Taq polymerase (condition (1)), 75.9% when the cleaved sample was dA-tailed concomitantly with Cas9 cleavage at 37°C (condition (2)), and 86.3% when the sample was dA-tailed following heat-inactivation of Cas9 (condition (3)).

**Table 1: The locations of the rrs gene in E.Coli and the read bias between forward and reverse orientation reads obtained when the cleaved sample was dA-tailed at 72°C using Taq polymerase**

| **Peak** | **Gene** | **Genomic coordinates** | **Location of crRNA** | **Chromosomal orientation** | **Number of + reads** | **Number of** - **reads** | **Overall read bias (+ : -)** |
|---|---|---|---|---|---|---|---|
| *i* | *rrsH* | 223771-225312 | 223960 | + | 971 | 158 | 6.1:1 |
| *ii* | *rrsG* | 2729616-2731157 | 2730968 | - | 372 | 364 | 1:1 |
| *iii* | *rrsD* | 3427221-3428762 | 3428573 | - | 100 | 163 | 1:1.63 |
| *iv* | *rrsC* | 3941808-3943349 | 3941997 | + | 1053 | 184 | 5.7:1 |
| *v* | *rrsA* | 4035531-4037072 | 4035720 | + | 1035 | 166 | 6.2:1 |
| *vi* | *rrsB* | 4166659-4168200 | 4166848 | + | 1149 | 330 | 3.5:1 |
| *vii* | *rrsE* | 4208147-4209688 | 4208336 | + | 943 | 203 | 4.6:1 |

We have already established (as described in WO 2018/060740) that bound, nuclease-deficient S. *pyogenes* dCas9 dissociates from target DNA upon incubation of the enzyme above ~60°C for 5 min. Here, the heat-inactivation of wild-type Cas9 was either 5 min at 72°C (for the Taq condition, condition (1)), or 20 min at 75°C (for the Klenow exo-sequential condition, condition (2)). The similarity of the percentage of on-target reads for conditions (1) and (2) demonstrates that 5 min at 72°C is sufficient to render at least the PAM-proximal side of a Cas9-generated double-stranded break accessible to a dA-tailing enzyme.

Taken together, the data suggest: (i) that heat-inactivation of Cas9 following Cas9-mediated cleavage is required to increase the accessibility of the cut site to the dA-tailing polymerase; (ii) that, upon heat denaturation, the short (PAM-proximal) side of the cut is preferentially released by Cas9, whereas the PAM-distal side remains bound by denatured Cas9 and is significantly less accessible to dA-tailing enzymes; and (iii), that an incubation of 72°C for 5 min is sufficient to render Cas9-generated ends accessible to dA-tailing enzymes.

### Example 2

This Example demonstrates that a plurality of synthetic crRNA probes may be used to excise and sequence multiple regions of interest (ROIs) from a human genomic DNA (gDNA) sample. Here, ten human gene targets were excised, using a series of redundant probes, and sequenced using Cas9 to high coverage depth (>100x per allele) without amplification. The lack of amplification preserves certain interesting structural features such as disease-relevant nucleotide expansion repeats. Furthermore, we show here that dephosphorylation of the gDNA library is required to reduce the number of background DNA strands that are read, thus increasing the throughput of on-target DNA reads.

### Materials and Methods

High-molecular weight genomic DNA ("**gDNA**") was purified by extraction from cultured human cells (cell line GM12878; Coriell Institute) using a Qiagen tip-500, according to the manufacturer's instructions. A total of 25 µg gDNA was dephosphorylated in bulk via treatment with calf intestinal dephosphorylase. 12.5 µL Quick CIP (from 'NEB Quick CIP kit', New England Biolabs, Inc., Cat # M0508) were added to the 25 µg of gDNA in a total of 250 µL NEB CutSmart Buffer (New England Biolabs, Inc., Catalogue # B7204) for 10 min at 37°C, followed by heat inactivation of the dephosphorylase at 80°C for 2 min. This step yielded **"end-protected gDNA".**

Separately, a control library was prepared adding 5 µg of non-dephosphorylated GM12878 to a total of 50 µL NEB CutSmart buffer. This step yielded **"non-dephosphorylated gDNA".**

Wild-type *S. pyogenes* Cas9 ribonucleoprotein complexes (RNPs) were prepared as follows. An equimolar mix of 41 custom Alt-R Cas9 crRNAs (synthesized by Integrated DNA Technologies, Inc.) was prepared by mixing 1 µL of each crRNA (resuspended at 100 µM TE buffer, pH 7.5) in an Eppendorf DNA Lo-Bind tube. Oligonucleotides AR363 (synthetic tracrRNA bearing 5' DNA extension, here not used) and the 41-probe pool of synthetic crRNAs were annealed by incubating 1 µL of AR363 (at 100 µM), 1 µL crRNA mix (at 100 µM) and 8 µL nuclease-free duplex buffer (Integrated DNA Technologies, Inc., Cat # 11-01-03-01) at 95°C for 5 min, followed by cooling to room temperature, to form 10 µM **tracrRNA-crRNA complex.** RNPs were then formed by incubating 7.5 µL of tracrRNA-crRNA complex (600 nM final concentration) with 300 nM *S. pyogenes* Cas9 (New England Biolabs, Inc., Cat # M0386M) in a total of 125 µL NEB CutSmart buffer at room temperature for 20 minutes. This step yielded 125 µL of **"Cas9 RNPs".**
50 µL (5 µg) end-protected gDNA was cleaved by the addition of 25 µL Cas9 RNPs. The reaction was incubated for 37°C for 60 min, followed by heat inactivation at 75°C for 20 min, followed by slow-cooling to room temperature. The gDNA was dA-tailed by the addition, to the same tube, of 1.6 µL of 10 mM dATP, and 4.5 µL of Klenow exo- (NEB, Cat # M0212), and incubation at 37°C for 30 min, followed by heat-inactivation at 75°C for 20 min. This procedure replicates condition (3) as described in Example 1. This procedure yielded **Library A** (75 µL).

As control for the requirement of dephosphorylation, 50 µL (5 µg) non-dephosphorylated gDNA was cleaved and dA-tailed exactly as for the end-protected gDNA. This procedure yielded **Library B** (75 µL).

As a control for the requirement of Cas9-generated ends for reads in the target region, 25 µL NEB CutSmart buffer was added to 50 µL (5 µg) end-protected gDNA. The mixture was incubated for 37°C for 60 min, followed by heat inactivation at 75°C for 20 min, followed by slow-cooling to room temperature. The gDNA was dA-tailed by the addition, to the same tube, of 1.6 µL of 10 mM dATP, and 4.5 µL of Klenow exo- (NEB, Cat # M0212), and incubation at 37°C for 30 min, followed by heat-inactivation at 75°C for 20 min. This procedure replicates condition (3) as described in Example 1. This procedure yielded **Library C** (75 µL).

Adapter ligation to Libraries A, B and C was performed by incubating Library A, Library B or Library C, separately, with 40 µL 4x ligation buffer (**ONLS13117**), 2.35 µL **AMX 1D** (from Oxford Nanopore LSK-108, concentrated to 1.7 µM using a Vivaspin-500 concentrator; Sartorius), 10 µL T4 DNA ligase (2 million units/mL, from NEB Quick Ligase kit; NEB, Cat # M2200) and 26.7 µL nuclease-free water for a total volume of ~154 µL. This mixture was incubated for 10 min at room-temperature to yield **adapter-ligated gDNA.** The mixture was then subjected to SPRI purification to remove unligated adapter and other contaminants. 0.4 volumes (~62 µL) SPRI beads (AMPure XP beads, Beckman Coulter, Inc.) were added to adapter-ligated DNA, mixed gently by inversion, and incubated for 10 min at room temperature to bind the adapter-ligated DNA to the beads. The beads were pelleted using a magnetic separator, the supernatant removed, and washed twice with 250 µL **ABB** (from Oxford Nanopore LSK-108), with complete resuspension of the beads at each wash and repelleting of the beads following the wash. Following the second wash, the beads were pelleted once more, the excess wash buffer removed, and the DNA eluted from the beads by resuspension of the bead pellet in 16 µL Tris elution buffer (10 mM Tris-Cl, 20 mM NaCl, pH 7.5 at room temperature) for 10 min at room temperature. The beads were pelleted once more and the eluate (supernatant), containing purified gDNA, adapted at the target sites, retained. 23.3 µL **RBF** and 11.7 µL **LLB** (both from Oxford Nanopore Technologies' LSK-108) were added to 15 µL of the eluate to yield **"MinION sequencing mixes A, B and C"** pertaining to Libraries A, B and C respectively.

To sequence target DNA, three Oxford Nanopore Technologies FLO-MIN106 flowcells were prepared by introducing 800 µL **flowcell preparation mix** (prepared using: 480 µL RBF from **Oxford Nanopore LSK-108,** 520 µL nuclease-free water, 0.5 µL of 100 µM of a cholesterol adapter-tether **SK43**) via the inlet port. The SpotON port was subsequently opened and a further 200 µL **flowcell preparation mix** perfused via the inlet port. 50 µL of MinION sequencing mixes A, B or C were added to each flowcell via the SpotON port, and the ports closed. 48 h of sequencing data were collected using Oxford Nanopore Technologies' MinKNOW (version 1.10.6), basecalled online using MinKNOW during the sequencing run, and aligned to the NA12878 human reference genome offline using bwa.

### Results

Figure 17 shows the pileups resulting from alignment of sequencing reads to the human NA12878 reference for **Library A.** The crRNAs used in the experiment described above target protospacer sequences in ten human genes. Enrichment of the target regions was observed, as expected, showing that Cas9 cut predominantly in the correct location, the cut sites were released (to varying extents), dA-tailed, and adapter efficiently ligated to the cut sites. Approximately 10% of all reads mapped to one of the ten target regions. An itemized list of reads for each target is given in Table 2 below.

**Table 2: Locations, number or reads and % on target reads for each target polynucleotide in Library A**

| **Target** | **Genomic coordinates of cut sites** | **Reads** | **% on target** |
|---|---|---|---|
| *HTT* | Chr4:3072436, 3072537, 3077290, 3079447 | 1156 | 1.03 |
| *FMR1* | ChrX:147911805, 147911857, 147910984, 147911228, 147932674 | 250 | 0.22 |
| *SCA10* | Chr22:45791502, 45792656, 45798180, 45798335 | 677 | 0.60 |
| *SCA2* | Chr12:111596525, 111597802, 111600589, 111602312 | 3471 | 3.09 |
| *SCA3* | Chr14:92068270, 92068306, 92073109, 92074370 | 634 | 0.56 |
| *SCA17* | Chr6:170557049, 170557884, 170563749, 170565282 | 679 | 0.61 |
| *SCA6* | Chr19:13205503, 13205664, 13210029, 13210853 | 1433 | 1.28 |
| *C9orf72* | Chr9:27572705, 27573133, 27574814, 27576479 | 1573 | 1.40 |
| *MUC1* | Chr1:155181544, 155183902, 155196219, 155197032 | 514 | 0.46 |
| *INS* | Chr11:2159199, 2159800, 2165720, 2166471 | 926 | 0.83 |
| **all on target** | | **11313** | **10.1** |
| **all reads** | | **112222** | **100** |

Table 3 below shows that approximately one-third the number of reads for the same ten-gene target panel was obtained when the sample was not dephosphorylated before initiating the Cas9 cut, but was otherwise identical to Library A (**Library B**). Only 1 in 300 reads mapped to one of the target regions (~0.33%), compared with 1 in 10 for Library A. Thus, dephosphorylation of non-target DNA significantly reduced the number of non-target reads.

**Table 3: Locations, number or reads and % on target reads for each target polynucleotide in Library A**

| **Target** | **Genomic coordinates of cut sites** | **Reads** | **% on target** |
|---|---|---|---|
| *HTT* | Chr4:3072436, 3072537, 3077290, 3079447 | 386 | 0.031 |
| *FMR1* | ChrX:147911805, 147911857, 147910984, 147911228, 147932674 | 78 | 0.006 |
| *SCA10* | Chr22:45791502, 45792656, 45798180, 45798335 | 252 | 0.020 |
| *SCA2* | Chr12:111596525, 111597802, 111600589, 111602312 | 1380 | 0.111 |
| *SCA3* | Chr14:92068270, 92068306, 92073109, 92074370 | 193 | 0.016 |
| *SCA17* | Chr6:170557049, 170557884, 170563749, 170565282 | 244 | 0.020 |
| *SCA6* | Chr19:13205503, 13205664, 13210029, 13210853 | 438 | 0.035 |
| *C9orf72* | Chr9:27572705, 27573133, 27574814, 27576479 | 702 | 0.057 |
| *MUC1* | Chr1:155181544, 155183902, 155196219, 155197032 | 161 | 0.013 |
| *INS* | Chr11:2159199, 2159800, 2165720, 2166471 | 326 | 0.026 |
| **all on target** | | **4160** | **0.33** |
| **all reads** | | **1240852** | **100** |

Table 4 below shows that only a single read corresponding to the *FMR1* gene was obtained when the library was dephosphorylated, but not cut with Cas9 (**Library C**). Thus, cutting by Cas9 is absolutely required to yield on-target reads when the library is dephosphorylated.

**Table 4: Locations, number or reads and % on target reads for each target polynucleotide in Library A**

| **Target** | **Genomic coordinates of cut sites** | **Reads** | **% on target** |
|---|---|---|---|
| *HTT* | Chr4:3072436, 3072537, 3077290, 3079447 | 0 | 0 |
| *FMR1* | ChrX:147911805, 147911857, 147910984, 147911228, 147932674 | 1 | 0.0066 |
| *SCA10* | Chr22:45791502, 45792656, 45798180, 45798335 | 0 | 0 |
| *SCA2* | Chr12:111596525, 111597802, 111600589, 111602312 | 0 | 0 |
| *SCA3* | Chr14:92068270, 92068306, 92073109, 92074370 | 0 | 0 |
| *SCA17* | Chr6:170557049, 170557884, 170563749, 170565282 | 0 | 0 |
| *SCA6* | Chr19:13205503, 13205664, 13210029, 13210853 | 0 | 0 |
| *C9orf72* | Chr9:27572705, 27573133, 27574814, 27576479 | 0 | 0 |
| *MUC1* | Chr1:155181544, 155183902, 155196219, 155197032 | 0 | 0 |
| *INS* | Chr11:2159199, 2159800, 2165720, 2166471 | 0 | 0 |
| **all on target** | | **1** | **0.0066** |
| **all reads** | | **15088** | **100** |

### OLIGONUCLEOTIDES

**tracrRNA**

| | **Sequence (5' → 3')** |
|---|---|
| AR363 | TACATTTAAGACCCTAATAT/iSp18/mA*mG*mCmAmUmAmGmCmArArGrUrUrArArArArUrArArG rGrCrUrArGrUrCrCrGrUrUrArUrCrArAmCmUmUmGmAmAmAmAmAmGmUmGmGmCmAmCmCmGmAmG mUmCmGmGmUmGmCmU*mU*mU |

**crRNA**
The crRNAs used throughout were custom purchased from IDT ("Alt-R^{®} CRISPR-Cas9 crRNA")

| **Cas9 crRNA** | **Sequence (5' → 3')** |
|---|---|
| AR400 | AGACCAAAGAGGGGGACCTT |
| HTT_Cas9_2561_+ | TTTGCCCATTGGTTAGAAGC |
| HTT_Cas9_2662_+ | TCTTATGAGTCTGCCCACTG |
| HTT_Cas9_7412_- | GGACAAAGTTAGGTACTCAG |
| HTT_Cas9_9569_- | CTAGACTCTTAACTCGCTTG |
| SCA10_Cas9_1149_+ | AATAGGGGCTAAGCATGGTC |
| SCA10_Cas9_2303_+ | TCCCTGAGAAAGTCTTGGTA |
| SCA10_Cas9_7824_- | CGGATTTGGGAACAGAGTAA |
| SCA10_Cas9_7979_- | CGGCTGAGATAAACCATCAT |
| SCA2_Cas9_2576_+ | GATACGCACAAACCTAAGTG |
| SCA2_Cas9_3853_+ | CATTTCCGAAATTGGGGCGG |
| SCA2_Cas9_6637_- | GTTGGACTACTGAAAACTGC |
| SCA2_Cas9_8360_- | CAAACTGCCCACCATCGTGA |
| SCA3_Cas9_2261_+ | CCAGGTTGGGGTACATATCT |
| SCA3_Cas9_2297_+ | TTTGCTGACAGGGGTGAATG |
| SCA3_Cas9_7097_- | TCACATACCTTCTTGAGTGG |
| SCA3_Cas9_8358_- | CAGAGAACAACCAAAGTGGA |
| SCA17_Cas9_143_+ | GCCACCTTACGCTCAGGGCT |
| SCA17_Cas9_978_+ | ATAGTCACTCTGCTGGCCCC |
| SCA17_Cas9_6840_- | TGCTCAACAACTGTCTCGCA |
| SCA17_Cas9_8373_- | TATAGACTGCTGTACTCCCA |
| SCA6_Cas9_2646_+ | ACCCAAGGTAAGCTCAAGCA |
| SCA6_Cas9_2807_+ | ATGGCTGAAACACTTCGTGG |
| SCA6_Cas9_7169_- | AGAAGGACTCAGACTTGTGG |
| SCA6_Cas9_7993_- | ATAGAGGACGCCCAGCCCCG |
| C9orf72_Cas9_2221_+ | AGATAGACCCAATGAGCACA |
| C9orf72_Cas9_2649_+ | CCCCGGGAAGGAGACAGCTC |
| C9orf72_Cas9_4327_- | AAACTGGTCTCAGGTCACAA |
| C9orf72_Cas9_5992_- | TCCATAAGCTGTGAAGCCGG |
| MUC1_Cas9_1546_+ | ATGGGGCTGGCCACAAGTAA |
| MUC1_Cas9_3904_+ | TCGGGGGCAAGCTCAAACGC |
| MUC1_Cas9_16218_- | AGGCCTGGTGAGCTCAAGGG |
| MUC1_Cas9_17031_- | TGGCTACATTCGGTAAGGAG |
| INS_Cas9_1201_+ | ACCTGGGCTGGCATAAGCTG |
| INS_Cas9_1802_+ | ATCTCTCTCGGTGCAGGAGG |
| INS_Cas9_7719_- | CGGGCTGTGTAAGCAGAACG |
| INS_Cas9_8470_- | CAGTTCTCGCAGGTACGCCG |
| AR849_FMR1 | CCACTTGAAGAGAGAGGGCG |
| AR852_FMR1 | ACAGCGTTGATCACGTGACG |
| AR853_FMR1 | GATTAAGGCAGCTATAAGCA |
| AR855_FMR1 | GTTGAGGAAAGGCGAGTACG |
| AR777_FMR1 | CATCCTGATCCTAATAAAAG |

wt Cas9 Nuclease, S. pyogenes ONLS13117
4x ligation buffer composition: 202mM Tris-HCl (pH8 - 4°C), 2.5M NaCl, 30 % PEG-8000 (w/v), 40 mM ATP

### Example 3

This Example demonstrates how a synthetic crRNA probes can be used to excise and sequence regions of interest (ROIs) for a duplicated region of a bacterial genome for nanopore sequencing. Here is described a simple, one-pot approach, in which the enzymatic steps (dephosphorylation, Cpf1-mediated cleavage, barcoding or dA-tailing and adapter ligation) are performed sequentially.

### Materials and Methods

High-molecular weight genomic DNA ("**gDNA**") was purified by extraction from *Escherichia coli* (strain SCS110) using a Qiagen tip-500, according to the manufacturer's instructions. 2 µg gDNA was dephosphorylated via treatment with calf intestinal dephosphorylase. 6 µL Quick CIP (from 'NEB Quick CIP kit', New England Biolabs, Inc., Cat # M0508) were added to the 2 µg of gDNA in a total of 120 µL NEB CutSmart Buffer (New England Biolabs, Inc., Catalogue # B7204) for 10 min at 37°C, followed by heat inactivation of the dephosphorylase at 80°C for 2 min. This step yielded **"end-protected gDNA".**

Oligonucleotides **AR630** to **AR643** (known as **"guide RNAs"**) were pooled together and diluted to 10 µM with nuclease-free water. Prior to complex formation, 500 nM **"guide RNAs"** in CutSmart buffer (New England Biolabs B72004) were incubated at 95°C for 4 minutes and then cooled to 21°C. CRISPR-Cpf1 complexes were formed by adding 500 nM *L.bacterium* Cpf1 (New England Biolabs M0653) to the reaction, for 20 minutes at 21°C, yielding 500 nM of **CRISPR-Cpf1 complex. End-protected gDNA** was cleaved with the addition of a final concentration of 125 nM of **CRISPR-Cpf1 complex** and incubated for 15 minutes at 37°C, resulting in a complex known as **"probe-target complex".**

Four distinct reactions were performed in four single tubes as follows:
**A.** The **probe-target complex** was ligated to the sequencing adapter via a library of specific barcodes matching the 5'nt overhang sequence of each cutting site.
   Oligonucleotides **AR598, AR656** and **AR657** were each annealed to **NB01,** each at 40 µM, in 10 mM Tris-Cl (pH 8.0), 1 mM EDTA, 100 mM NaCl, from 95°C to 25°C at 1°C per minute. The hybridised DNAs were pool together and were known as **"specific barcodes".** Approximately 33 nM of BAM 1D (**ONT SQK-LSK308**), bearing the helicase, was ligated to the **probe-target complex** with 0.2 µL of **specific barcodes** diluted to 1 µM using 50 µL of Blunt T/A Ligase Master Mix (New England Biolabs M0367) for 20 minutes at 21°C. This step yielded 500 ng **"target-cleaved DNA with specific barcodes".**
**B.** The **probe-target complex** was ligated to the sequencing adapter via a library of generic barcode using partially matching 5'nt overhang sequence of each cutting site.
   Oligonucleotides **CPBC34** and **CPBC37** were each annealed to **NB01,** each at 40 µM, in 10 mM Tris-Cl (pH 8.0), 1 mM EDTA, 100 mM NaCl, from 95°C to 25°C at 1°C per minute. The hybridised DNAs were pool together and were known as **"generic barcodes".** Approximately 33 nM of BAM 1D (**ONT SQK-LSK308**), bearing the helicase, was ligated to the **probe-target complex** with 0.2 µL of **generic barcodes** diluted to 1 µM using 50 µL of Blunt T/A Ligase Master Mix (New England Biolabs M0367) for a total of 120 µL for 20 minutes at 21°C. This step yielded 500 ng **"target-cleaved DNA with generic barcodes".**
**C.** The **probe-target complex** was dA-tailed using an exonuclease mutant of *E. coli* DNA Polymerase I, Klenow fragment.
   5,000 units (1 µL) of Klenow Fragment (3'-5' exo-) (New England Biolabs M0212) was added to the **probe-target complex** with 20 µM of dNTP (New England Biolabs N0446S) and 100 µM of dATP (New England Biolabs N0446S) and incubated for 15 minutes at 37°C and 5 minutes at 65°C. Approximately 25 nM of AMX 1D (from Oxford Nanopore LSK-108, concentrated to 1.7 µM using a Vivaspin-500 concentrator; Sartorius), bearing the helicase, was ligated to **probe-target complex** using 50 µL of Blunt T/A Ligase Master Mix (New England Biolabs M0367) for 10 minutes at 21°C.This step yielded 500 ng **"target-cleaved DNA dA-tailed by Klenow fragment".**
**D.** The **probe-target complex** was dA-tailed using Taq polymerase.
   5,000 units (1 µL) Taq polymerase (New England Biolabs M0273) was added to the **probe-target complex** with 20 µM of dNTP (New England Biolabs N0446S) and 100 µM of dATP (New England Biolabs N0446S) and incubated for 5 minutes at 65°C. Approximately 25 nM of AMX 1D (from Oxford Nanopore LSK-108, concentrated to 1.7 µM using a Vivaspin-500 concentrator; Sartorius), bearing the helicase, was ligated to **probe-target complex** using 50 µL of Blunt T/A Ligase Master Mix (New England Biolabs M0367) for 10 minutes at 21°C.This step yielded 500 ng **"target-cleaved DNA dA-tailed by Taq polymerase".**

Each mixture was subjected to purification step using SPRI magnetic beads, as follows: 0.4 volume equivalents of AMPure XP SPRI magnetic beads (Beckman Coulter) were added to the mixture and incubated for 10 min at 21°C. The magnetic beads were pelleted using a magnetic separator, the supernatant aspirated, and 250 µL of ABB (**ONT SQK-LSK108**) diluted with DLB added to resuspend the beads. The beads were immediately pelleted once more and the supernatant aspirated, after which the tube was removed from the rack and 16 µL Tris elution buffer (10 mM Tris-Cl, 20 mM NaCl, pH 7.5 at room temperature) for 10 min at room temperature. The beads were pelleted using the magnetic separator, and the eluate retained. This yielded a double-stranded DNAs bearing an adapter on each end, known as **"MinION sequencing mix A, B, C and D".**

To sequence target DNA, an Oxford Nanopore Technologies FLO-MIN106 flowcell was prepared by introducing 800 µL **flowcell preparation mix** (prepared using: 480 µL RBF from **Oxford Nanopore LSK-108,** 520 µL nuclease-free water, 0.5 µL of 100 µM of a cholesterol adapter-tether **SK43**) via the inlet port. The SpotON port was subsequently opened and a further 200 µL **flowcell preparation mix** perfused via the inlet port. 50 µL of MinION sequencing mix A, B, C or D were added to the flowcell via the SpotON port, and the ports closed. 6 h of sequencing data were collected using Oxford Nanopore Technologies' MinKNOW (version 1.10.6), and subsequently basecalled (using **Albacore**) and aligned to the *E. coli* SCS110 reference genome offline.

### Results

Figure 18 shows the pileups resulting from alignment of sequencing reads to the *E. coli* reference. Enrichment of the target regions was observed, as expected, at each of the seven *rrs* genes (the locations of which are shown in Table 5) showing that Cpf1 cut predominantly in the correct locations. The locations of the crRNA used to excise each copy of the *rrs* gene in strain *E. coli* SCS110 are listed in Table 5, which shows the seven expected binding locations of the single probe used in the pulldown.

Figure 19 compares the pileups resulting from the four different approaches (A to D) following Cpf1 cutting described above. Table 6 shows the number of reads and the percentage of on target reads for each of the approaches (A to D). The highest on-target throughput (90%) was obtained when the cleaved sample was barcoded using specific barcodes (condition A). The highest number of reads on target (118208) was achieved using dA-tailing with Taq polymerase.

**Table 5: The locations of the seven rrs genes in E. coli and the locations of the crRNA used to excise each copy of the rrs gene**

| | | | **Location of crRNA** | |
|---|---|---|---|---|
| **Peak** | **Gene** | **Genomic coordinates** | **sense strand** | **antisense strand** |
| *iv* | *rrsA* | 4035531-4037072 | 4034811 | 4040921 |
| *v* | *rrsB* | 4166659-4168200 | 4166190 | 4172975 |
| *iii* | *rrsC* | 3941808-3943349 | 3936397 | 3947016 |
| *vii* | *rrsD* | 3427221-3428762 | 3421595 | 3433252 |
| *vi* | *rrsE* | 4208147-4209688 | 4201886 | 4219583 |
| *ii* | *rrsG* | 2729616-2731157 | 2725057 | 2740503 |
| *i* | *rrsH* | 223771-225312 | 223018 | 233850 |

**Table 6: the number of reads and the percentage of on target reads for each of the approaches from the four different approaches following Cpf1 cutting**

| **Approach** | **Description** | **No. of reads** | **% on target** |
|---|---|---|---|
| A | Specific barcodes | 9969 | 90% |
| B | Generic barcodes | 15396 | 85% |
| C | dA tailing (Klenow (exo-)) | 68738 | 60% |
| D | dA tailing (Taq) | 118208 | 54% |

### Example 4

This Example demonstrates that a plurality of synthetic crRNA probes may be used to excise and sequence multiple regions of interest (ROIs) from a human genomic DNA sample. Here, ten human gene targets were excised, using a series of redundant probes, and sequenced using Cpf1 to high coverage depth (>100x per allele) without amplification. The lack of amplification preserves certain interesting structural features such as disease-relevant nucleotide expansion repeats.

### Materials and Methods

High-molecular weight genomic DNA ("**gDNA**") was purified by extraction from cultured human cells (cell line GM12878; Coriell Institute) using a Qiagen tip-500, according to the manufacturer's instructions. A total of 10 µg gDNA was dephosphorylated in bulk via treatment with calf intestinal dephosphorylase. 3 µL Quick CIP (from 'NEB Quick CIP kit', New England Biolabs M0508) were added to the 10 µg of gDNA in a total of 60 µL NEB CutSmart Buffer (New England Biolabs B7204) for 10 min at 37°C, followed by heat inactivation of the dephosphorylase at 80°C for 2 min. This step yielded **"end-protected gDNA".**

An equimolar mix of 39 custom Alt-R Cpf1 crRNAs (synthesized by Integrated DNA Technologies, Inc.) was prepared by mixing 1 µL of each crRNA (resuspended at 100 µM TE buffer, pH 7.5) in an Eppendorf DNA Lo-Bind tube. The mixture was then diluted to 10 µM with nuclease-free water and was known as **"guide RNAs".** Prior to complex formation, 500 nM **"guide RNAs"** in CutSmart buffer (New England Biolabs B72004) were incubated at 95°C for 4 minutes and then cooled to 21°C. CRISPR-Cpf1 complexes were formed by adding 500 nM *L.bacterium* Cpf1 (New England Biolabs M0653) to the reaction, for 20 minutes at 21°C, yielding 500 nM of **CRISPR-Cpf1 complex.** 125 nM of **CRISPR-Cpf1 complex** were added to the **end-protected gDNA** and incubated for 15 minutes at 37°C, resulting in a complex known as **"probe-target complex".**

Two distinct reactions were performed in two single tubes as follows:
**A.** The **probe-target complex** was ligated to the sequencing adapter via a specific barcode using specific 5'nt overhang cutting sequences.
   Oligonucleotides **AR598, AR656** and **AR657** were each annealed to **NB01,** each at 40 µM, in 10 mM Tris-Cl (pH 8.0), 1 mM EDTA, 100 mM NaCl, from 95°C to 25°C at 1°C per minute. The hybridised DNAs were pool together and were known as **"specific barcodes".** Approximately 33 nM of BAM 1D (**ONT SQK-LSK308**), bearing the helicase, was ligated to the **probe-target complex** with 0.2 µL of **specific barcodes** diluted to 1 µM using 50 µL of Blunt T/A Ligase Master Mix (New England Biolabs M0367) for 20 minutes at 21°C. This step yielded 500 ng **"target-cleaved DNA with specific barcodes".**
**B.** The **probe-target complex** was dA-tailed using an exonuclease mutant of *E. coli* DNA Polymerase I, Klenow fragment.
   5,000 units (1 µL) of Klenow Fragment (3'-5' exo-) (New England Biolabs M0212) was added to the **probe-target complex** with 20 µM of dNTP (New England Biolabs N0446S)and 100 µM of dATP (New England Biolabs N0446S) and incubated for 15 minutes at 37°C and 5 minutes at 65°C. Approximately 25 nM of AMX 1D (from Oxford Nanopore LSK-108, concentrated to 1.7 µM using a Vivaspin-500 concentrator; Sartorius), bearing the helicase, was ligated to **probe-target complex** using 50 µL of Blunt T/A Ligase Master Mix (New England Biolabs M0367) for 10 minutes at 21°C.This step yielded 500 ng **"target-cleaved DNA dA-tailed by Klenow fragment".**

The mixture was then subjected to SPRI purification to remove unligated adapter and other contaminants. 0.4 volumes SPRI beads (AMPure XP beads, Beckman Coulter, Inc.) were added to adapter-ligated DNA, mixed gently by inversion, and incubated for 10 min at room temperature to bind the adapter-ligated DNA to the beads. The beads were pelleted using a magnetic separator, the supernatant removed, and washed twice with 250 µL **ABB** (from Oxford Nanopore LSK-108), with complete resuspension of the beads at each wash and repelleting of the beads following the wash. Following the second wash, the beads were pelleted once more, the excess wash buffer removed, and the DNA eluted from the beads by resuspension of the bead pellet in 16 µL Tris elution buffer (10 mM Tris-Cl, 20 mM NaCl, pH 7.5 at room temperature) for 10 min at room temperature. The beads were pelleted once more and the eluate (supernatant), containing purified gDNA, adapted at the target sites, retained. 23.3 µL **RBF** and 11.7 µL **LLB** (both from Oxford Nanopore Technologies' LSK-108) were added to 15 µL of the eluate to yield **"MinION sequencing mixes A and B".**

To sequence target DNA, four Oxford Nanopore Technologies FLO-MIN106 flowcells were prepared by introducing 800 µL **flowcell preparation mix** (prepared using: 480 µL RBF from **Oxford Nanopore LSK-108,** 520 µL nuclease-free water, 0.5 µL of 100 µM of a cholesterol adapter-tether **SK43**) via the inlet port. The SpotON port was subsequently opened and a further 200 µL **flowcell preparation mix** perfused via the inlet port. 50 µL of MinION sequencing mixes A or B were added to each flowcell via the SpotON port, and the ports closed. 48 h of sequencing data were collected using Oxford Nanopore Technologies' MinKNOW (version 1.10.6), basecalled online using MinKNOW during the sequencing run, and aligned to the NA12878 human reference genome offline using bwa.

### Results

Figure 20 shows the pileups resulting from alignment of sequencing reads to the human NA12878 reference following the specific barcode approach. The crRNAs used in the experiment described above target protospacer sequences in ten human genes. Enrichment of the target regions was observed, as expected, showing that Cpf1 cut predominantly in the correct location, the cut sites were released (to varying extents), barcoded, and adapter efficiently ligated to the cut sites. Approximately 5% of all reads mapped to one of the ten target regions. An itemized list of reads for each target is given in Table 7.

Figure 21 shows the pileups resulting from alignment of sequencing reads to the human NA12878 reference following the dA-tailing with Klenow (exo-) approach. The crRNAs used in the experiment described above target protospacer sequences in ten human genes. Enrichment of the target regions was observed, as expected, showing that Cpf1 cut predominantly in the correct location, the cut sites were released (to varying extents), dA-tailed, and adapter efficiently ligated to the cut sites. Approximately 0.2% of all reads mapped to one of the ten target regions. An itemized list of reads for each target is given in Table 8.

**Table 7: Locations, number or reads and % on target reads for each target polynucleotide obtained using a specific barcode in approach A**

| | **Target** | **Genomic coordinates of cut sites** | **reads** | **% on target** |
|---|---|---|---|---|
| (i) | *HTT* | Chr4:3072436,3076713 | 363 | 1.1 |
| (ii) | *FMR1* | ChrX:147910462,147913441 | 109 | 0.3 |
| (iii) | *SCA10* | Chr22:45793272,45798243 | 167 | 0.5 |
| (iv) | *SCA17* | Chr12:170561302,170565756 | 374 | 1.1 |
| (v) | *SCA2* | Chr14:111597110,111600537 | 231 | 0.7 |
| (vi) | *SCA3* | Chr6:92069092,92073524 | 193 | 0.6 |
| (vii) | *SCA6* | Chr19:13206830,13210486 | 52 | 0.2 |
| (viii) | *C9orf72* | Chr9:27571959,27573673 | 118 | 0.3 |
| (ix) | *MUC1* | Chr1:155182116,155193330 | 124 | 0.4 |
| (x) | *INS* | Chr11:2161349,2163822 | 28 | 0.1 |
| | **all on target** | | **1759** | **5.2** |
| | **all reads** | | **33881** | **100.0** |

**Table 8: Locations, number or reads and % on target reads for each target polynucleotide obtained by dA tailing in approach B**

| | **Target** | **Genomic coordinates of cut sites** | **reads** | **% on target** |
|---|---|---|---|---|
| (i) | *HTT* | Chr4:3072436,3076713 | 363 | 1.1 |
| (ii) | *FMR1* | ChrX: 147910462,147913441 | 109 | 0.3 |
| (iii) | *SCA10* | Chr22:45793272,45798243 | 167 | 0.5 |
| (iv) | *SCA17* | Chr12:170561302,170565756 | 374 | 1.1 |
| (v) | *SCA2* | Chr14:111597110,111600537 | 231 | 0.7 |
| (vi) | *SCA3* | Chr6:92069092,92073524 | 193 | 0.6 |
| (vii) | *SCA6* | Chr19:13206830,13210486 | 52 | 0.2 |
| (viii) | *C9orf72* | Chr9:27571959,27573673 | 118 | 0.3 |
| (ix) | *MUC1* | Chr1:155182116,155193330 | 124 | 0.4 |
| (x) | *INS* | Chr11:2161349,2163822 | 28 | 0.1 |
| | **all on target** | | **1759** | **5.2** |
| | **all reads** | | **33881** | **100.0** |

### OLIGONUCLEOTIDES

### crRNA

The crRNAs used throughout were custom purchased from IDT ("Alt-R^{®} CRISPR-Cpf1 crRNA")

| **Probes** | **21 mer protospacer sequence (5'** → **3')** |
|---|---|
| AR630 | CCGAAGCACAGTTTGAAACGC |
| AR631 | TGCAGCTGGTCAAGGGGAAGC |
| AR632 | AAGCGCGCGTTTCTTGTTGCG |
| AR633 | TTGGCATTAACCAGGCAGGGC |
| AR634 | CCCACACGACCAACGCTGGCG |
| AR635 | TTGAAGGAGAACTGCACGCGC |
| AR636 | TATCGCTGAAAGATGGCGCGC |
| AR637 | TGGCAGGGGCGGAGAGACTCG |
| AR638 | TCAAAAAACATGCGACGCGGC |
| AR639 | TGGTGGAGTGGATGCAAAAGC |
| AR640 | TATGGCAATGACGCCAGGAGC |
| AR641 | TGTCTTACATGATGCGCCAGC |
| AR642 | TGCTGTCAGAAAGGGATGAGC |
| AR643 | AATACCCGATCAAAGCCCGGC |
| FMR1_Cpf1_147913435_AGGT- | CAGCCTTCCTTCCACACGCACC |
| FMR1_Cpf1_147916118 _CCTG- | TAACTTTATCTTTCCTTAACAG |
| FMR1_Cpf1_147908316_CTGC+ | ATGGAAACCAAGGGCCAAGGCA |
| FMR1_Cpf1_147910464_ | AGCCCTATTGGGTTCTTGGCCT |
| HTT_Cpf1_2326_CCTG_+ | CAATCTCACGTGGTGTTGGCA |
| HTT_Cpf1_2561_GTGG_+ | CCCATTGGTTAGAAGCAGGCC |
| HTT_Cpf1_6830_CTGC_- | GAATGATCAAGTGTCTGAAGC |
| HTT_Cpf1_9892_GAAG_- | TGCTTTTGCCGGTGTTCCCCT |
| SCA10_Cpf1_2674_CTGA_+ | CAGGCTCTGCAGTTGCTTCTC |
| SCA10_Cpf1_2919_ACTG_+ | TCCTCAGCATGTCTTCCATCA |
| SCA10_Cpf1_7882_CAGA_- | TGACCATGAGAGACACTGCTC |
| SCA10_Cpf1_7888_AGTG_- | TGTTTCTGACCATGAGAGACA |
| SCA2_Cpf1_918_TCTG_+ | CTCAGTACTATCAGCACGACA |
| SCA2_Cpf1_3161_AAGC_+ | GCTAAGTAGTGTTTGGGATGC |
| SCA2_Cpf1_6580_TCCT_- | CCTTTATCTGGACAGTTCTAG |
| SCA2_Cpf1_9275_TCTC_- | GCAACTCTATTAACTGAACGA |
| SCA3_Cpf1_2297_CTGG_+ | CTGACAGGGGTGAATGGGGCC |
| SCA3_Cpf1_3083_GTGA_+ | AGAAGGAGTTTTGGTCTTGTC |
| SCA3_Cpf1_7507_CAAC_- | GTAGAGACAGTTTTGCCATGT |
| SCA3_Cpf1_8754_TGGT_- | ATTGCCTAATACTTGAGCCAC |
| SCA17_Cpf1_1412_GTTG_+ | AGTTGCTCCACATCCTCACCA |
| SCA17_Cpf_4396_GGTT_+ | TTGAGATGGTCTGGAACCTAA |
| SCA17_ Cpf1_8842_CAGG_- | AAACCTGCTCTATGTCTTCCC |
| SCA6_Cpf1_1662_AAGC_+ | AGTTCAGGGCTCATGGGGGGC |
| SCA6_Cpf1_3973_AGAC_+ | CCGCACTCGGCCACCAGCTGT |
| SCA6_Cpf1_7621_TGGA_- | GCAATCGCACCCTCTCCCCTC |
| SCA6_Cpf1_7810_GGAT_- | TGTTTTTTCTGTGTGCACCAT |
| C9orf72_Cpf1_1388_GTGT_+ | CAGTACCAGAAAGTTCACAAC |
| C9orf72_Cpf1_1475_GTCT_+ | TCACAGTTCCAAGTTTCTCAG |
| C9orf72_Cpf1_3181_CAAG_- | CCACCCTCTCTCCCCACTACT |
| C9orf72_Cpf1_4092_TCAC_- | TTCCTCCCTTTCTTCCTCGGT |
| MUC1_Cpf1_1659_GAGG_+ | GAATGCCCCCTTCTTTTTTCC |
| MUC1_Cpf1_2118_CTGA_+ | CAGGGTGCCCCCGATGTGATC |
| MUC1_Cpf1_13324_CCAC_- | TCGGCCCCGCTCTGCTTCAGT |
| MUC1_Cpf1_13532_AAGC_- | TTCCCCCACTCCCTCCTTGGC |
| INS_Cpf1_2511_CCTC_+ | TTTGAGGGGCGAGTGGAGGGA |
| INS_Cpf1_3351_CTTC_+ | CCTGGTGCTGGGTCTGTGGGA |
| INS_Cpf1_10636_CTCT_- | AAGCCAAAATCCACCATCTAG |
| INS_Cpf1_5816_CAGA_- | GCCCTGGCCTCCTTCCTCCTC |

### Barcodes

The barcodes used throughout were purchased from IDT ("Custom DNA oligos")

| **Barcodes** | **Sequence (5' → 3')** |
|---|---|
| NB01 | /5Phos/AAGGTTAACACAAAGACACCGACAACTTTCTTCAGCACC |
| AR598 | /5Phos/CAGCGGTGCTGAAGAAAGTTGTCGGTGTCTTTGTGTTAACCTTAGCAAT |
| AR656 | /5Phos/CTGCGGTGCTGAAGAAAGTTGTCGGTCTTTGTGTTAACCTTAGCAAT |
| AR657 | /5Phos/TTCGGGTGCTGAAGAAAGTTGTCGGTCTTTGTGTTAACCTTAGCAAT |
| AR470 | /5Phos/CCACGGTGCTGAAGAAAGTTGTCGGTGTCTTTGTGTTAACCTTAGCAAT |
| AR471 | /5Phos/GTGGGGTGCTGAAGAAAGTTGTCGGTGTCTTTGTGTTAACCTTAGCAAT |
| AR472 | /5Phos/TCTGGGTGCTGAAGAAAGTTGTCGGTGTCTTTGTGTTAACCTTAGCAAT |
| AR473 | /5Phos/CAGAGGTGCTGAAGAAAGTTGTCGGTGTCTTTGTGTTAACCTTAGCAAT |
| AR473 | /5Phos/CAGAGGTGCTGAAGAAAGTTGTCGGTGTCTTTGTGTTAACCTTAGCAAT |
| AR595 | /5Phos/CTGAGGTGCTGAAGAAAGTTGTCGGTGTCTTTGTGTTAACCTTAGCAAT |
| AR595 | /5Phos/CTGAGGTGCTGAAGAAAGTTGTCGGTGTCTTTGTGTT AACCTT AGCAA T |
| AR599 | /5Phos/CCTGGGTGCTGAAGAAAGTTGTCGGTGTCTTTGTGTT AACCTT AGCAA T |
| AR599 | /5Phos/CCTGGGTGCTGAAGAAAGTTGTCGGTGTCTTTGTGTT AACCTT AGCAA T |
| AR601 | /5Phos/AGGTGGTGCTGAAGAAAGTTGTCGGTGTCTTTGTGTTAACCTTAGCAAT |
| AR656 | /5Phos/CTGCGGTGCTGAAGAAAGTTGTCGGTGTCTTTGTGTTAACCTTAGCAAT |
| AR656 | /5Phos/CTGCGGTGCTGAAGAAAGTTGTCGGTGTCTTTGTGTTAACCTTAGCAAT |
| AR660 | /5Phos/GAGGGGTGCTGAAGAAAGTTGTCGGTGTCTTTGTGTTAACCTTAGCAAT |
| AR660 | /5Phos/GAGGGGTGCTGAAGAAGAAAGTTGTCGGTGTCTTTGTGTTAACCTTAGCAAT |
| AR662 | /5Phos/CAGGGGTGCTGAAGAAAGTTGTCGGTGTCTTTGTGTTAACCTTAGCAAT |
| CPBC1 | /5Phos/CTTCGGTGCTGAAGAAAGTTGTCGGTGTCTTTGTGTTAACCTTAGCAAT |
| CPBC3 | /5Phos/AGTGGGTGCTGAAGAAAGTTGTCGGTGTCTTTGTGTTAACCTTAGCAAT |
| CPBC7 | /5Phos/AAGCGGTGCTGAAGAAAGTTGTCGGTGTCTTTGTGTTAACCTTAGCAAT |
| CPBC7 | /5Phos/AAGCGGTGCTGAAGAAAGTTGTCGGTGTCTTTGTGTTAACCTTAGCAAT |
| CPBC7 | /5Phos/AAGCGGTGCTGAAGAAAGTTGTCGGTGTCTTTGTGTTAACCTTAGCAAT |
| CPBC8 | /5Phos/ACTGGGTGCTGAAGAAAGTTGTCGGTGTCTTTGTGTTAACCTTAGCAAT |
| CPBC9 | /5Phos/AGACGGTGCTGAAGAAAGTTGTCGGTGTCTTTGTGTTAACCTTAGCAAT |
| CPBC10 | /5Phos/CAACGGTGCTGAAGAAAGTTGTCGGTGTCTTTGTGTTAACCTTAGCAAT |
| CPBC11 | /5Phos/CAAGGGTGCTGAAGAAAGTTGTCGGTGTCTTTGTGTTAACCTTAGCAAT |
| CPBC12 | /5Phos/CCTCGGTGCTGAAGAAAGTTGTCGGTGTCTTTGTGTTAACCTTAGCAAT |
| CPBC13 | /5Phos/CTCTGGTGCTGAAGAAAGTTGTCGGTGTCTTTGTGTTAACCTTAGCAAT |
| CPBC14 | /5Phos/CTGGGGTGCTGAAGAAAGTTGTCGGTGTCTTTGTGTTAACCTTAGCAAT |
| CPBC15 | /5Phos/GAAGGGTGCTGAAGAAAGTTGTCGGTGTCTTTGTGTTAACCTTAGCAAT |
| CPBC16 | /5Phos/GGATGGTGCTGAAGAAAGTTGTCGGTGTCTTTGTGTTAACCTTAGCAAT |
| CPBC17 | /5Phos/GGTTGGTGCTGAAGAAAGTTGTCGGTGTCTTTGTGTTAACCTTAGCAAT |
| CPBC18 | /5Phos/GTCTGGTGCTGAAGAAAGTTGTCGGTGTCTTTGTGTTAACCTTAGCAAT |
| CPBC19 | /5Phos/GTGAGGTGCTGAAGAAAGTTGTCGGTGTCTTTGTGTTAACCTTAGCAAT |
| CPBC20 | /5Phos/GTGTGGTGCTGAAGAAAGTTGTCGGTGTCTTTGTGTTAACCTTAGCAAT |
| CPBC21 | /5Phos/GTTGGGTGCTGAAGAAAGTTGTCGGTGTCTTTGTGTTAACCTTAGCAAT |
| CPBC22 | /5Phos/TCACGGTGCTGAAGAAAGTTGTCGGTGTCTTTGTGTTAACCTTAGCAAT |
| CPBC23 | /5Phos/TCCTGGTGCTGAAGAAAGTTGTCGGTGTCTTTGTGTTAACCTTAGCAAT |
| CPBC24 | /5Phos/TCTCGGTGCTGAAGAAAGTTGTCGGTGTCTTTGTGTTAACCTTAGCAAT |
| CPBC25 | /5Phos/TGGAGGTGCTGAAGAAAGTTGTCGGTGTCTTTGTGTTAACCTTAGCAAT |
| CPBC26 | /5Phos/TGGTGGTGCTGAAGAAAGTTGTCGGTGTCTTTGTGTTAACCTTAGCAAT |
| CPBC28 | /5Phos/NNCCGGTGCTGAAGAAAGTTGTCGGTGTCTTTGTGTTAACCTTAGCAAT |
| CPBC29 | /5Phos/NNGGGGTGCTGAAGAAAGTTGTCGGTGTCTTTGTGTTAACCTTAGCAAT |
| CPBC30 | /5Phos/NNAAGGTGCTGAAGAAAGTTGTCGGTGTCTTTGTGTTAACCTTAGCAAT |
| CPBC31 | /5Phos/NNTTGGTGCTGAAGAAAGTTGTCGGTGTCTTTGTGTTAACCTTAGCAAT |
| CPBC32 | /5Phos/NNCAGGTGCTGAAGAAAGTTGTCGGTGTCTTTGTGTTAACCTTAGCAAT |
| CPBC33 | /5Phos/NNCTGGTGCTGAAGAAAGTTGTCGGTGTCTTTGTGTTAACCTTAGCAAT |
| CPBC34 | /5Phos/NNCGGGTGCTGAAGAAAGTTGTCGGTGTCTTTGTGTTAACCTTAGCAAT |
| CPBC35 | /5Phos/NNGAGGTGCTGAAGAAAGTTGTCGGTGTCTTTGTGTTAACCTTAGCAAT |
| CPBC36 | /5Phos/NNGTGGTGCTGAAGAAAGTTGTCGGTGTCTTTGTGTTAACCTTAGCAAT |
| CPBC37 | /5Phos/NNGCGGTGCTGAAGAAAGTTGTCGGTGTCTTTGTGTTAACCTTAGCAAT |
| CPBC38 | /5Phos/NNATGGTGCTGAAGAAAGTTGTCGGTGTCTTTGTGTTAACCTTAGCAAT |
| CPBC39 | /5Phos/NNAGGGTGCTGAAGAAAGTTGTCGGTGTCTTTGTGTTAACCTTAGCAAT |
| CPBC40 | /5Phos/NNACGGTGCTGAAGAAAGTTGTCGGTGTCTTTGTGTTAACCTTAGCAAT |
| CPBC41 | /5Phos/NNTAGGTGCTGAAGAAAGTTGTCGGTGTCTTTGTGTTAACCTTAGCAAT |
| CPBC42 | /5Phos/NNTGGGTGCTGAAGAAAGTTGTCGGTGTCTTTGTGTTAACCTTAGCAAT |
| CPBC43 | /5Phos/NNTCGGTGCTGAAGAAAGTTGTCGGTGTCTTTGTGTTAACCTTAGCAAT |

/5Phos/ = 5' phosphate moiety

### Adapter sequence

The barcodes used throughout were purchased from IDT ("Custom DNA oligos")

| **Oligo** | **(5' → 3') Sequence** |
|---|---|
| SK43 | //CholTEG/TTGACCGCTCGCCTC |

/CholTEG / = Cholesterol-TEG

### Example 5

This Example demonstrates that a plurality of synthetic crRNA probes may be used to excise and sequence multiple regions of interest (ROIs) from different human genomic DNA (gDNA) samples. Here, ten human gene targets were excised from 5 different reactions, using a series of probes and barcodes, and sequenced using Cas9 to high coverage depth (>100x per allele) without amplification.

### Materials and Methods

High-molecular weight genomic DNA ("**gDNA**") was purified by extraction from cultured human cells (cell line GM12878; Coriell Institute) using a Qiagen tip-500, according to the manufacturer's instructions. A total of 25 µg gDNA was dephosphorylated in bulk via treatment with calf intestinal dephosphorylase. 15 µL 10x CutSmart Buffer and 15 µL Quick CIP (both from 'NEB Quick CIP kit', New England Biolabs, Inc., Cat # M0508) were added to the 25 µg of gDNA in a total of 150 µL (New England Biolabs, Inc., Catalogue # B7204) for 10 min at 37°C, followed by heat inactivation of the dephosphorylase at 80°C for 2 min. This step yielded **"end-protected gDNA".**

Wild-type S. *pyogenes* Cas9 ribonucleoprotein complexes (RNPs) were prepared as follows. An equimolar mix of 41 custom Alt-R Cas9 crRNAs (synthesized by Integrated DNA Technologies, Inc.) was prepared by mixing 1 µL of each crRNA (resuspended at 100 µM TE buffer, pH 7.5) in an Eppendorf DNA Lo-Bind tube. Alt-R^{®} CRISPR-Cas9 tracrRNA (Integrated DNA Technologies, Inc.) and the 41-probe pool of synthetic crRNAs were annealed by incubating 1 µL of tracrRNA (at 100 µM), 1 µL crRNA mix (at 100 µM) and 8 µL nuclease-free duplex buffer (Integrated DNA Technologies, Inc., Cat # 11-01-03-01) at 95°C for 5 min, followed by cooling to room temperature, to form 10 µM tracrRNA-crRNA complex. RNPs were then formed by incubating 4.8 µL of **tracrRNA-crRNA complex** (800 nM final concentration) with 400 nM S. *pyogenes* Cas9 (New England Biolabs, Inc., Cat # M0386M) in a total of 60 µL NEB CutSmart buffer at room temperature for 20 minutes. This step yielded 60 µL of **"Cas9 RNPs".**

Two separate libraries, A and B, were generated as follows:
**A.** 15 µL of **End-protected gDNA** (2.5 µg) was cleaved by Cas9 RNPs by adding 10 µL of the **Cas9 RNP** mix to the **end-protected gDNA** in a total volume of 30 µL. 5 units (1 µL) Taq polymerase (New England Biolabs M0273) and 200 µM of dATP were also added to the same tube (New England Biolabs N0446S). The reaction was incubated for 15 minutes at 37°C then 5 minutes at 72°C. In the same tube, 5 µL of AMX sequencing adapter (from Oxford Nanopore LSK-109), was ligated to the library using 10 µL of T4 ligase (from Oxford Nanopore) and 20 µL of LNB Buffer (from Oxford Nanopore LSK-109) in a total volume of 80 µL for 10 minutes at 21°C. This step yielded 2.5 µg **"target-cleaved DNA dA-tailed by Taq polymerase".**
**B.** Five separate tubes of 30 µL of **End-protected gDNA** (25 µg total; 5 µg per tube) was cleaved by Cas9 RNPs by adding 10 µL of the **Cas9 RNP** mix to each tube of **end-protected gDNA.** 5 units (1 µL) Taq polymerase (New England Biolabs M0273) was added to the same tube with 200 µM of dATP (New England Biolabs N0446S) and incubated for 15 minutes at 37 °C then 5 minutes at 72°C. Approximately 25 nM of native barcodes NB01 to NB05 (from Oxford Nanopore EXP-NBD-104), was ligated to 5 different probe-target complex using 20 µL of Blunt T/A Ligase Master Mix (New England Biolabs M0367) for 10 minutes at 21°C. Each mixture was subjected purified using SPRI magnetic beads, as follows: 0.7 volume equivalents of AMPure XP SPRI magnetic beads (Beckman Coulter) were added to the mixture and incubated for 10 min at 21°C. The magnetic beads were pelleted using a magnetic separator, the supernatant aspirated, and 250 µL of 70 % mix of Ethanol and nuclease-free water solution was used to wash the beads. The beads were immediately pelleted once more and the supernatant aspirated, after which the tube was removed from the rack and 14 µL nuclease-free water for 10 min at room temperature. The beads were pelleted using the magnetic separator, and the eluate retained. 13 µL of each eluate was pooled the same tube, resulting in a final volume of 65 µL. 5 µL of AMII barcode sequencing adapter (from Oxford Nanopore NBD-104) was ligated to probe-target complex using 10 µL of T4 ligase (from Oxford Nanopore) and 20 µL of LNB Buffer (from Oxford Nanopore LSK-109) for 10 minutes at 21°C in a total volume of 80 µL. This step yielded 12.5 µg "target-cleaved DNA with native barcodes".

Each mixture was subjected to purification step using SPRI magnetic beads, as follows: 1 volume equivalent of IDTE (Integrated DNA Technologies) and 0.3 volume equivalents of AMPure XP SPRI magnetic beads (Beckman Coulter) were added to the mixture and incubated for 10 min at 21°C. The magnetic beads were pelleted using a magnetic separator, the supernatant aspirated, and 250 µL of LFB (from **Oxford Nanopore SQK-LSK109**) added to resuspend the beads. The beads were immediately pelleted once more and the supernatant aspirated, after which the tube was removed from the rack and 16 µL EB buffer (Oxford Nanopore - LSK109) for 10 min at room temperature. The beads were pelleted using the magnetic separator, and the eluate retained. 13 µL **LB** and 25 µL **SQB** (both from Oxford Nanopore Technologies' LSK-109) were added to 12 µL of the eluate to yield **"MinION sequencing mixes A and B".**

To sequence target DNA, an Oxford Nanopore Technologies FLO-MIN106 flowcell was prepared by introducing 800 µL flowcell preparation mix (prepared using: 1170 µL FLB from Oxford Nanopore LSK-109, 30 µL FLT from Oxford Nanopore LSK-109) via the inlet port. The SpotON port was subsequently opened and a further 200 µL **flowcell preparation mix** perfused via the inlet port. 50 µL of MinION sequencing mix A, B were added to the flowcell via the SpotON port, and the ports closed. 16 h of sequencing data were collected using Oxford Nanopore Technologies' MinKNOW (version 1.15), and basecalled online using MinKNOW during the sequencing run, and aligned to the NA12878 human reference genome offline using minimap2. Library B was demultiplexed using Oxford Nanopore Technologies' Guppy basecaller.

### Results

Figure 23 shows the pileups resulting from alignment of sequencing reads to the human NA12878 reference (HTT gene) for **Library A and B** as well as the number of reads per barcodes per gene in **library B.** The crRNAs used in the experiment described above target protospacer sequences in ten human genes. Enrichment of the target regions was observed, as expected, showing that Cas9 cut predominantly in the correct location, the cut sites were released (to varying extents), dA-tailed, barcoding, and adapter efficiently ligated to the cut sites. Approximately 10% of all reads mapped to one of the ten target regions. An itemized list of reads for each target is given in Table 9.

**Table 9: Locations, number or reads and % on target reads for each target polynucleotide in Library A**

| **Target** | **Genomic coordinates of cut sites** | **Reads** | **% on target** |
|---|---|---|---|
| *HTT* | Chr4:3072436, 3072537, 3077290, 3079447 | 973 | 0.34 |
| *FMR1* | ChrX:147911805, 147911857, 147910984, 147911228, 147932674 | 537 | 0.19 |
| *SCA10* | Chr22:45791502, 45792656, 45798180, 45798335 | 1408 | 0.50 |
| *SCA2* | Chr12:111596525, 111597802, 111600589, 111602312 | 3260 | 1.15 |
| *SCA3* | Chr14:92068270, 92068306, 92073109, 92074370 | 1436 | 0.50 |
| *SCA17* | Chr6:170557049, 170557884, 170563749, 170565282 | 1738 | 0.61 |
| *SCA6* | Chr19:13205503, 13205664, 13210029, 13210853 | 1675 | 0.59 |
| *C9orf72* | Chr9:27572705, 27573133, 27574814, 27576479 | 1392 | 0.49 |
| *MUC1* | Chr1:155181544, 155183902, 155196219, 155197032 | 783 | 0.28 |
| *INS* | Chr11:2159199, 2159800, 2165720, 2166471 | 1006 | 0.35 |
| **all on target** | | **14208** | **5.00** |
| **all reads** | | **283789** | **100** |

Table 10 shows that approximately as many reads for the same ten-gene target panel were obtained when the 5 different samples were barcoded and pooled together (**Library B**). Only 1 in 150 reads mapped to one of the target regions (~0.6%), compared with 1 in 10 for Library A. Because the samples were pooled, more background reads were sequenced hence a reduction in percentage of reads on target was observed.

**Table 10: Locations, number or reads and % on target reads for each target polynucleotide in Library B (all barcodes)**

| **Target** | **Genomic coordinates of cut sites** | **Reads** | **% on target** |
|---|---|---|---|
| *HTT* | Chr4:3072436, 3072537, 3077290, 3079447 | 633 | 0.038 |
| *FMR1* | ChrX:147911805, 147911857, 147910984, 147911228, 147932674 | 387 | 0.023 |
| *SCA10* | Chr22:45791502, 45792656, 45798180, 45798335 | 956 | 0.057 |
| *SCA2* | Chr12:111596525, 111597802, 111600589, 111602312 | 2601 | 0.155 |
| *SCA3* | Chr14:92068270, 92068306, 92073109, 92074370 | 1167 | 0.070 |
| *SCA17* | Chr6:170557049, 170557884, 170563749, 170565282 | 1375 | 0.082 |
| *SCA6* | Chr19:13205503, 13205664, 13210029, 13210853 | 737 | 0.044 |
| *C9orf72* | Chr9:27572705, 27573133, 27574814, 27576479 | 1104 | 0.066 |
| *MUC1* | Chr1:155181544, 155183902, 155196219, 155197032 | 530 | 0.032 |
| *INS* | Chr11:2159199, 2159800, 2165720, 2166471 | 769 | 0.046 |
| **all on target** | | **10259** | **0.612** |
| **all reads** | | **1677458** | **100** |

Table 11 shows the distribution of reads per barcode used on one of the targets (the HTT gene) in Library B. The amount of reads per barcode is fairly consistent across all the barcodes used. Unclassified reads are low indicating barcoding and demultiplexing were efficient.

**Table 11: Reads and % on target reads per barcode used for HTT in Library B**

| **Target** | **Native barcode** | **Reads** | **% on target** |
|---|---|---|---|
| *HTT* | Native Barcode 02 | 176 | 0.168 |
| *HTT* | Native Barcode 04 | 150 | 0.156 |
| *HTT* | Native Barcode 07 | 102 | 0.149 |
| *HTT* | Native Barcode 10 | 77 | 0.135 |
| *HTT* | Native Barcode 11 | 82 | 0.134 |
| *HTT* | Unclassified Barcode | 45 | 0.004 |
| **all on target** | | **632** | **0.038** |
| **all reads** | | **1668604** | **100** |

### Example 6

This Example demonstrates how a synthetic crRNA probe can be used to excise and sequence regions of interest (ROIs) for a duplicated region of a low input bacterial genome for nanopore sequencing. Here is described a simple, one to two-pot approach, in which the enzymatic steps (dephosphorylation, cleavage, barcoding, amplification and adapter ligation) are performed sequentially.

### Materials and Methods

High-molecular weight genomic DNA ("**gDNA**") was purified by extraction from *Escherichia coli* (strain SCS110) using a Qiagen tip-500, according to the manufacturer's instructions. 2 µg gDNA was dephosphorylated via treatment with calf intestinal dephosphorylase. 3 µL Quick CIP (from 'NEB Quick CIP kit', New England Biolabs, Inc., Cat # M0508) was added to the 2 µg of gDNA in a total of 30 µL NEB CutSmart Buffer (New England Biolabs, Inc., Catalogue # B7204) for 10 min at 37°C, followed by heat inactivation of the dephosphorylase at 80°C for 2 min. This step yielded **"end-protected gDNA".**

40 µM of CasAmp top strand and 40 µM of CasAmp bottom strand were annealed in 25 µL of Nuclease-Free Duplex Buffer (Integrated DNA Technologies, Inc.) by incubating the reaction at 95°C for 5 min, followed by cooling to room temperature. The reaction was diluted to 1 µM by the addition of 1 µL of the annealed CasAmp strands to 39 µL of Nuclease-Free Duplex Buffer. This generated 40 µL of **"dephosphorylated PCR adapter".**

Wild-type *S. pyogenes* Cas9 ribonucleoprotein complexes (RNPs) were prepared as follows. Oligonucleotides **CPD1** and **CPD8** (known as **"guide RNAs")** were first pooled together at equimolar ratio. Alt-R^{®} CRISPR-Cas9 tracrRNA (Integrated DNA Technologies, Inc.) and the guide crRNAs were then annealed by incubating 1 µL of tracrRNA (at 100 µM), 1 µL **guide RNAs** (at 100 µM) and 8 µL nuclease-free duplex buffer (Integrated DNA Technologies, Inc., Cat # 11-01-03-01) at 95°C for 5 min, followed by cooling to room temperature to form 10 µM **tracrRNA-crRNA complex.** RNPs were then formed by incubating 2.4 µL of tracrRNA-crRNA complex (800 nM final concentration) with 400 nM HiFi Cas9 V3 (Integrated DNA Technologies, Inc.) in a total of 30 µL NEB CutSmart buffer at room temperature for 20 minutes. This step yielded 30 µL of "**Cas9 RNPs". 300 ng (from the total of 2 µg) end-protected gDNA** was cleaved and dA-tailed by incubation of 4.5 µL (300 ng) of the dephosphorylated library (**end-protected gDNA,** above), 30 µL Cas9 RNPs (above), 200 µM dATP (1.6 µL of 10 mM stock), 15 units (3 µL) Taq polymerase (New England Biolabs, Inc., Cat # M0273) for a total of 126 µL. This mixture was incubated at 37°C for 30 min to cleave target sites using Cas9, then 72°C for 5 min to both denature Cas9 and dA-tail all accessible 3' ends, using a PCR thermocycler, to yield 300 ng **"target-cleaved DNA, dA-tailed by Taq polymerase".** This step was performed in the same tube as the dephosphorylation step above and carried forwards for the next ligation step.

Three distinct reactions were performed in three single tubes as follows:
**(1)** A reaction which was not carried through an amplification step.
   **100 ng of target-cleaved DNA, dA-tailed by Taq polymerase** was carried to the next step.
**(2)** A reaction in which a PCR adapter was ligated to the target-cleaved, dA-tailed sample and an amplification step was performed.
   Approximately 25 nM of PCA adapter (from Oxford Nanopore EXP-PCA001), was ligated to 100 ng of **target-cleaved DNA, dA-tailed by Taq polymerase** complex using 10 µL of T4 ligase (from Oxford Nanopore) and 25 µL of LNB Buffer (from Oxford Nanopore LSK-109) for 10 minutes at 21°C.
**(3)** A reaction in which a dephosphorylated PCR adapter was ligated to the target-cleaved, dA-tailed sample and an amplification step was performed.

Approximately 25 nM of **"dephosphorylated PCR adapter"** was ligated to 100 ng of **target-cleaved DNA, dA-tailed by Taq polymerase** complex using 10 µL of T4 ligase (from Oxford Nanopore) and 25 µL of LNB Buffer (from Oxford Nanopore LSK-109) for 10 minutes at 21°C.

Mixture (2) and (3) were then subjected to SPRI purification to remove unligated adapter and other contaminants. 0.5 volumes (~50 µL) SPRI beads (AMPure XP beads, Beckman Coulter, Inc.) were added to the mixture, mixed gently by inversion, and incubated for 10 min at room temperature to bind the DNA to the beads. The beads were pelleted using a magnetic separator, the supernatant removed, and washed twice with 250 µL **LFB** (from Oxford Nanopore LSK-109), with complete resuspension of the beads at each wash and repelleting of the beads following the wash. Following the second wash, the beads were pelleted once more, the excess wash buffer removed, and the DNA eluted from the beads by resuspension of the bead pellet in 25 µL Nuclease-free water for 10 min at room temperature. This step yielded respectively 100 µg **"PCA adapted target-cleaved DNA"** and 100 µg **"dephosphorylated PCA adapted target-cleaved DNA".**

24 µL of these libraries were carried over with the addition of 200 nM PCR primer in 50 µL LongAmp^{®} Taq 2X Master Mix (New England Biolabs, Inc., Cat # M0287). Amplification was performed as follow using a PCR thermocycler: 72°C for 30 sec, 3 cycles of 95°C for 30 sec, 56°C for 30 sec and 72°C for 5 sec followed by 15 cycles of 95°C for 30 sec and 72°C for 5 min. Amplification was finished by 72°C for 5 min and on hold at 4°C.

Following the target cleavage, dA-tailing, PCR adapter ligation and amplification steps (for libraries (2) and (3)), sequencing adapter was ligated to each library. Adapter ligation was performed using 50 nM AMX (from Oxford Nanopore - LSK109), 10 µL of T4 ligase (from Oxford Nanopore) and 20 µL of LNB Buffer (from Oxford Nanopore LSK-109) for 10 minutes at 21°C.

Each mixture was subjected to purification step using SPRI magnetic beads, as follows: 1 volume equivalent of IDTE pH8 (Integrated DNA Technologies) and 0.3 volume equivalents of AMPure XP SPRI magnetic beads (Beckman Coulter) were added to the mixture and incubated for 10 min at 21°C. The magnetic beads were pelleted using a magnetic separator, the supernatant aspirated, and 250 µL of LFB **(ONT SQK-LSK109)** added to resuspend the beads. The beads were immediately pelleted once more and the supernatant aspirated, after which the tube was removed from the rack and 16 µL EB buffer (Oxford Nanopore - LSK109) for 10 min at room temperature. The beads were pelleted using the magnetic separator, and the eluate retained. This yielded a doublestranded DNAs bearing an adapter on each end, known as **"MinION sequencing mix (1), (2) and (3)".**

To sequence target DNA, an Oxford Nanopore Technologies FLO-MIN106 flowcell was prepared by introducing 800 µL flowcell preparation mix (prepared using: 1170 µL FLB from Oxford Nanopore LSK-109, 30 µL FLT from Oxford Nanopore LSK-109) via the inlet port. The SpotON port was subsequently opened and a further 200 µL **flowcell preparation mix** perfused via the inlet port. 50 µL of MinION sequencing mix (1), (2) and (3) were added to the flowcell via the SpotON port, and the ports closed. 16 h of sequencing data were collected using Oxford Nanopore Technologies' MinKNOW (version 1.15), and basecalled online using MinKNOW during the sequencing run, and aligned to the *E. coli* SCS110 reference genome offline.

### Results

Figure 24 shows the pileups resulting from alignment of sequencing reads to the *E. coli* SCS110 reference following the no amplification, amplification with phosphorylated or dephosphorylated PCR adapter approaches. The crRNAs used in the experiment described above target a 4kb region in the *E.coli* genome. Enrichment of the target region was observed in all the conditions indicating that the cleavage and dA-tailing occurred, as expected, in the correct location. The highest number of reads on target is observed when a dephosphorylated PCR adapter is ligated to the cut and dA-tailed sample, showing that the ligation of the adapter and amplification occurred as expected. The amplification step increased the number of reads by more that 10 times with a very high specificity (almost 95%).

Table 12 shows the number of reads and the percentage of on target reads for each of the libraries ((1) to (3)). The highest on-target throughput (94.87%) was obtained when the cleaved sample was amplified using dephosphorylated PCR adapter indicating that Cas9 cleavage, dA-tailing and amplification is possible from a low input genome.

**Table 12: Number or reads and % on target reads for each library**

| **Library** | **Description** | **reads** | **Reads on target** | **% target** |
|---|---|---|---|---|
| **(1)** | No amplification | 1984 | 1736 | 87.50 |
| **(2)** | Amplification with PCA | 237 | 131 | 55.27 |
| **(3)** | Amplification with dephosphorylated PCA | 24377 | 23127 | 94.87 |

### OLIGONUCLEOTIDES

**crRNA probes**

| | **Sequence 5' → 3'** |
|---|---|
| CPD1 | TAATGAGGATTTTTTCCGCG |
| CPD8 | TCGCCATTACGCATCAACAG |

**CasAmp oligonucleotides**

| | **Sequence 5' → 3'** |
|---|---|
| Top Strand | GGTTGTTTCTGTTGGTGCTGATATTGCGGCGTCTGCTTGGGTGTTTAACCT |
| Bottom Strand | GGTTAAACACCCAAGCAGACGCCG |

**PCR oligonucleotide**

| | **Sequence 5' → 3'** |
|---|---|
| PCR Primer | P-GGTGCTGAAGAAAGTTGTCGGTGTCTTTGTGTTAACCTTTCTGTTGGTGCTGATATTGC |

### Example 7

This Example demonstrates how a synthetic crRNA probe can be used to excise and sequence regions of interest (ROIs) for a duplicated region of a bacterial genome for nanopore sequencing and how the bias in the read directions can be modulated with the use of RNAse. Here is described a simple, one-pot approach, in which the enzymatic steps (dephosphorylation, cleavage, digestion and adapter ligation) are performed sequentially.

### Materials and Methods

High-molecular weight genomic DNA **("gDNA")** was purified by extraction from *Escherichia coli* (strain SCS110) using a Qiagen tip-500, according to the manufacturer's instructions. 1.5 µg gDNA was dephosphorylated via treatment with calf intestinal dephosphorylase. 7.5 µL Quick CIP (from 'NEB Quick CIP kit', New England Biolabs, Inc., Cat # M0508) was added to the 1.5 µg of gDNA in a total of 150 µL NEB CutSmart Buffer (New England Biolabs, Inc., Catalogue # B7204) for 10 min at 37°C, followed by heat inactivation of the dephosphorylase at 80°C for 2 min. This step yielded **"end-protected gDNA".**

Wild-type *S. pyogenes* Cas9 ribonucleoprotein complexes (RNPs) were prepared as follows. Alt-R^{®} CRISPR-Cas9 tracrRNA (Integrated DNA Technologies, Inc.) and AR400 (synthetic crRNA) were first annealed by incubating 1 µL of tracrRNA (at 100 µM), 1 µL AR400 (at 100 µM) and 8 µL nuclease-free duplex buffer (Integrated DNA Technologies, Inc., Cat # 11-01-03-01) at 95°C for 5 min, followed by cooling to room temperature to form 10 µM **tracrRNA-crRNA complex.** RNPs were then formed by incubating 4.5 µL of tracrRNA-crRNA complex (600 nM final concentration) with 300 nM *S. pyogenes* Cas9 (New England Biolabs, Inc., Cat # M0386M) in a total of 75 µL NEB CutSmart buffer at room temperature for 20 minutes. This step yielded 75 µL of **"Cas9 RNPs".**

Three distinct reactions were performed in three single tubes as follows:
**(1)** A reaction in which the sequencing adapter was ligated to the target-cleaved, dA-tailed sample
   **500 ng of end-protected gDNA** was cleaved and dA-tailed by incubation of 50 µL (100 ng) of the dephosphorylated library **(end-protected gDNA,** above), 25 µL Cas9 RNPs (above), 200 µM dATP (1.7 µL of 10 mM stock), 5 units (1 µL) Taq polymerase (New England Biolabs, Inc., Cat # M0273) for a total of 85 µL. This mixture was incubated at 37°C for 30 min to cleave target sites using Cas9, then 72°C for 5 min to both denature Cas9 and dA-tail all accessible 3' ends, using a PCR thermocycler, to yield 500 ng **"target-cleaved DNA, dA-tailed by Taq polymerase".**
(2) A reaction in which the target-cleaved DNA was digested by RNAseH then dA-tailed by Taq Polymerase. The sequencing adapter was then ligated to this sample.
   **500 ng of end-protected gDNA** was cleaved and dA-tailed by incubation of 50 µL (100 ng) of the dephosphorylated library **(end-protected gDNA,** above) and 25 µL Cas9 RNPs (above) was incubated at 37°C for 25 min to cleave target sites using Cas9. 5 units (1 µL) RNAseH (New England Biolabs, Inc., Cat # M0297) were added for a total of 85 µL NEBuffer^{™} 3 (New England Biolabs, Inc., Cat # #B7003). The reaction was incubated at 37°C for 20 min in order to digest DNA:RNA duplexes and 20°C min at 65°C in order to denature both Cas9 and RNAseH. 200 µM dATP (1.7 µL of 10 mM stock), 5 units (1 µL) Taq polymerase (New England Biolabs, Inc., Cat # M0273) were added to the same tube for a total of 85 µL. This mixture was incubated at 72°C for 5 min to dA-tail all accessible 3' ends, using a PCR thermocycler, to yield 500 ng **"target-cleaved DNA, digested by RNAseH and dA-tailed".**
**(3)** A reaction in which the target-cleaved DNA was incubated with RNAseH following Cas9 denaturation and then dA-tailed. The sequencing adapter was then ligated to this sample.
   **500 ng of end-protected gDNA** was cleaved and dA-tailed by incubation of 50 µL (100 ng) of the dephosphorylated library **(end-protected gDNA,** above) and 25 µL Cas9 RNPs (above) was incubated at 37°C for 25 min to cleave target sites using Cas9 and 5 mins at 65°C in order to denature Cas9. 5 units (1 µL) RNAseH (New England Biolabs, Inc., Cat # M0297) was added to the reaction for a total of 85 µL NEBuffer^{™} 3 (New England Biolabs, Inc., Cat # #B7003). The reaction was incubated at 37°C for 20 min in order to digest DNA:RNA duplexes and 20°C min at 65°C in order to denature RNAseH. 200 µM dATP (1.7 µL of 10 mM stock), 5 units (1 µL) Taq polymerase (New England Biolabs, Inc., Cat # M0273) were added to the same tube for a total of 85 µL. This mixture was incubated at 72°C for 5 min to dA-tail all accessible 3' ends, using a PCR thermocycler, to yield 500 ng **"target-cleaved DNA, digested by RNAseH and dA-tailed".**

Sequencing adapter was then ligated to each library by adding 25 nM of AMX 1D (from Oxford Nanopore LSK-108, concentrated to 1.7 µM using a Vivaspin-500 concentrator; Sartorius), 10 µL of T4 ligase (from Oxford Nanopore internal production) in 165 µL ligation buffer **(ONLS13117).** Following a 10 minute incubation at 21°C, each mixture was subjected to purification step using SPRI magnetic beads, as follows: 1 volume equivalent of IDTE pH8 (Integrated DNA Technologies) and 0.4 volume equivalents of AMPure XP SPRI magnetic beads (Beckman Coulter) were added to the mixture and incubated for 10 min at 21°C. The beads were pelleted using a magnetic separator, the supernatant removed, and washed twice with 250 µL **ABB** (from Oxford Nanopore LSK-108) ) diluted with DLB, with complete resuspension of the beads at each wash and repelleting of the beads following the wash. Following the second wash, the beads were pelleted once more, the excess wash buffer removed, and the DNA eluted from the beads by resuspension of the bead pellet in 15 µL ELB (From Oxford Nanopore SQK-LSK108) for 10 min at room temperature. 25 µL **SQB** and 10 µL **LB** (both from Oxford Nanopore Technologies' LSK-109) were added to 15 µL of the eluate to yield **"MinION sequencing mix".**

To sequence target DNA, an Oxford Nanopore Technologies FLO-MIN106 flowcell was prepared by introducing 800 µL flowcell preparation mix (prepared using: 1170 µL FLB from Oxford Nanopore LSK-109, 30 µL FLT from Oxford Nanopore LSK-109) via the inlet port. The SpotON port was subsequently opened and a further 200 µL **flowcell preparation mix** perfused via the inlet port. 50 µL of MinION sequencing mix (1), (2) and (3) were added to the flowcell via the SpotON port, and the ports closed. 6 h of sequencing data were collected using Oxford Nanopore Technologies' MinKNOW (version 1.10.6), and subsequently basecalled (using **Albacore**) and aligned to the *E. coli* SCS110 reference genome offline.

### Results

Figure 25 shows the pileups resulting from alignment of sequencing reads to the *E. coli* reference. The crRNA used in the experiment described above targets a protospacer sequence common to all seven copies of the *rrs* gene in strain *E. coli* SCS110. Enrichment of the target region was observed, as expected, at each of the seven *rrs* genes (the locations of which are shown in Tables 13 to 15), showing that Cas9 cut predominantly in the correct location, and that the cut sites were released (to varying extents) and dA-tailed, and that the adapter was efficiently ligated to the cut sites. This figure also highlights that more bidirectional reads are observed with the addition of RNAseH following Cas9 cleavage and denaturation.

Table 13 examines the bias between forwards and reverse orientation reads from the Taq polymerase condition (library (1)). The *rrs* gene, targeted by the degenerate crRNA probe, is found in both orientations in the *E. coli* SCS110 reference. Six out of the seven *rrs* genes exhibited a clear bias in read direction, which correlated with the orientation of the gene in the reference genome. A similar bias was observed with other conditions (library (2), Table 14, Figure 25).

However, Table 15, examining the read bias in library (3) shows that the addition of RNAseH following Cas9 cleavage and denaturation relieved some of the read bias compared to libraries (1) and (2). For example, the read bias for the peak *i*, corresponding to *rrsH* gene was lowered to about 42% with the addition of RNAseH compared to 34% in library (1).

**Table 13: The locations of the rrs gene in E.coli and the read bias between forward and reverse orientation reads obtained for library (1) when the cleaved sample was dA-tailed at 72°C using Taq polymerase**

| **Peak** | **Gene** | **Genomic coordinates** | **Location of crRNA** | **Chromosomal orientation** | **Number of + reads** | **Number of** - **reads** | **Overall read bias (% of** - **reads)** |
|---|---|---|---|---|---|---|---|
| *i* | *rrsH* | 223771-225312 | 223960 | + | 807 | 422 | 34.34 |
| *ii* | *rrsG* | 2729616-2731157 | 2730968 | - | 366 | 682 | 65.08 |
| *iii* | *rrsD* | 3427221-3428762 | 3428573 | - | 101 | 549 | 84.46 |
| *iv* | *rrsC* | 3941808-3943349 | 3941997 | + | 934 | 417 | 30.87 |
| *v* | *rrsA* | 4035531-4037072 | 4035720 | + | 778 | 409 | 34.46 |
| *vi* | *rrsB* | 4166659-4168200 | 4166848 | + | 968 | 394 | 28.93 |
| *vii* | *rrsE* | 4208147-4209688 | 4208336 | + | 629 | 623 | 49.76 |

**Table 14: The locations of the rrs gene in E.Coli and the read bias between forward and reverse orientation reads obtained for library (2) when the cleaved sample was digested with RNAseH following Cas9 cleavage.**

| **Peak** | **Gene** | **Genomic coordinates** | **Location of crRNA** | **Chromosomal orientation** | **Number of + reads** | **Number of** - **reads** | **Overall read bias (% of** - **reads)** |
|---|---|---|---|---|---|---|---|
| *i* | *rrsH* | 223771-225312 | 223960 | + | 840 | 355 | 29.71 |
| *ii* | *rrsG* | 2729616-2731157 | 2730968 | - | 265 | 668 | 71.6 |
| *iii* | *rrsD* | 3427221-3428762 | 3428573 | - | 185 | 547 | 74.73 |
| *iv* | *rrsC* | 3941808-3943349 | 3941997 | + | 881 | 333 | 27.43 |
| *v* | *rrsA* | 4035531-4037072 | 4035720 | + | 822 | 362 | 30.57 |
| *vi* | *rrsB* | 4166659-4168200 | 4166848 | + | 1019 | 362 | 26.21 |
| *vii* | *rrsE* | 4208147-4209688 | 4208336 | + | 621 | 563 | 47.55 |

**Table 15: The locations of the rrs gene in E.Coli and the read bias between forward and reverse orientation reads obtained for library (3) when the cleaved sample was digested with RNAseH following Cas9 cleavage and Cas9 denaturation.**

| **Peak** | **Gene** | **Genomic coordinates** | **Location of crRNA** | **Chromosomal orientation** | **Number of + reads** | **Number of - reads** | **Overall read bias (% of** - **reads)** |
|---|---|---|---|---|---|---|---|
| *i* | *rrsH* | 223771-225312 | 223960 | + | 638 | 461 | 41.95 |
| *ii* | *rrsG* | 2729616-2731157 | 2730968 | - | 335 | 544 | 61.89 |
| *iii* | *rrsD* | 3427221-3428762 | 3428573 | - | 223 | 460 | 67.35 |
| *iv* | *rrsC* | 3941808-3943349 | 3941997 | + | 693 | 455 | 39.63 |
| *v* | *rrsA* | 4035531-4037072 | 4035720 | + | 605 | 440 | 42.11 |
| *vi* | *rrsB* | 4166659-4168200 | 4166848 | + | 1049 | 431 | 29.12 |
| *vii* | *rrsE* | 4208147-4209688 | 4208336 | + | 485 | 896 | 64.88 |

### Example 8

This Example demonstrates how a synthetic crRNA probe can be used to excise and sequence regions of interest (ROIs) for a duplicated region of a bacterial genome for nanopore sequencing and how the sequencing direction of the reads originating from the cleavage can be biased to one direction via the use of T4 polymerase. Here is described a simple, one-pot approach, in which the enzymatic steps (dephosphorylation, cleavage, digestion and adapter ligation) are performed sequentially.

### Materials and Methods

High-molecular weight genomic DNA **("gDNA")** was purified by extraction from *Escherichia coli* (strain SCS110) using a Qiagen tip-500, according to the manufacturer's instructions. 1.5 µg gDNA was dephosphorylated via treatment with calf intestinal dephosphorylase. 7.5 µL Quick CIP (from 'NEB Quick CIP kit', New England Biolabs, Inc., Cat # M0508) was added to the 1.5 µg of gDNA in a total of 150 µL NEB CutSmart Buffer (New England Biolabs, Inc., Catalogue # B7204) for 10 min at 37°C, followed by heat inactivation of the dephosphorylase at 80°C for 2 min. This step yielded "end-**protected gDNA".**

Wild-type *S. pyogenes* Cas9 ribonucleoprotein complexes (RNPs) were prepared as follows. Alt-R^{®} CRISPR-Cas9 tracrRNA (Integrated DNA Technologies, Inc.) and AR400 (synthetic crRNA) were first annealed by incubating 1 µL of tracrRNA (at 100 µM), 1 µL AR400 (at 100 µM) and 8 µL nuclease-free duplex buffer (Integrated DNA Technologies, Inc., Cat # 11-01-03-01) at 95°C for 5 min, followed by cooling to room temperature to form 10 µM **tracrRNA-crRNA complex.** RNPs were then formed by incubating 4.5 µL of tracrRNA-crRNA complex (600 nM final concentration) with 300 nM *S. pyogenes* Cas9 (New England Biolabs, Inc., Cat # M0386M) in a total of 75 µL NEB CutSmart buffer at room temperature for 20 minutes. This step yielded 75 µL of **"Cas9 RNPs".**

Three distinct reactions were performed in three single tubes as follows:
**(1)** A reaction in which the sequencing adapter was ligated to the target-cleaved, dA-tailed sample
   **500 ng of end-protected gDNA** was cleaved and dA-tailed by incubation of 50 µL (500 ng) of the dephosphorylated library **(end-protected gDNA,** above), 25 µL Cas9 RNPs (above), 200 µM dATP (1.7 µL of 10 mM stock), 5 units (1 µL) Taq polymerase (New England Biolabs, Inc., Cat # M0273) for a total of 85 µL. This mixture was incubated at 37°C for 30 min to cleave target sites using Cas9, then 72°C for 5 min to both denature Cas9 and dA-tail all accessible 3' ends, using a PCR thermocycler, to yield 500 ng **"target-cleaved DNA, dA-tailed by Taq polymerase".**
**(2)** A reaction in which the target-cleaved, was incubated with T4 DNA polymerase and then dA-tailed. The sequencing adapter was then ligated to this sample.
   **500 ng of end-protected gDNA** was cleaved and dA-tailed by incubation of 50 µL (100 ng) of the dephosphorylated library **(end-protected gDNA,** above) and 25 µL Cas9 RNPs (above) was incubated at 37°C for 25 min to cleave target sites using Cas9. 3 units (1 µL) T4 DNA Polymerase (New England Biolabs, Inc., Cat # M0203) were added for a total of 85 µL. In the absence of dNTPs, T4 DNA Polymerase acts as a 3' to 5' end exonuclease and is here used to remove any potential 3'end overhang. The reaction was incubated at 21°C for 5 min. 200 µM dATP (1.7 µL of 10 mM stock), 5 units (1 µL) Taq polymerase (New England Biolabs, Inc., Cat # M0273) were added to the same tube for a total of 80 µL. This mixture was incubated at 72°C for 5 min to dA-tail all accessible 3' ends, using a PCR thermocycler, to yield 500 ng **"target-cleaved DNA, digested by T4 DNA Polymerase and dA-tailed".**
**(3)** A reaction in which the target-cleaved, was incubated with T4 DNA polymerase following Cas9 denaturation, dA-tailed. The sequencing adapter was then ligated to this sample.
   **500 ng of end-protected gDNA** was cleaved and dA-tailed by incubation of 50 µL (100 ng) of the dephosphorylated library **(end-protected gDNA,** above) and 25 µL Cas9 RNPs (above) was incubated at 37°C for 25 min to cleave target sites using Cas9 and 5 mins at 65°C in order to denature Cas9. 3 units (1 µL) T4 DNA Polymerase (New England Biolabs, Inc., Cat # M0203) were added to the reaction for a total of 80 µL. In the absence of dNTPs, T4 DNA Polymerase acts as a 3' to 5' end exonuclease and is here used to remove any potential 3'end overhang. The reaction was incubated at 21°C for 5 min. 200 µM dATP (1.7 µL of 10 mM stock), 5 units (1 µL) Taq polymerase (New England Biolabs, Inc., Cat # M0273) were added to the same tube for a total of 80 µL. This mixture was incubated at 72°C for 5 min to dA-tail all accessible 3' ends, using a PCR thermocycler, to yield 500 ng **"target-cleaved DNA, denatured, digested by T4 DNA Polymerase and dA-tailed".**

Sequencing adapter was then ligated to each library by adding 25 nM of AMX 1D (from Oxford Nanopore LSK-108, concentrated to 1.7 µM using a Vivaspin-500 concentrator; Sartorius), 10 µL of T4 ligase (from Oxford Nanopore internal production) in 165 µL ligation buffer **(ONLS13117).** Following a 10 mins incubation at 21°C, each mixture was subjected to purification step using SPRI magnetic beads, as follows: 1 volume equivalent of IDTE pH8 (Integrated DNA Technologies) and 0.4 volume equivalents of AMPure XP SPRI magnetic beads (Beckman Coulter) were added to the mixture and incubated for 10 min at 21°C. The beads were pelleted using a magnetic separator, the supernatant removed, and washed twice with 250 µL **ABB** (from Oxford Nanopore LSK-108) ) diluted with DLB, with complete resuspension of the beads at each wash and repelleting of the beads following the wash. Following the second wash, the beads were pelleted once more, the excess wash buffer removed, and the DNA eluted from the beads by resuspension of the bead pellet in 15 µL ELB (From Oxford Nanopore SQK-LSK108) for 10 min at room temperature. 25 µL **SQB** and 10 µL **LB** (both from Oxford Nanopore Technologies' LSK-109) were added to 15 µL of the eluate to yield **"MinION sequencing mix".**

To sequence target DNA, an Oxford Nanopore Technologies FLO-MIN106 flowcell was prepared by introducing 800 µL flowcell preparation mix (prepared using: 1170 µL FLB from Oxford Nanopore LSK-109, 30 µL FLT from Oxford Nanopore LSK-109) via the inlet port. The SpotON port was subsequently opened and a further 200 µL **flowcell preparation mix** perfused via the inlet port. 50 µL of MinION sequencing mix (1), (2) and (3) were added to the flowcell via the SpotON port, and the ports closed. 6 h of sequencing data were collected using Oxford Nanopore Technologies' MinKNOW (version 1.10.6), and subsequently basecalled (using **Albacore**) and aligned to the *E. coli* SCS110 reference genome offline.

### Results

Figure 26 shows the pileups resulting from alignment of sequencing reads to the *E. coli* reference. The crRNA used in the experiment described above targets a protospacer sequence common to all seven copies of the *rrs* gene in strain *E. coli* SCS110. Enrichment of the target region as observed, as expected, at each of the seven *rrs* genes (the locations of which are shown in tables 17 to 19), showing that Cas9 cut predominantly in the correct location, and that the cut sites were released (to varying extents) and dA-tailed, and that the adapter was efficiently ligated to the cut sites. This figure also highlights that fewer bidirectional reads were observed with the addition of T4 DNA Polymerase following Cas9 cleavage.

Tables 17 to 19 examine the bias between forwards and reverse orientation reads from the Taq polymerase condition (library (1)). The *rrs* gene, targeted by the degenerate crRNA probe, is found in both orientations in the *E. coli* SCS110 reference. Six out of the seven *rrs* genes exhibited a clear bias in read direction, which correlated with the orientation of the gene in the reference genome.

However, Table 18 and 19, examining the read bias in library (2) and (3) show that the addition of T4 DNA Polymerase following Cas9 cleavage with or without Cas9 denaturation increases of the read bias compared to libraries (1). For example, the read bias toward the (+) direction for the peak *i,* corresponding to *rrsH* gene was about 96% with the addition of T4 DNA polymerase compared to 65% in library (1). This indicate that the addition of T4 DNA Polymerase reduces the efficiency of the sequencing adapter ligation to the PAM-distal side of Cas9 cleavage sites.

**Table 17: The locations of the rrs gene in E.Coli and the read bias between forward and reverse orientation reads obtained for library (1) when the cleaved sample was dA-tailed at 72°C using Taq polymerase**

| **Peak** | **Gene** | **Genomic coordinates** | **Location of crRNA** | **Chromosomal orientation** | **Number of + reads** | **Number of - reads** | **Overall read bias (% of + reads)** |
|---|---|---|---|---|---|---|---|
| *i* | *rrsH* | 223771-225312 | 223960 | + | 836 | 444 | 65.31 |
| *ii* | *rrsG* | 2729616-2731157 | 2730968 | - | 338 | 674 | 33.40 |
| *iii* | *rrsD* | 3427221-3428762 | 3428573 | - | 93 | 534 | 14.83 |
| *iv* | *rrsC* | 3941808-3943349 | 3941997 | + | 893 | 361 | 71.21 |
| *v* | *rrsA* | 4035531-4037072 | 4035720 | + | 748 | 403 | 64.99 |
| *vi* | *rrsB* | 4166659-4168200 | 4166848 | + | 1040 | 425 | 70.99 |
| *vii* | *rrsE* | 4208147-4209688 | 4208336 | + | 668 | 627 | 51.58 |

**Table 18: The locations of the rrs gene in E.Coli and the read bias between forward and reverse orientation reads obtained for library (2) when the cleaved sample was digested with T4 DNA Polymerase following Cas9 cleavage.**

| **Peak** | **Gene** | **Genomic coordinates** | **Location of crRNA** | **Chromosomal orientation** | **Number of + reads** | **Number of - reads** | **Overall read bias (% of + reads)** |
|---|---|---|---|---|---|---|---|
| *i* | *rrsH* | 223771-225312 | 223960 | + | 1046 | 41 | 96.23 |
| *ii* | *rrsG* | 2729616-2731157 | 2730968 | - | 33 | 877 | 3.63 |
| *iii* | *rrsD* | 3427221-3428762 | 3428573 | - | 32 | 307 | 9.44 |
| *iv* | *rrsC* | 3941808-3943349 | 3941997 | + | 1048 | 50 | 95.45 |
| *v* | *rrsA* | 4035531-4037072 | 4035720 | + | 845 | 37 | 95.80 |
| *vi* | *rrsB* | 4166659-4168200 | 4166848 | + | 1084 | 43 | 96.18 |
| *vii* | *rrsE* | 4208147-4209688 | 4208336 | + | 853 | 70 | 92.42 |

**Table 19: The locations of the rrs gene in E.Coli and the read bias between forward and reverse orientation reads obtained for library (3) when the cleaved sample was digested with T4 DNA Polymerase following Cas9 cleavage and Cas9 denaturation.**

| **Peak** | **Gene** | **Genomic coordinates** | **Location of crRNA** | **Chromosomal orientation** | **Number of + reads** | **Number of - reads** | **Overall read bias (% of + reads)** |
|---|---|---|---|---|---|---|---|
| *i* | *rrsH* | 223771-225312 | 223960 | + | 92.08 | 92.08 | 92.08 |
| *ii* | *rrsG* | 2729616-2731157 | 2730968 | - | 8.81 | 8.81 | 8.81 |
| *iii* | *rrsD* | 3427221-3428762 | 3428573 | - | 85.71 | 85.71 | 85.71 |
| *iv* | *rrsC* | 3941808-3943349 | 3941997 | + | 91.04 | 91.04 | 91.04 |
| *v* | *rrsA* | 4035531-4037072 | 4035720 | + | 90.43 | 90.43 | 90.43 |
| *vi* | *rrsB* | 4166659-4168200 | 4166848 | + | 90.35 | 90.35 | 90.35 |
| *vii* | *rrsE* | 4208147-4209688 | 4208336 | + | 80.43 | 80.43 | 80.43 |

### ASPECTS

1. A method for selectively adapting a target polynucleotide in a sample of polynucleotides, the method comprising:
   (a) protecting the ends of the polynucleotides in the sample;
   (b) contacting the polynucleotides with a guide polynucleotide that binds to a sequence in the target polynucleotide and a polynucleotide-guided effector protein such that the polynucleotide-guided effector protein cuts the target polynucleotide to produce two opposing cut ends at a site determined by the sequence to which the guide polynucleotide binds; and
   (c) attaching an adapter to one or both of the two opposing cut ends in the target polynucleotide,
      wherein the adapter attaches to one or both of the cut ends in the target polynucleotide but does not attach to the protected ends of the polynucleotides in the sample.
2. A method according to aspect 1, wherein the ends of the polynucleotides in the sample are protected by dephosphorylating the 5' ends of the polynucleotides.
3. A method according to aspect 2, wherein the 5' ends of the polynucleotides are dephosphorylated by adding a dephosphorylase to the sample of polynucleotides.
4. A method according to aspect 1, wherein the ends of the polynucleotides in the sample are protected by extending the 3' ends of the polynucleotides to produce a single stranded overhang.
5. A method according to aspect 4, wherein the 3' ends of the polynucleotides are extended by adding a terminal transferase and a dNTP to the sample of polynucleotides.
6. A method according to any one of the preceding aspects, wherein the polynucleotide-guided effector protein is an RNA-guided effector protein.
7. A method according to aspect 6, wherein the polynucleotide-guided effector protein is Cas3, Cas4, Cas8a, Cas8b, Cas8c, Cas9, Cas10, Cas10d, Cas12a, Casl3, Csn2, Csfl, Cmr5, Csm2, Csyl, Csel or C2c2.
8. A method according to any one of the preceding aspects, wherein the target polynucleotide comprises double stranded DNA.
9. A method according to any one of the preceding aspects, wherein the polynucleotide-guided effector protein cuts one strand of a double stranded polynucleotide.
10. A method according to any one of the preceding aspects, wherein the polynucleotide-guided effector protein cuts both strands of a double stranded polynucleotide to produce a blunt end.
11. A method according to any one of the preceding aspects, wherein the polynucleotide-guided effector protein cuts both strands of a double stranded polynucleotide to produce a single stranded overhang.
12. A method according to aspect 11, wherein the method comprises contacting the sample with a polymerase or terminal transferase and dNTPs to fill in the overhang to produce a blunt end, or a single nucleotide overhang.
13. A method according to any one of the preceding aspects, wherein the adapter comprises a single T or polyT tail and the method further comprises contacting the sample prior to step (c) with a polymerase and dATP to add a single A tail to at least one of the cut ends in the target polynucleotide.
14. A method according to aspect 13, wherein the polymerase is active at a temperature over about 60°C.
15. A method according to aspect 13 or 14, wherein the polymerase is Taq polymerase.
16. A method according to any one of the preceding aspects, wherein the adapter is covalently attached to the target polynucleotide.
17. A method according to aspect 16, wherein the adapter is covalently attached to the target polynucleotide by ligation or topoisomerisation.
18. A method according to any one of the preceding aspects, wherein the polynucleotide-guided effector protein remains attached to one of the two opposing cut ends and the adapter is attached to the other one of the two opposing cut ends.
19. A method according to any one of the preceding aspects, wherein the polynucleotide-guided effector protein does not remain attached to the target polynucleotide, or is removed from the target polynucleotide.
20. A method according to any one of the preceding aspects, wherein the adapter is an intermediate adapter and the method comprises attaching a further adapter to the intermediate adapter.
21. A method according to aspect 20, wherein the further adapter is a sequencing adapter.
22. A method according to any one of the preceding aspects, wherein the polynucleotides are contacted with one or more guide polynucleotides that bind to the target polynucleotide within a region of interest.
23. A method according to any one of aspects 1 to 21, wherein the polynucleotides are contacted with one or more guide polynucleotides that bind to the target polynucleotide outside a region of interest.
24. A method according to any one of the preceding aspects, wherein the polynucleotides are contacted with two or more guide polynucleotides that bind to different sequences in the target polynucleotide such that the polynucleotide-guided effector protein cuts the target polynucleotide at two or more sites to produce two opposing cut ends at each site.
25. A method according to aspect 24, wherein at least one of the two or more sites is located on each side of the region of interest in the target polynucleotide, and none of the two or more sites is located within the region of interest.
26. A method according to aspect 24 or 25, wherein the guide polynucleotides are orientated such that, after cutting the target polynucleotide at the sites located on each side of the region of interest, the polynucleotide-guided effector protein remains attached to the cut end of the polynucleotide that does not contain the region of interest.
27. A method according to any one of aspects 24 to 26, wherein the two or more sites comprise at least two sites on either side of a region of interest in the target polynucleotide.
28. A method according to any one of aspects 24 to 27, wherein the same polynucleotide-guided effector protein is used to cut at all of the two or more sites.
29. A method according to any one of aspects 24 to 27, wherein different polynucleotide-guided effector proteins are used to cut at the two or more sites.
30. A method according to any one of aspects 24 to 29, wherein the method further comprises amplifying a region of interest in a target polynucleotide using a pair of PCR primers that hybridise to sequences within the adapter that flank the region of interest in the adapted polynucleotide.
31. A method according to any one of the preceding aspects, wherein two or more guide polynucleotides that bind to sequences in two or more different target polynucleotides are used in the method in order to attach adapters within or flanking at least one region of interest in each of the target polynucleotides.
32. A method according to any one of the preceding aspects, wherein two or more guide polynucleotides are used in the method in order to attach adapters within or flanking two or more regions of interest in a target polynucleotide.
33. A method according to any one of the preceding aspects, which further comprises attaching a further adapter to the adapters on the adapted polynucleotides.
34. A method according to aspect 33, wherein the further adapter is a sequencing adapter.
35. A method according to aspect 34, wherein the sequencing adapter comprises a single stranded leader sequence, a polynucleotide binding protein and/or a membrane or pore anchor.
36. A method according to any one of the preceding aspects, wherein the method further comprises characterising the target polynucleotide.
37. A method of detecting and/or characterising a target polynucleotide comprising:
   (i) contacting a sample obtained by a method according to any one of aspects 1 to 35 with a nanopore;
   (ii) applying a potential difference across the nanopore; and
   (iii) monitoring for the presence or absence of an effect resulting from the interaction of the target polynucleotide with the nanopore to determine the presence or absence of the target polynucleotide, thereby detecting the target polynucleotide in the sample and/or monitoring the interaction of the target polynucleotide with the nanopore to determine one or more characteristics of the target polynucleotide.
38. A method according to aspect 37, wherein the target polynucleotide is characterised by sequencing.
39. A kit for selectively modifying a target polynucleotide in a sample of polynucleotides, the kit comprising a dephosphorylase, an adapter comprising a single N or polyN tail, wherein N is the nucleotide A, T, C or G, and optionally one or more of a polymerase, a ligase, a polynucleotide-guided effector protein and a guide polynucleotide.
40. A method for selectively adapting a target polynucleotide in a sample of polynucleotides, the method comprising:
   (a) contacting the polynucleotides in the sample with two guide polynucleotides that bind to a sequences in the target polynucleotide and a polynucleotide-guided effector protein, wherein the sequences to which the two guide polynucleotides bind direct the polynucleotide-guided effector protein to two sites, such that the polynucleotide-guided effector protein cuts the target polynucleotide at at least one of the two sites to produce two opposing cut ends; and
   (b) attaching an adapter to one or both of the two opposing cut ends in the target polynucleotide.

## Claims

1. A method for selectively adapting a target polynucleotide in a sample of polynucleotides, the method comprising:
(a) protecting the ends of the polynucleotides in the sample;
(b) contacting the polynucleotides with two or more guide polynucleotides that bind to different sequences in the target polynucleotide and a polynucleotide-guided effector protein such that the polynucleotide-guided effector protein cuts the target polynucleotide to produce two opposing cut ends at each of two or more sites determined by the sequences to which the guide polynucleotides bind, wherein at least one of the two or more sites is located on each side of the region of interest in the target polynucleotide and wherein the guide polynucleotides are orientated such that, after cutting the target polynucleotide at the sites located on each side of the region of interest, the polynucleotide-guided effector protein remains attached to the cut end of the polynucleotide that does not contain the region of interest; and
(c) attaching an adapter to one or both of the two opposing cut ends in the target polynucleotide,
wherein the adapter attaches to one or both of the cut ends in the target polynucleotide but does not attach to the protected ends of the polynucleotides in the sample.

2. A method according to claim 1, wherein the ends of the polynucleotides in the sample are protected by chemically altering the ends of the polynucleotides using an enzyme, wherein the ends of the polynucleotides in the sample are protected by (i) dephosphorylating the 5' ends of the polynucleotides or (ii) extending the 3' ends of the polynucleotides to produce a single stranded overhang.

3. A method according to claim 1 or 2, wherein the 5' ends of the polynucleotides are dephosphorylated by adding a dephosphorylase to the sample of polynucleotides.

4. A method according to claim 1 or 2, wherein the 3' ends of the polynucleotides are extended by adding a terminal transferase and a dNTP to the sample of polynucleotides.

5. A method according to any one of the preceding claims, wherein the polynucleotide-guided effector protein is an RNA-guided effector protein, optionally wherein the polynucleotide-guided effector protein is Cas3, Cas4, Cas8a, Cas8b, Cas8c, Cas9, Cas10, Cas10d, Cas12a, Cas13, Csn2, Csf1, Cmr5, Csm2, Csy1, Cse1 or C2c2.

6. A method according to any one of the preceding claims, wherein the target polynucleotide comprises double stranded DNA.

7. A method according to any one of the preceding claims, wherein (i) the polynucleotide-guided effector protein cuts one strand of a double stranded polynucleotide or wherein (ii) the polynucleotide-guided effector protein cuts both strands of a double stranded polynucleotide to produce a blunt end or to produce a single stranded overhang.

8. A method according to claim 7, wherein the method comprises contacting the sample with a polymerase or terminal transferase and dNTPs to fill in the overhang to produce a blunt end, or a single nucleotide overhang.

9. A method according to any one of the preceding claims, wherein the adapter comprises a single T or polyT tail and the method further comprises contacting the sample prior to step (c) with a polymerase and dATP to add a single A tail to at least one of the cut ends in the target polynucleotide, optionally wherein the polymerase is Taq polymerase.

10. A method according to any one of the preceding claims, wherein (i) the adapter is a sequencing adapter or (ii) the adapter is an intermediate adapter and the method comprises attaching a further adapter to the intermediate adapter, optionally wherein the further adapter is a sequencing adapter.

11. A method according to claim 10, wherein the sequencing adapter is a sequencing adapter comprises a single stranded leader sequence, a polynucleotide binding protein and/or a membrane or pore anchor.

12. A method according to any one of the preceding claims, wherein the two or more sites comprise at least two sites on either side of the region of interest.

13. A method according to any one of the preceding claims, wherein the method further comprises amplifying a region of interest in a target polynucleotide using a pair of PCR primers that hybridise to sequences within the adapter that flank the region of interest in the adapted polynucleotide.

14. A method according to any one of the preceding claims, wherein (i) two or more guide polynucleotides that bind to sequences in two or more different target polynucleotides are used in the method in order to attach adapters within or flanking at least one region of interest in each of the target polynucleotides or (ii) wherein two or more guide polynucleotides are used in the method in order to attach adapters within or flanking two or more regions of interest in a target polynucleotide.

15. A method of detecting and/or characterising a target polynucleotide comprising:
(i) contacting a sample obtained by a method according to any one of claims 1 to 14 with a nanopore;
(ii) applying a potential difference across the nanopore; and
(iii) monitoring for the presence or absence of an effect resulting from the interaction of the target polynucleotide with the nanopore to determine the presence or absence of the target polynucleotide, thereby detecting the target polynucleotide in the sample and/or monitoring the interaction of the target polynucleotide with the nanopore to determine one or more characteristics of the target polynucleotide.
